# EUROPEAN PATENT APPLICATION

(11) **EP 4 813 357 A1**
(43) Date of publication of application: **30.09.2026**
(21) Application number: 26168454.2
(22) Date of filing: 27.03.2026
(51) Int. Cl.: A61F 7/00, A61G 7/00

(54) **TEMPERATURE CONTROL SYSTEMS FOR A SUPPORT SURFACE**

(30) Priority: 28.03.2025 US 202563779478 P
(71) Applicant: Stryker Corporation, Portage, MI 49002 (US)
(72) Inventor: Alvarez, Ryan Ariel, Kalamazoo, 49004 (US); Gargioni, Gregory, Portage, 49002 (US); Falkowski, Rachel, Kalamazoo, 49007 (US); Galer, James K., Byron Center, 49315 (US); McNeeley, Grace, Portage, 49002 (US); Koch, Nicholas D., Battle Creek, 49015 (US); Nahavandi, Kurosh, Portage, 49002 (US); Graves, Michael W., Augusta, 49012 (US)
(74) Representative: Schott, Jakob Valentin

(57) **Abstract**

A patient support system and a cooling system for the same is provided. In one aspect, the patient support system may include a closed loop cooling system operable to cool a patient without significantly affecting a core body temperature of the patient.

## Description

### TECHNICAL FIELD

The present disclosure relates to mattresses used for supporting individuals thereon, and more particularly to mattresses that are used in healthcare settings and that have one or more regions for which a temperature can be controlled.

### BACKGROUND

Pressure injuries constitute the second highest hospital-acquired condition in U.S. hospitals, claiming the lives of 60,000 patients per year. Risk factors include pressure, shear, and microclimate (heat and moisture trapped at the patient's skin as they lie supine on a support surface [e.g., a hospital mattress]). As patients lie in bed, heat and moisture accumulate beneath them and continue to increase until they rotate. Some conventional support surfaces contain microclimate management features such as low air loss that circulate air to dissipate that trapped heat and moisture. Increased performance often involves a breathable cover to allow the circulating air to remove the moisture vapor and heat of the patient interface.

It is understood that there is a difference in the layers tested for microclimate performance and those used in clinical practice. Performance tests are either run on the top cover or with a bed sheet when in practice patients also lay on repositioning devices, incontinence pads, and have other layers between them and the support surface. Each additional layer provides benefits that the support surface does not, thereby causing the caregiver to weigh care priorities for their patients. These layers prevent the support surface's microclimate management features from dissipating the trapped heat and moisture from the patient's skin.

Various conventional microclimate management solutions exist to address patient moisture and heat accumulation. These conventional solutions often focus on transferring moisture vapor through the top cover of the support surface working on the assumption that a patient will either lay directly on the top cover or with a single bedsheet. This assumption is often not adhered to in high acuity settings due to higher priority concerns such as safe patient handling (using a repositioning device), incontinence management (using an incontinence pad), or use of a prophylactic dressing to address existing pressure injuries. Moisture vapor often cannot be removed through these layers; only heat can pass through them, which is the value of localized cooling. No conventional localized cooling solution exists in the healthcare market for pressure injury prevention. The highest performing microclimate management system can remove 73 W/m2 with a single bedsheet on top. However, this is considered insufficient to achieve skin temperatures necessary to realize the benefits of localized cooling.

### SUMMARY OF THE DESCRIPTION

In general, one innovative aspect of the subject matter described herein can be embodied in a patient support system for a patient support apparatus including a deck with one or more deck sections, the patient support system may include a patient support being supported on the deck. The patient support system may include a thermal control element including a fluid flow path for directing fluid within the thermal control element. The thermal control element may include an inlet to receive fluid and direct the fluid to the fluid flow path, and may include an outlet to receive fluid from the fluid flow path. The patient support system may include a fluid mover operable to direct fluid to the inlet of the thermal control element and to receive fluid output from the outlet of the thermal control element. The patient support system may include a heat exchanger operable to change a temperature of fluid directed by the inlet of the thermal control element.

In general, one innovative aspect of the subject matter described herein can be embodied in a patient support system for a patient support apparatus including a deck with one or more deck sections. The patient support system may include a patient support being supported on the deck. The patient support system may include a thermal control element operable to affect a temperature of a portion of a patient without significantly affecting a core body temperature of the patient. The patient support system may include a fluid mover operable to direct fluid to the thermal control element and to receive fluid output from the thermal control element. The patient support system may include a heat exchanger operable to change a temperature of fluid directed to the thermal control element.

The foregoing and other aspects can each optionally include one or more of the following features, alone or in combination. In particular, one aspect includes all the following features in combination.

In one aspect, the deck may be movable relative to a base of the patient support system, where at least one of the one or more deck sections may be capable of moving relative to another of the one or more deck sections.

In one aspect, the thermal control element may include a fluid flow path for directing fluid within the thermal control element, where the thermal control element may include an inlet to receive fluid and direct the fluid to the fluid flow path, and where the thermal control element may include an outlet to receive fluid from the fluid flow path.

In one aspect, the thermal control element, the fluid mover, and the heat exchanger may form a closed loop system for circulating fluid to and from the thermal control element.

In one aspect, the heat exchanger may be operable to cool fluid prior to being supplied to the thermal control element.

In one aspect, the thermal control element may correspond to a pad provided within the patient support and arranged proximal to a zone of the patient support to provide localized cooling for a patient supported on the patient support.

In one aspect, the thermal control element may include a plurality of thermal control zones that are selectively enabled or disabled.

In one aspect, the thermal control element may include a plurality of selectable zones for which fluid flow is selectively enabled and disabled, whereby selectively enabling and disabling fluid flow may enable selective cooling with respect to each of the plurality of selectable zones.

In one aspect, the patient support system may include one or more valves operable to selectively control fluid flow through the plurality of selectable zones of the thermal control element.

In one aspect, a zone of the thermal control element may be selectively activated or deactivated based on selectively offloading of one or more inflatable pods located proximal to the zone of the thermal control element.

In general, one innovative aspect of the subject matter described herein can be embodied in a patient support system for a patient support apparatus including a deck with one or more deck sections. The patient support system may include a patient support being supported on the deck. The patient support system may include a thermal control element including a fluid flow path for directing fluid within the thermal control element. The thermal control element may include an inlet to receive fluid and direct the fluid to the fluid flow path, and may include an outlet to receive fluid from the fluid flow path. The thermal control element may include a plurality of selectable zones for which fluid flow is selectively enabled and disabled, whereby selectively enabling and disabling fluid flow enables selective cooling with respect to each of the plurality of selectable zones.

The patient support system may include a fluid mover operable to direct fluid to the inlet of the thermal control element and to receive fluid output from the outlet of the thermal control element. The patient support system may include a heat exchanger operable to change a temperature of fluid directed by the inlet of the thermal control element.

The patient support system may include a control system operable to direct selection of the plurality of selectable zones. The control system may be operable to direct selective enablement of a first selectable zone of the plurality of selectable zones based on a status of the patient support system.

The foregoing and other aspects can each optionally include one or more of the following features, alone or in combination. In particular, one aspect includes all the following features in combination.

In one aspect, the status of the patient support system may correspond to sensor information output from a sensor associated with a patient.

In one aspect, the sensor may be an interface pressure sensor mat capable of outputting information indicative of an interface pressure for one or more locations of the patient support.

In one aspect, the status of the patient support system may correspond to a turning bladder being on or off.

In one aspect, the thermal control element may be configured to cool a portion of a patient without significantly affecting a core body temperature of the patient.

In one aspect, the thermal control element may be configured to affect a core body temperature of a patient.

In one aspect, the thermal control element may include a plurality of fluid outlets.

In one aspect, the thermal control element may include a plurality of fluid inlets.

In one aspect, the heat exchanger may be operable to supply heated fluid to the thermal control element.

In one aspect, the thermal control element may be a non-disposable component of the patient support.

In one aspect, the fluid mover may be upstream of the heat exchanger, and where a fluid inlet of the thermal control element may receive fluid output from the heat exchanger via a supply conduit.

In one aspect, the heat exchanger may cool fluid received from the fluid mover, where the fluid mover may impart heat energy to fluid as it passes through the fluid mover.

In one aspect, the heat exchanger may provide enhanced cooling of fluid received from the fluid mover to increase cooling for the thermal control element.

In one aspect, the fluid mover may be downstream of the heat exchanger so that the fluid mover supplies fluid to the thermal control element via a supply conduit.

In one aspect, the fluid mover may be arranged to at least one of draw fluid from the thermal control element under vacuum and supply fluid to the thermal control element under pressure.

In one aspect, the patient support system may include a first flexible coupling configured to extend from a lower portion of the patient support to an upper portion of the patient support proximal to the thermal control element, where the first flexible coupling may be fluidly connected to the inlet of the thermal control element, and where the first flexible coupling may be operable to change in length in response to vertical and/or lateral movement of the thermal control element due to at least one of movement and weight of a patient on the thermal control element and movement of a component of the patient support system in response to control over the patient support system by a caregiver.

In one aspect, the first flexible coupling may be integral to the thermal control element.

In one aspect, the first flexible coupling may include a layer coupler and a conduit coupler, where the layer coupler may be connected to the inlet of the thermal control element, and where the conduit coupler may be connected to a supply conduit that is fluidly coupled to the heat exchanger.

In one aspect, the first flexible coupling may include a filler material disposed within a fluid flow path, and where the filler material may be porous and operable to maintain flow in the fluid flow path despite compression caused by compression of the first flexible coupling.

In one aspect, the patient support system may include a second flexible coupling configured to extend from the lower portion of the patient support to the upper portion of the patient support proximal to the thermal control element, where the second flexible coupling may be fluidly connected to the outlet of the thermal control element, and where the second flexible coupling may be connected to a return conduit that is fluidly coupled to the fluid mover.

In one aspect, the patient support system may include a second flexible coupling configured to extend from the lower portion of the patient support to the upper portion of the patient support proximal to the thermal control element, where the second flexible coupling may be fluidly connected to the outlet of the thermal control element, where the second flexible coupling may be operable to change in length in response to vertical and/or lateral movement of the thermal control element due to movement and weight of a patient on the thermal control element.

In one aspect, the second flexible coupling may include a filler material disposed within a fluid flow path, and where the filler material may be porous and operable to maintain flow in the fluid flow path despite compression caused by compression of the second flexible coupling.

In one aspect, the thermal control element may include a filler material disposed within a fluid flow path, where the filler material may be porous and operable to support separation between layers of the thermal control element for facilitating flow through the fluid flow path despite compression caused by weight of a patient on the thermal control element.

In one aspect, the patient support may include at least one inflatable bladder.

In one aspect, the at least one inflatable bladder may include first and second turn bladders operable to facilitate turning a patient disposed on the patient support.

In one aspect, the thermal control element may include first and second zones associated respectively with the first and second turn bladders, where the first zone may be activated or deactivated in response to activation of the first turn bladder to turn the patient, and where the second zone may be activated or deactivated in response to activation of the second turn bladder to turn the patient.

In one aspect, the first zone may be activated in response to activation of the first turn bladder to concentrate cooling for the patient in a turned position due to the change in pressure injury site potential caused by activation of the first turn bladder.

In one aspect, the first zone may be associated with the first turn bladder based on an increase in pressure proximal to the first zone caused by activation of the first turn bladder.

In one aspect, the fluid mover may be a compressor.

In one aspect, the thermal control element may include a plurality of openings through which the fluid is capable of escaping for cooling purposes relative to a patient.

In one aspect, the patient support may be a mattress.

In one aspect, the thermal control element may be disposed inside the patient support or outside the patient support.

In one aspect, the thermal control element may be provided within the patient support.

In one aspect, the thermal control element may be a thermal control layer within the patient support.

In one aspect, the thermal control element may be a pad within the patient support.

In one aspect, the thermal control element may facilitate flow of fluid within the patient support.

In one aspect, the fluid mover may be disposed outside the patient support.

In one aspect, the heat exchanger may be disposed outside the patient support.

In one aspect, the thermal control element may be a thermal control layer, where a plurality of additional layers of material may be disposed between the thermal control layer and a patient, and where a cooling capability of the thermal control layer in response to fluid flow may be operable to affect a temperature of the patient despite presence of the plurality of additional layers disposed between the thermal control layer and the patient.

In one aspect, fluid flow to the thermal control layer may be controlled to adjust a temperature of the patient toward a target temperature.

In one aspect, the patient support system may include a sensor associated with the patient and capable of transmitting sensor information indicative of the temperature of the patient, and where the fluid flow to the thermal control layer may be controlled based on the sensor information received from the sensor.

In one aspect, the fluid mover may be a blower, and where the heat exchanger may be a thermoelectric cooler.

Before the embodiments and aspects of the disclosure are explained in detail, it is to be understood that the disclosure is not limited to the details of operation or to the details of construction and the arrangement of the components set forth in the following description or illustrated in the drawings. The disclosure may be implemented in various other embodiments and aspects and of being practiced or being carried out in alternative ways not expressly disclosed herein. Also, it is to be understood that the phraseology and terminology used herein are for the purpose of description and should not be regarded as limiting. The use of "including" and "comprising" and variations thereof is meant to encompass the items listed thereafter and equivalents thereof as well as additional items and equivalents thereof. Further, enumeration may be used in the description of various embodiments and aspects. Unless otherwise expressly stated, the use of enumeration should not be construed as limiting one or more embodiments or aspects to any specific order or number of components. Nor should the use of enumeration be construed as excluding from the scope of an embodiment or aspect any additional steps or components that might be combined with or into the enumerated steps or components.

### DRAWINGS

Fig. 1 shows a patient support apparatus according to one aspect of the present disclosure.
Fig. 2 shows a patient support according to one aspect.
Fig. 3A shows an exploded view of the patient support in Fig. 2.
Fig. 3B shows a thermal control element according to one aspect.
Fig. 4 shows a control system for the patient support apparatus according to one aspect.
Fig. 5 shows a patient support according to one aspect.
Fig. 6 shows a top view of a patient support according to one aspect.
Fig. 7 shows a partial, sectional view of the patient support of Fig. 6.
Fig. 8 shows a partial, sectional, perspective view of the patient support of Fig. 6.
Fig. 9 shows a heat map of the patient support of Fig. 6.
Fig. 10 shows a plurality of layers for a patient support apparatus.
Fig. 11 shows dry heat flux performance of a thermal control element in conjunction with the patient support of Fig. 6.
Fig. 12 shows human skin temperature performance for persons in conjunction with a thermal control element and the patient support of Fig. 6.
Fig. 13 shows a patient support apparatus with a thermal control element (e.g., a thermal control layer) outside the patient support apparatus so that the thermal control element is placed in a clinical layer close to a patient.
Fig. 14 shows a thermal control element according to one aspect.
Fig. 15 shows a thermal control element affixed beneath an incontinence layer according to one aspect.
Fig. 16 shows a top view of a thermal control element with holes, openings, or apertures according to one aspect.
Fig. 17 shows a bottom view of a thermal control element according to one aspect.
Fig. 18 shows a top view of a thermal control element according to one aspect.
Fig. 19 shows a side-by-side view of thermal control elements according to multiple aspects.
Fig. 20 shows flow rate performance of thermal control elements, one with and one without an extended inlet, according to multiple aspects.
Fig. 21 shows a top view of a thermal control element with slits according to one aspect.
Fig. 22A shows a thermal control element with multizone control capabilities according to one aspect.
Fig. 22B shows a thermal control element according to one aspect.
Fig. 22C shows a thermal control element according to one aspect.
Fig. 23A shows a thermal control element according to one aspect.
Fig. 23B shows a thermal control element according to one aspect.
Fig. 23C shows a thermal control element according to one aspect.
Fig. 23D shows a thermal control element according to one aspect.
Fig. 23E shows a thermal control element according to one aspect.
Fig. 23F shows a thermal control element according to one aspect.
Fig. 23G shows a thermal control element according to one aspect.
Fig. 23H shows a thermal control element according to one aspect.
Fig. 23I shows a thermal control element according to one aspect.
Fig. 24 shows a thermal control element according to one aspect.
Fig. 25 shows a thermal control element according to one aspect.
Fig. 26A shows spacer fabric according to one aspect.
Fig. 26B shows spacer fabric according to one aspect.
Fig. 26C shows spacer fabric according to one aspect.
Fig. 27 shows performance of spacer fabric according to multiple aspects.
Fig. 28 shows a patient support apparatus according to one aspect.
Fig. 29 shows temperature history according to one aspect.
Fig. 30 shows a flexible coupling according to one aspect.
Fig. 31 shows a layer coupler according to one aspect.
Fig. 32 shows a conduit coupler according to one aspect.
Fig. 33 shows a flexible conduit according to one aspect.
Fig. 34A shows a layer coupler according to one aspect.
Fig. 34B shows a cross-section view of a layer coupler according to one aspect.
Fig. 35A shows a conduit coupler according to one aspect.
Fig. 35B shows a cross-section view of a conduit coupler according to one aspect.
Fig. 36A shows a layer coupler according to one aspect.
Fig. 36B shows a cross-section view of a layer coupler according to one aspect.
Fig. 36C shows the layer coupler according to one aspect.
Fig. 37A shows a conduit coupler according to one aspect.
Fig. 37B shows a cross-section view of a conduit coupler according to one aspect.
Fig. 37C shows the conduit coupler according to one aspect.
Fig. 38A shows a layer coupler according to one aspect.
Fig. 38B shows a cross-section view of a layer coupler according to one aspect.
Fig. 39A shows a conduit coupler according to one aspect.
Fig. 39B shows a cross-section view of a conduit coupler according to one aspect.
Fig. 40A shows a flexible coupling according to one aspect.
Fig. 40B shows a flexible coupling according to one aspect.
Fig. 41A shows a housing according to one aspect.
Fig. 41B shows a housing according to one aspect.
Fig. 41C shows a housing according to one aspect.
Fig. 42A shows a portion of the patient support apparatus according to one aspect.
Fig. 42B shows a portion of the patient support apparatus according to one aspect.
Fig. 43 shows a portion of the patient support apparatus according to one aspect.
Fig. 44 shows a portion of the patient support apparatus according to one aspect.
Fig. 45A shows a flexible coupling according to one aspect
Fig. 45B shows a flexible coupling according to one aspect.
Fig. 46A shows a flexible coupling according to one aspect.
Fig. 46B shows a flexible coupling according to one aspect.
Fig. 46C shows a flexible coupling according to one aspect.
Fig. 46D shows a flexible coupling according to one aspect.
Fig. 47A shows a flexible coupling according to one aspect.
Fig. 47B shows a flexible coupling according to one aspect.
Fig. 47C shows a flexible coupling according to one aspect.
Fig. 48 shows a flexible coupling according to one aspect.
Fig. 49 shows a tooling setup for manufacturing a fluid conduit according to one aspect.
Fig. 50 shows a flexible coupling according to one aspect.
Fig. 51 shows a flexible coupling according to one aspect.
Fig. 52 shows performance for operation of the fluid couplings and thermal control elements according to one aspect.
Fig. 53 shows a thermal control element and a flexible couplings according to one aspect.
Fig. 53A shows a top view of a portion of a patient support with a thermal control element according to one aspect.
Fig. 53B shows a bottom view of a portion of a patient support with a thermal control element according to one aspect.
Fig. 53C shows a bottom view of a portion of a patient support with a thermal control element according to one aspect with only one zone active.
Fig. 53D shows a top view of a portion of a patient support with a thermal control element according to one aspect with only one zone active.
Fig. 53E shows a bottom view of a portion of a patient support with a thermal control element according to one aspect.
Fig. 53F shows an internal view of a portion of a patient support with a thermal control element according to one aspect.
Fig. 53G shows representative fluid connections between portions of a patient support with a thermal control element according to one aspect.
Fig. 53H shows a representative cross section of the patient support in Fig. 53D.
Fig. 53I shows a representative cross section of the patient support in Fig. 53C.
Fig. 53J shows a representative cross section of the patient support in Fig. 53F.
Fig. 54 shows a patient support according to one aspect.
Fig. 55A shows a thermal control element according to one aspect.
Fig. 55B shows a thermal control element according to one aspect.
Fig. 55C shows a thermal control element according to one aspect.

### DESCRIPTION

A patient support system and a cooling system for the same is provided. In one aspect, the patient support system may include a closed loop cooling system operable to cool a patient without significantly affecting a core body temperature of the patient. In one aspect, the closed loop cooling system may be operable to affect the core body temperature of the patient.

In one aspect, the cooling system (and/or heating system) may be operable to remove sufficient heat to cool through multiple layers between the cooling system and the patient (and/or to add heat to affect a temperature of the patient). A pad localized cooling system, such as a thermal control element described herein, may be operable to remove enough heat necessary to cool the skin through six or more clinically relevant layers. Using the sweating guarded hot plate test (ANSI RESNA SS-1:2019 Section 4) to evaluate dry heat flux, one configuration achieved 446 W/m² heat removal of the thermal control element through a single bedsheet. This value is more than six times greater than the highest performing conventional product and just less than ten times greater than other commercially available products.

It is noted that, with additional layers, the dry heat flux performance decreases. Adding a repositioning device and incontinence pad may reduce performance to 247 W/m², with a second incontinence pad to 190 W/m², adding a sensor pad (e.g., a sensor 125 described herein) to 164 W/m², and adding a prophylactic dressing to 111 W/m². The addition of five layers may reduce the dry heat flux performance to 335 W/m². Based on estimates of human heat output between 50 - 100 W/m², a target of at least 100 W/m² dry heat flux performance may be provided.

To evaluate the pressure redistribution impact of the thermal control element, a S3I hemispherical indenter test (ANSI RESNA SS-1:2019 Section 6) was conducted. The test fixture consists of 61 pressure sensors comprising 10 rings of six sensors and one apex sensor to assess the pressure redistribution properties of a support surface. A support surface with and without the thermal control element was tested with the following layer configurations:
- Bed sheet only;
- Bed sheet + repositioning device + 1 incontinence pad (chux); and
- Bed sheet + repositioning device + 2 incontinence pads (chux).

Immersion performance for the thermal control element in one aspect decreased from baseline up to 4 mm across the layer configurations, but envelopment performance increased up to 4%. Apex pressure slightly decreased but the average ring pressure increased across the trials, equivalent to the addition of a repositioning device and one chux.

The thermal control element according to one aspect may be configured to cool a target region of the patient support. The thermal control element and related components (e.g., a heat exchanger and a fluid mover) may provide cooling within a focused area of the patient support (e.g., to provide sufficient sacral cooling on areas of highest pressure). Using a BodiTrack temperature mat, the cooling area of the system can be assessed. For instance, a bedsheet may be placed between the temperature mat and the mattress with a blanket on top before activating the cooling system. Measuring the temperature at peak cooling, the temperature distribution over the mattress top surface can be determined. The pad cooling system according to one aspect confined cooling closely within the cooling area between 9 - 16 °C.

With respect to a sacral region, the thermal control element may be constructed to provide a sufficient drop in sacral temperature to a target temperature. For instance, the thermal control element may reduce sacral temperature to the low end of target temperature (28 °C) through five layers plus the participants' clothes. In one aspect, volunteers taped a temperature probe to their sacral region using thermally conductive tape. They then laid on the mattress with the cooling system off and began recording the temperature. The sacral temperature increased for a set time duration until the cooling system was activated. Sacral temperature returned to baseline within 13 minutes of the pad cooling system activation and then achieved the target temperature within 1.5 - 2.5 hrs.

Heat may impact physiological conditions such as sweat rate (the source of skin moisture targeted by microclimate management) and metabolic demand. The layers between a patient and the support surface may not allow moisture to dissipate but heat can be conducted through the layers. Localized cooling works by cooling skin areas of highest pressure to 1) decrease the sweat rate and 2) decrease the metabolic demand of the skin. However, cooling too large of an area or cooling to a low enough temperature can decrease core body temperature and have adverse effects. An effective localized cooling system may substantially contain cooling within a specified region and achieve sufficiently dry heat flux removal to arrive at or be close to or maintain a target skin temperature through multiple clinical layers.

The pad localized cooling system (e.g., the thermal control element and supporting components, such as a heat exchanger and a fluid mover) may be capable of achieving sufficient heat removal to cool a patient's sacral skin temperature to a target temperature. A trade off with the system is a negative impact to the support surface pressure redistribution performance. For instance, if semi-rigid tubing conduits are utilized proximal to the patient, increased pressure may be a consideration. Such semi-rigid components proximal to the patient (e.g., for connection to the thermal control element) may cause such components to press back into a person when they submerge into the patient support. A flexible coupling, as described herein, may be utilized to mitigate against this pressure distribution concern, if present with the semi-rigid components.

Performance criteria considered for construction of the thermal control element and supporting components may be balanced so that sufficient volumetric air flow for thermal performance is provided while decreasing the adverse pressure redistribution impact of the system.

In one aspect, a localized cooling system may be provided that operates on two principles: 1) maintaining as close to a closed loop system as possible to continually cool the same volume of air without generating condensation and 2) circulating the conditioned air as close to the patient's skin as possible. This system, including one or more of the thermal control element aspects and associated components, described herein may include one or more of the following subsystems:
- Pad or thermal control element → contain fluid with a region close to the patient, concentrate cooling in the region of highest pressure, and limit pressure redistribution impact
- Collapsing air channels → maintain closed loop airflow, mitigate the pressure redistribution impact
- Cold-side fluid mover → provide sufficient fluid flow, limit the heat added to the fluid flow
- Thermoelectric (TE) cooler → remove heat from the airflow, limit the size to fit within an enclosure
- TE cooler enclosure → limit the pressure drop within the system, induce turbulent flow within the TE fins, provide even airflow distribution through the TE fins
- Hot-side fans → increase airflow across fin, limit the heat added to the airflow
- Exhaust → maintain a fluid impermeable barrier, maximize the airflow rate out of the mattress
- Fluid inlet → maintain a fluid impermeable barrier, maximize the airflow rate into the support surface
- Desiccant → decrease the condensation generated in the system, limit the effect on airflow rate
- Non-collapsing air channels → limit the effect on airflow, prevent external condensation accumulation and heat added to the airflow

Each of the listed subsystems may perform one or more of the associated functions listed above. The thermal control element according to one aspect may be configured to provide one or more of the following:
- Cooling may provide the closest proximity to the patient while still within the patient support.
- Channels or fluid flow paths in the thermal control element may include spacer fabric embedded to keep the channels open with the patient load applied.
- Radially configured supply channels or fluid flow paths may concentrate cool air in the target region center.
- Central cooling supply delivery allows for a more even distribution of cool air.

Location of placement of the thermal control element may be inside or outside the patient support, and/or in between each patient layer but not directly against the patient's skin. The thermal control element may form a localized cooling system incorporated beneath or within an incontinence management system. The primary challenge with the localized cooling system within the patient support is the lower temperatures needed to compensate for the thermal resistance of the remaining external layers.

Using thermal conductivity measurements gathered from external testing, the thermal resistance of aspects of the patient support may be determined, including for a bed sheet, repositioning device, and an incontinence pad. If a person's heat generation is 50 W/m², the thermal resistance of a bed sheet, repositioning device, and incontinence pad is 1.5 °C. Therefore, to cool the skin to 32 °C a cooling device beneath these layers may be configured to produce a surface temperature of 30.5 °C to compensate for the 1.5 °C thermal resistance. If a person's heat generation is 100 W/m², the thermal resistance of these layers may be 3.0 °C, the cooling device may be configured to target 29 °C to maintain 32 °C at the skin. The more layers added, the lower the temperature the cooling device may be configured to achieve to compensate. This further creates a challenge for the size of the cooling region.

Cooling region size may be large enough to cool the areas of highest pressure for a target patient population but small enough to not significantly drop a patient's core body temperature. The location of cooling may also change based on frame head of bed angle, patient turns, and patient migration. To accommodate the variation in these criteria, a multizone activation aspect for the thermal control element may be provided, for user selection or automatic activation based on one or more of the following variables:
- Patient size
- Patient weight
- Patient core body temperature
- Patient skin temperature
- Patient location on the mattress
- Frame head of bed angle
- Patient turn position
- Location of existing pressure injuries or other wounds
- Presence of incontinence
- Pod offloading functionality engagement

Cooling, while beneficial for pressure injury prevention of skin tissue under load, may have adverse effects for existing pressure injuries or wounds. Blood flow slows with cooling due to capillary constriction. Skin regions with no loading would benefit from not cooling to limit the associated decrease in blood flow. Multizone cooling would allow for deactivation in regions undergoing pod offloading as described in PCT Appl. No. PCT/US2024/030233, filed May 20, 2024, to Derenne et al. and entitled INFLATABLE MATTRESS AND CONTROL THEREOF-the disclosure of which is hereby incorporated by reference in its entirety.

Incontinent episodes increase the thermal conductivity of the skin in contact with the moisture. Detecting the presence of incontinence and deactivating the associated multizone cooling region may mitigate the risk of decreasing the patient's core body temperature.

The fluid described herein may be any type of fluid, including, for instance, air or water or a refrigerant.

### I. Overview

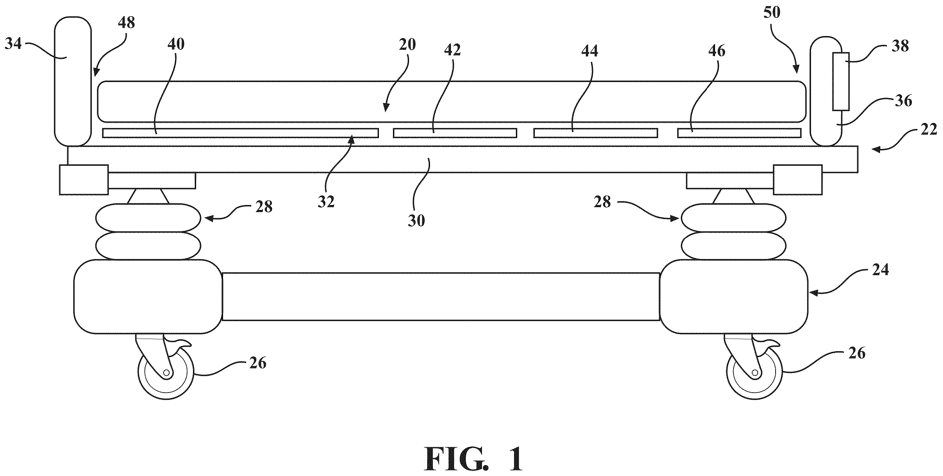

Fig. 1 illustrates a patient support system 21 including a patient support 20 according to one aspect of the present disclosure. In the example of Fig. 1, the patient support 20 is a mattress. However, it will be understood that the patient support 20 may take on other manifestations, such as cushions, pads, etc. Indeed, in one aspect, the patient support 20 may be a cushion or pad for a chair, such as a wheelchair or a stationary chair. In general, the patient support 20 may be utilized wherever and whenever a patient is to be supported on a surface and it is desirable to reduce interface pressures experienced by the patient while positioned on the patient support 20.

In Fig. 1, the patient support 20 is supported on a patient support apparatus 22 that, in this particular configuration, is a bed. The patient support apparatus 22 may take on other forms besides beds, such as, but not limited to cots, stretchers, operating tables, gurneys, and the like. The patient support apparatus 22 may be a conventional support apparatus that is commercially available and that merely provides a supporting function for the patient support 20. In other aspects, the patient support apparatus 22 may include one or more controls that are integrated therein and which are used in controlling the operation of the patient support 20, as will be discussed in greater detail herein.

As shown in Fig. 1, the patient support apparatus 22 includes a base 24 having a plurality of wheels 26, a pair of elevation adjustment mechanisms 28 supported on the base 24, a frame or litter 30 supported on the elevation adjustment mechanisms 28, and a patient support deck 32 supported on the frame 30. The patient support apparatus 22 may also include a headboard 34 and a footboard 36. Either, or both, of the headboard 34 and the footboard 36 may be removable from the frame 30 and may include one or more electrical connectors for establishing electrical communication between electronic components on or in the footboard 36 and/or the headboard 34 and other electronic components supported on or in the frame 30. Such electrical connector(s) may include any one or more of the connectors disclosed in commonly assigned U.S. Pat. 9,306,322, issued April 5, 2016, to Krishna Bhimavarapu and entitled PATIENT SUPPORT APPARATUS CONNECTORS, the disclosure of which is incorporated herein by reference in its entirety. Other types of connectors may also be used.

In one aspect, electrical connectors may be provided for establishing an electrical link between the patient support 20 and a user interface 38 that is positioned on, or integrated into, the footboard 36 or another aspect of the patient support apparatus 22. The user interface 38 may take on a variety of different forms, such as, but not limited to, a touch screen, a Liquid Crystal Display (LCD), a plurality of buttons, switches, knobs, or the like, or any combination of these components. The user interface 38 may allow a user to control the operation of the patient support 20. The electrical connection between the user interface 38 and the patient support 20 may take on different forms, including a direct electrical cable that runs from the footboard 36 to the patient support 20. As another example, the footboard 36 may include electrical connectors that electrically couple the user interface 38 to circuitry supported on the frame 30. This circuitry may be in further electrical communication with a port (not shown) to which an electrical cable from the patient support 20 may be inserted, thereby establishing an electrical link between the user interface 38 and the patient support 20. In yet another example, communication between the user interface 38 and the patient support 20 may be entirely wireless. A further example of such wireless communication is disclosed in commonly assigned U.S. Pat. 9,966,997, issued May 28, 2018, to Michael Hayes et al. and entitled COMMUNICATION SYSTEMS FOR PATIENT SUPPORT APPARATUSES, the complete disclosure of which is hereby incorporated herein by reference.

The elevation adjustment mechanisms 28 are adapted to raise and lower the frame 30 with respect to the base 24. The elevation adjustment mechanisms 28 may be implemented as hydraulic actuators, electric actuators, or any other suitable device for raising and lowering the frame 30 with respect to the base 24. In Fig. 1, the elevation adjustment mechanisms 28 are depicted to be operable independently so that the orientation of the frame 30 with respect to the base 24 may also be adjusted. This may allow the patient support apparatus 22 to tilt a patient supported on the patient support 20 to either the Trendelenburg orientation, or the reverse Trendelenburg orientation.

The frame 30 may provide a structure for supporting the patient support deck 32, the headboard 34, and the footboard 36. The patient support deck 32 may provide a surface on which the patient support 20 can be positioned so that a patient may lie and/or sit thereon. The patient support deck 32 may be made of a plurality of sections, some of which may be pivotable about generally horizontal pivot axes-although it is to be understood that the patient support deck 32 may be configured differently without a plurality of sections (e.g., one section). In the configuration shown in FIG. 1, the patient support deck 32 includes a head or back section 40, a seat section 42, a thigh section 44, and a foot section 46. In other configurations, the patient support deck 32 may include fewer or greater numbers of sections. The head section 40, which is also sometimes referred to as a Fowler section or back section, may be pivotable between a generally horizontal orientation (shown in Fig. 1) and a plurality of raised positions (not shown in Fig. 1). The thigh section 44 and the foot section 46 may also be pivotable about horizontal pivot axes.

The general construction of any of the base 24, the elevation adjustment mechanisms 28, the frame 30, the patient support deck 32, the headboard 34, and/or the footboard 36 may take on any known or conventional design, such as, for example, that disclosed in commonly assigned, U.S. Pat. No. 7,690,059 issued April 6, 2010, to Lemire et al., and entitled HOSPITAL BED, the complete disclosure of which is incorporated herein by reference; or that disclosed in U.S. Pat. 8,689,376, issued April 8, 2014, to Becker et al. and entitled PATIENT HANDLING DEVICE INCLUDING LOCAL STATUS INDICATION, ONE-TOUCH FOWLER ANGLE ADJUSTMENT, AND POWER-ON ALARM CONFIGURATION, the complete disclosure of which is also hereby incorporated herein by reference. The construction of any of the base 24, the elevation adjustment mechanisms 28, the frame 30, the patient support deck 32, the headboard 34, and/or the footboard 36 may also take on forms different from what is disclosed in the aforementioned patent and patent publication.

In some aspects, the operation of the patient support 20 may be based at least partially upon sensor data that originates from sensors integrated into the patient support apparatus 22, while in other aspects, the patient support 20 may operate solely on sensor data originating from sensors positioned internally inside of the patient support 20. For those aspects in which the patient support 20 uses sensor data from the patient support apparatus 22, such sensor data may include angle data and/or weight data. More specifically, the patient support apparatus 22, in some aspects, may include one or more angle sensors that detect the angular orientation (with respect to horizontal) of the frame 30, as well as one or more angle sensors that detect the angular orientation (with respect to horizontal) of one or more of the sections of the support deck 32. Still further, the patient support apparatus 22, in some aspects, may include a load cell system that detects patient weight and/or a center of gravity of a patient positioned on the patient support 20. One such load cell system that may be used in patient support apparatus 22 is disclosed in commonly assigned U.S. Pat. No. 5,276,432 issued January 4, 1994, to Travis, and entitled PATIENT EXIT DETECTION MECHANISM FOR HOSPITAL BED, the complete disclosure of which is incorporated herein by reference. Other load cell systems may also be used. Regardless of the specific load cell system used, the patient support apparatus 22 may communicate any one or more of patient weight, patient center of gravity, the angular orientation of the frame 30, and/or the angular orientation of one or more of the deck sections 40, 42, 44, 46 to the patient support 20, which may use this data in manners discussed in further detail herein. At least one or more of the deck sections 40, 42, 44, 46 may be movable relative to another of the one or more deck sections 40, 42, 44, 46. For instance, the angular orientation of the head section 40 may be raised or tilted relative to the seat section 42. As another example, additionally, or alternatively, the foot section 46 may be raised or tilted relative to the seat section 42. The one or more deck sections 40, 42, 44, 46 may be movable relative to the base 24 in response to articulation of the frame 30 or aspects thereof. For instance, the head section 40 may be tilted upward in response to raising of the head end of the frame 30 upward relative to the base 24.

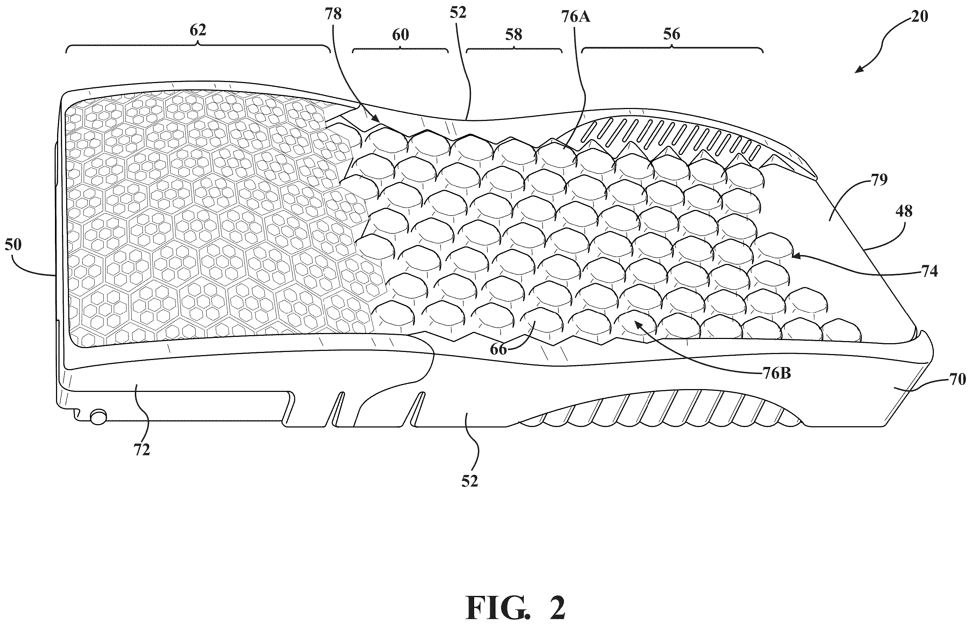

Turning to Fig. 2, the patient support 20 may further include a back zone 56, a thigh zone 60, and a foot zone 62. The back zone 56 may include a head zone or pillow zone. The physical boundaries of each of the zones may be modified from that shown, as well as the number of locations of each zone. In the configuration of Fig. 2, the back zone 56 is positioned such that it will generally be aligned with the head or back section 40 of the patient support apparatus 22 when the patient support 20 is positioned on the support deck 32. Similarly, the seat zone 58 will be generally aligned with the seat section 42, the thigh zone 60 will be generally aligned with the thigh section 44, and the foot zone 62 will be generally aligned with the foot section 46. Such alignment, however, is not necessary. Indeed, the patient support 20 may be used on patient support apparatuses 22 in which the support deck 32 has no individual sections, or which has a fewer or greater number than the four shown in Fig. 1.

The seat zone 58, as depicted shown in Fig. 2, is subdivided into right and left sides. That is, the seat zone 58 includes a right seat zone and a left seat zone. Each of the seat zones may define regions in which hermetically isolated inflatable bladders (also described herein or referred to as pods or inflatable pods) may be positioned so that the inflation level corresponding to the right seat zone can be controlled and/or set independently of the inflation level corresponding to the left seat zone. In this manner, if a patient is lying on his or her side or is otherwise positioned closer to one side 52 than the other, zones of the seat zone 58 can be set, at least in some configurations, to different inflation levels. Alternatively, the inflation levels may be set for zones of the seat zone 58 differently in situations where the patient is positioned more toward the middle of the patient support 20. In alternative configurations, the seat zone 58 may be a single zone that does not have separate subdivisions between the right and left side, but rather is inflatable and deflatable in a unitary manner. In still other alternative configurations, one or more of the other zones 56, 60, and/or 62 may be subdivided into left and right sub-zones, or subdivided in still other manners.

Fig. 2 shows the patient support 20 with its outer cover removed, exposing a plurality of inflatable pods 66, as well as a pillow bladder 79, a foam crib 70 that supports the pods 66, and molded foot end cushioning 72. Foam cushioning 72 is not inflatable, but instead provides cushioned support to a patient's feet through its soft pliability. In an alternative configuration, the foam crib 70 may be absent entirely or in one or more areas of the periphery of the patient support 20. In this configuration, in place of the foam crib 70, one or more inflatable pods 66 may be provided. As an example, the one or more inflatable pods 66 may be provided at the periphery in areas where the foam crib 70 is provided in the configuration depicted in Fig. 2. Further examples involving control over one or more of the inflatable pods 66, such as offloading one or more of the inflatable pods 66, are described in PCT Appl. No. PCT/US2024/030233, filed May 20, 2024, to Derenne et al. and entitled INFLATABLE MATTRESS AND CONTROL THEREOF and U.S. Appl. No. 63/723,340, filed November 21, 2024, to Derenne et al. and entitled INFLATABLE MATTRESS AND CONTROL THEREOF-the disclosures of which are hereby incorporated by reference in their entirety.

In one configuration, the inflatable pods 66 are fluidly coupled together in a manner that corresponds to zones 60, 62. For instance, all of the inflatable pods 66 within the back zone 56 may inflate and deflate together, and can be inflated and deflated separately from the inflatable pods 66 in any of the other zones. Similarly, all of the inflatable pods 66 in the right seat zone of the zone 58, all of the inflatable pods 66 in the left seat zone of the zone 58, as well as all of the inflatable pods 66 in the thigh zone 60, are respectively able to be inflated and deflated together, as well as separately from the inflatable pods 66 in the other zones. Thus, the inflatable pods 66 in the back zone 56 collectively define a back bladder 74, the inflatable pods 66 in the right seat zone of the seat zone 58 collectively define a right seat bladder *76a,* the inflatable pods 66 in the left seat zone of the seat zone 58 collectively define a left seat bladder 76b, and the inflatable pods 66 in the thigh zone 60 collectively define a thigh zone bladder 78. It is to be understood that the bladders 74, 76*a,* 76*b,* and/or 78 can be implemented, in alternative configurations, in manners other than pods, such as, but not limited to, elongated bladders, flat bladders, can-shaped bladders, or still other shapes.

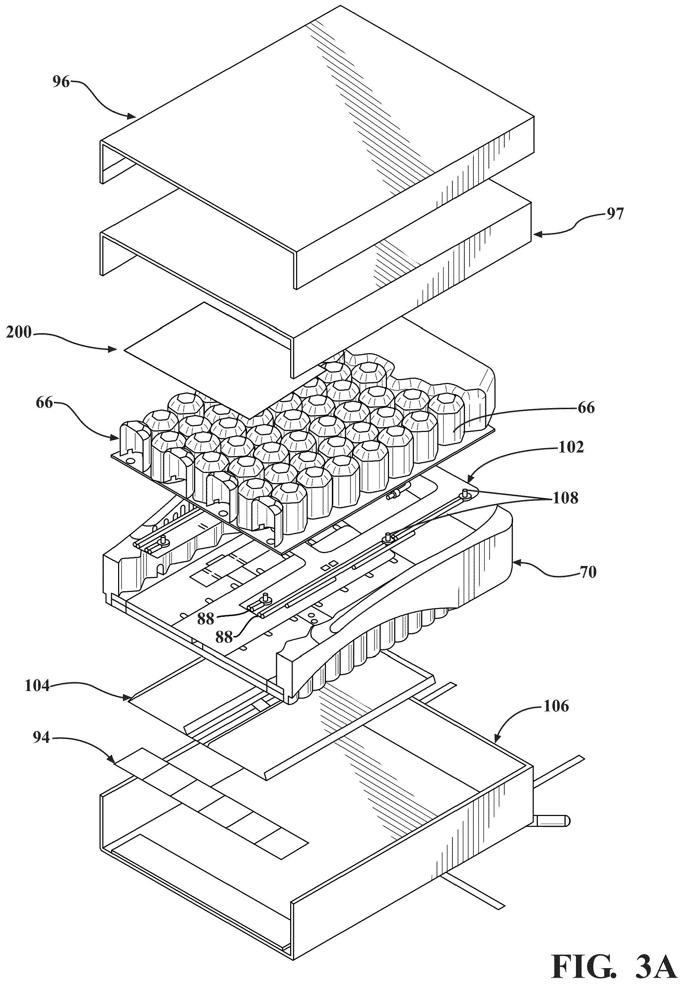

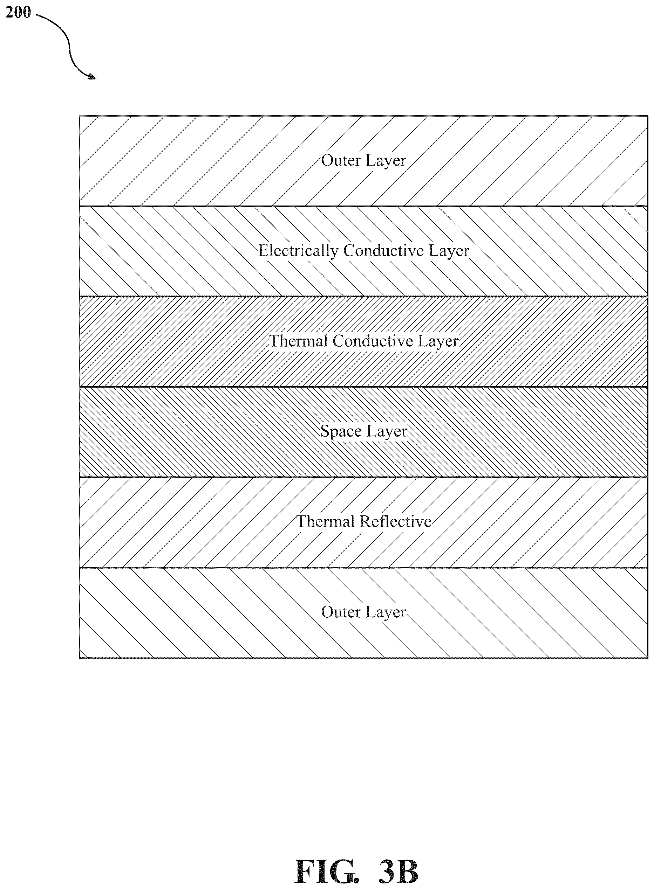

In Fig. 3, various components of the patient support 20 are shown in the back zone 56 and the seat zone 58 of Fig. 2. The patient support 20 includes a top cover 96, a fire barrier layer 97, a thermal control element 200, the inflatable pods 66, a fabric manifold 102, a foam crib 70, a plurality of turning bladders 104, sensors 94, and a bottom cover 106. The top cover 96 may be made of any conventional material used in the manufacture of hospital mattresses, such as, but not limited to, a knit polyester, and/or a polyurethane. The top cover 96 in Fig. 3 is the uppermost layer of the patient support 20-although it is to be understood that the uppermost layer may be different depending on the configuration of the patient support 20. Likewise, the fire barrier layer 97 and the thermal control element 200 may form intermediate layers between the uppermost layer and the inflatable pods 66. One or more or all of the layers may be absent in alternative configurations.

The fire barrier 97 may be positioned underneath the top cover 96 (or an uppermost layer) and may be made of any suitable material that resists the spread of fire. Such materials may vary. In one configuration, the fire barrier 97 may be made of, or include, Kevlar^{®} (poly-paraphenylene terephthalamide), or other brands of para-aramid synthetic fibers. Other materials may alternatively be used.

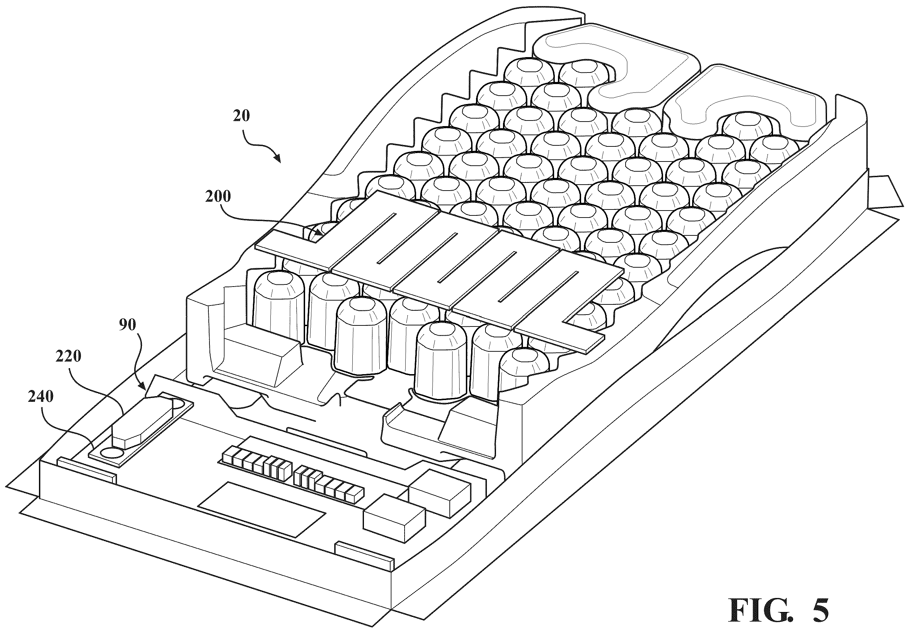

In one configuration, the top cover 96, or an intermediate layer, or a combination thereof, may include one or more sensors, such as a temperature sensor and/or interface sensors 125 (depicted in Fig. 5) operable to detect an interface pressure between the patient and the patient support 20. Additionally, or alternatively, one or more sensors may be provided above the top cover 96. The interface sensors may provide sensor feedback to a mattress control assembly 90, which may use the sensor feedback as a basis for controlling an inflation state of the inflatable pods 66, or groups thereof. The interface sensor may provide interface pressure sensor information corresponding to an interface pressure for one or more locations of the patient support.

The inflatable pods 66, as described herein, may be inflated and deflated in groups (e.g., zones 56, 58, and 60) under the control of a mattress control assembly 90 depicted in Fig. 2 and its associated control circuitry. The fluid connections between the inflatable pods 66 and the mattress control assembly 90 may be established by a plurality of hoses 88 that run between the mattress control assembly 90 and various of the inflatable pods 66. The hoses 88 are housed within the fabric manifold 102. The hoses 88 each include one or more connectors 108 for fluidly connecting the hose to one or more of the inflatable pods 66.

The mattress control assembly 90 may be provided within an enclosure provided proximal to the foot end 50 of the patient support 20. The mattress control assembly 90 may be provided beneath the foot zone 62 within an enclosure. It is to be understood that the mattress control assembly 90 may be configured and positioned differently depending on the application.

The mattress control assembly 90, as described herein, may include more than one air mover, such as an air pump, blower, pressure vessel, and another source of fluid (e.g., air) that may be supplied to the hoses 88 for delivery to the inflatable pods 66 and/or the turn bladders 104.

Additionally, the mattress control assembly 90 may be operable to remove fluid from the turn bladders 104 and/or the inflatable pods 66 via the hoses 88. In this way, with the capability to supply and remove fluid, the mattress control assembly 90 may selectively inflate and deflate the inflatable pods 66 in groups. In an alternative configuration, the mattress control assembly 90 may selectively inflate and deflate one or more of the turn bladders 104 and the inflatable pods 66, individually. As described herein, the mattress control assembly 90 may be operable to determine an inflation state of the turn bladders 104 and the inflatable pods 66 and/or groups thereof, and to direct a change in the inflation state based on at least one of sensor data and a directive from a caregiver provided via the user interface 38.

The turn bladders 104 may be positioned underneath the foam crib 70 and may be used to help turn a patient positioned on top of the patient support 20. To that end, the turn bladders 104 may extend generally longitudinally in a direction from the head end 48 to the foot end 50 and are each separately and independently inflatable and deflatable. The inflation of the turn bladders 104 may be controlled by the mattress control assembly 90 and its associated circuitry, as described herein.

The mattress control assembly 90, as described herein, may be operable to supply fluid to the thermal control element 200 in order to affect a temperature of the patient support 20. In one aspect, the supply of fluid may be conducted in a closed loop manner so that fluid is supplied to and received from the thermal control element 200. Thermal sensors may be incorporated within or beneath top cover 96. Temperature information may be provided to a control system to maintain thermal performance setpoints determined for clinical treatment, potentially optimal clinical treatment.

### II. Control System

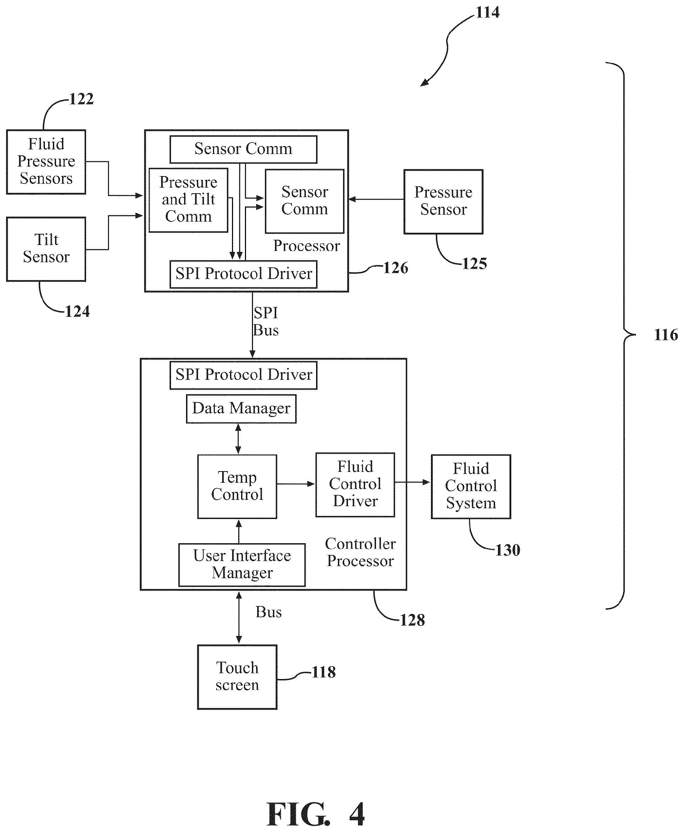

Fig. 4 shows one configuration of a control system 114 of the patient support system 21 that may be implemented to control the patient support 20 in the manner described herein. Other types, arrangements, and/or configurations of control systems may alternatively be used. The patient support system 21 may include a control system 114, a user interface 118, and a plurality of sensor systems, such as a tilt sensor 124, a moisture sensor with respect to the patient support surface for detecting moisture proximal to the patient, a temperature sensor for detecting temperature proximal to the patient, an air pressure sensor 122, and interface pressure sensor 125. It is to be understood that one or more of these aspects may be absent from implementations of the control system 114, such that the control system 114 may include a subset of the described aspects. An example sensor is described in U.S. Appl. No. 63/686,231, filed August 23, 2024, to Alvarez et al. and entitled MONITOR DEVICE-the disclosure of which is hereby incorporated by reference in its entirety. An example system utilizing an interface pressure sensor system is described in PCT Appl. No. PCT/US2024/030233, filed May 20, 2024, to Derenne et al. and entitled INFLATABLE MATTRESS AND CONTROL THEREOF-the disclosure of which is hereby incorporated by reference in its entirety.

Based on sensor information from the one or more sensors described herein (or any other type of sensor internal or external to the control system 114), the control system 114 may direct operation of one or more components of the patient support 20, 20'. For instance, one or more inputs provided as sensor information may correspond to but are not limited to one or more of the following: skin sensor data (e.g., temperature and/or humidity) from a sensor associated with the patient's skin, a core body temperature from a sensor associated with the patient, and temperature sensor information from a sensor associated with the thermal control element 200, and interface pressure sensor information from an interface pressure sensor 125 (which may be used as a basis for determining location information for areas of higher or lower interface pressures relative to adjacent areas [e.g., a heat map]). The control system 114 may direct operation of one or more outputs based on the inputs, including one or more but not limited to the following: one or more operational aspects of the heat exchanger 220 (e.g., cooling control), one or more operational aspects of the fluid mover 240 (e.g., a flow rate), and one or more operational aspects of the thermal control element 200 (e.g., fluid flow within the thermal control element 200 and/or enabling and/or disabling of one or more selectable zones). As an example, the control system 114 may direct enabling and/or disabling of the one or more selectable zones of the thermal control element 200 based on location information determined from the interface pressure sensor 125 output, such that one or more of the selectable zones may be enabled and/or disabled based on location information indicative of areas associated with the one or more selectable zones having higher or lower areas of interface pressure (relative to adjacent areas).

The user interface 118 may be the same as user interface 38, discussed above, which is incorporated into the footboard 36 of the patient support apparatus 22, or it may be a stand-alone user interface. Such stand-alone user interfaces may include user interfaces that are incorporated into pedestals that may be removable mounted on patient beds, such as the patient support apparatus 22. In the configuration shown in Fig. 4, the user interface 118 is a touch screen. It is to be understood that other types of user interfaces may be used, including buttons, switches, knobs, lights, and/or displays.

The control system 114 may be in electrical communication with both the user interface 118 as well as a plurality of air pressure sensors 122 and, in Fig. 4, one or more tilt sensors 124. The control system 114 in one configuration may be coupled to one or more interface pressure sensors 125, which as discussed herein may be operable to detect an interface pressure between the patient and the patient support 20. Additional sensors, such as a temperature sensor or a moisture sensor, or both may be coupled to the control system 114. Additionally, or alternatively, one or more sensors described herein may be absent from the control system 114.

In one aspect, one or more of the sensors may be separate from the control system 114 but capable of communicating information to the control system 114. For instance, a temperature sensor (and/or another type of sensor) may be associated with the patient, such as a sensor patch adhered to the patient's skin, and this sensor may be configured to communicate temperature information (and/or another type of sensor information) to the control system 114. Examples of such a sensor system are described in U.S. Pat. Appl. No. 63/686,231, entitled MONITOR DEVICE, filed August 23, 2024, to Alvarez et al.-the disclosure of which is incorporated by reference herein in its entirety. The control system 114 may adjust operation of the patient support 20 based on the sensor information received from the one or more sensors separate from the control system 114. For instance, the control system 114 may direct operational aspects of the patient support system 21, including fluid flow through the heat exchanger 220, fluid mover 240, and thermal control element 200 based on a target temperature for the patient and a sensed temperature for the patient that is communicated as part of the sensor information received from the one or more sensors separate from the control system 114 (e.g., a sensor patch adhered to the patient or proximal to the patient's skin).

The air pressure sensors 122 may measure the current air pressure inside one or more of the turn bladders 104 and the inflatable bladders 66 of the patient support 20 (e.g., back bladder 74, seat bladders 76a and 76b, thigh bladder 78, and pillow bladder 79). Each of these inflatable bladders 66 generally corresponds to zones 56, 58, 60, and 64, respectively.

Tilt sensors 124 measure the angular orientation of one or more portions of the patient support 20, and/or they measure the entire angular orientation of the patient support 20. In some aspects, as was discussed previously, tilt sensors 124 are omitted and the patient support 20 instead receives tilt data from one or more angle sensors that are incorporated into the patient support apparatus 22. In still other aspects, the patient support 20 is implemented without any tilt sensors 124, and without receiving any tilt data from the patient support apparatus 22.

The control system 114 in Fig. 4 includes two separate circuit boards: a sensor circuit board 126 and a main control circuit board 128. The sensor circuit board 126 may receive the electrical signals from all of the various sensors and oversees the operation of these sensors (e.g., air pressure sensors 122, tilt sensors 124, interface pressure sensors 125, a temperature sensor, and a moisture sensor). The data gathered from one or more of these various sensors may be forwarded from the sensor circuit board 126 to the main control circuit board 128, and may be used as a basis for controlling supply of fluid to an inflatable bladder of the patient support 20, such as one or more of the turn bladders 104 and the inflatable bladders 66.

In one configuration, this data from the one or more sensors may be communicated via a serial peripheral interface (SPI) bus, although it is to be understood that other buses may be used for this purpose. The main circuit board 128 may be programmed, or otherwise configured, to carry out the control algorithms described herein. Generally speaking, the main circuit board 128 may determine suitable inflation levels (e.g., a desired air pressure) for all of the various bladders and controls, valves, air movers, and other aspects to implement and maintain those suitable inflation levels.

As shown in Fig. 4, each circuit board 126 and 128 includes a processor, which may be a microprocessor or a microcontroller. Indeed, each circuit board 126 and 128 may include any electrical component, or group of electrical components, that are capable of carrying out the algorithms described herein. In many configurations, the circuit boards 126 and 128 may be microprocessor-based, although not all such configurations may utilize a microprocessor. In general, the circuit boards 126 and 128 may include any one or more microprocessors, microcontrollers, field programmable gate arrays, systems on a chip, volatile or nonvolatile memory, discrete circuitry, and/or other hardware, software, or firmware that is capable of carrying out the functions described herein. Such components may be physically configured in any suitable manner, such as by mounting them to one or more circuit boards, or arranging them in other manners, whether combined into a single unit or distributed across multiple units. It is to be further understood that the control system 114 may be implemented in different forms from the two boards 126 and 128 illustrated in Fig. 4. Such variations may include combining the functions of both boards 126 and 128 onto a single board, or further distributing the functions of these boards onto more than the two boards 126 and 128 shown in Fig. 4.

### III. Temperature Control System and Fluid Control System

In Fig. 4, the control system 114 of the patient support system 21 includes a fluid control system 130 (e.g., a fluid supply and return system) operably coupled to the main circuit board 128. The main circuit board 128 may include a fluid control driver configured to direct operation of the fluid control system 130 based on directives from a temperature control system implemented by the main control board 128-although control over the fluid control system 130 may be implemented differently. For instance, the fluid control system 130 may include a control system operable to control aspects of the fluid control system 130 independently of the main circuit board 128 and/or based on directives from the main circuit board 128 or another component of the patient support 20.

In one aspect, by way of electrical signals sent to the fluid control system 130, the main control board 128 may be able to control supply of fluid to the thermal control element 200. The fluid control system 130 may be operable to direct heat exchange with respect to fluid supplied to and returned from the thermal control element 200.

The fluid control system 130 may be operably coupled to a heat exchanger 220, such as a thermoelectric cooling system, which may form at least a portion of the patient support system 21 for the patient support 20.

One or more aspects of the fluid control system 130 and/or the heat exchanger 220 may be incorporated into the mattress control assembly 90. For instance, as described, the fluid control system 130 may include driver circuitry operable to control the heat exchanger 220 and to control operation of a fluid mover 240-these components or a subset thereof may be provided in the mattress control assembly (or system) 90 proximal to the foot end 50 of the patient support 20. Optionally, one or more components of the fluid control system 130 may be external to the mattress control assembly 90, and further optionally one or more of such components may be external to the patient support 20. For instance, the heat exchanger 220 and/or the fluid mover 240 may be external to the mattress control assembly 90 and optionally external to the patient support 20.

In Fig. 5, the mattress control assembly 90 is depicted without a cover assembly so that internal components are visible. Additionally, the patient support 20 is shown with a thermal control element 200 according to one embodiment but without supply and return fluid conduits for purposes of discussion.

The fluid mover 240, in one aspect, may correspond to a pump or compressor operable to draw air from the thermal control element 200 via one or more fluid conduits (not shown in Fig. 5) and push air through the heat exchanger 220 back to the thermal control element 200. In this way, the fluid mover 240, the heat exchanger 220, and the thermal control element 200 may form a closed loop system of fluid flow that is operable to cool and/or heat the thermal control element 200 to affect a temperature associated with the patient (e.g., to affect a skin temperature without significantly affecting a core temperature of the patient).

In operation, the main control board 128 may direct the fluid mover 240 to move fluid through the heat exchanger 220 and the thermal control element 200. Supply of fluid through fluid mover 240, heat exchanger 220, and the thermal control element 200 may be provided according to one or more criteria, such as for a predetermined period of time or until a sensor (e.g., a temperature sensor) detects a temperature associated with the patient.

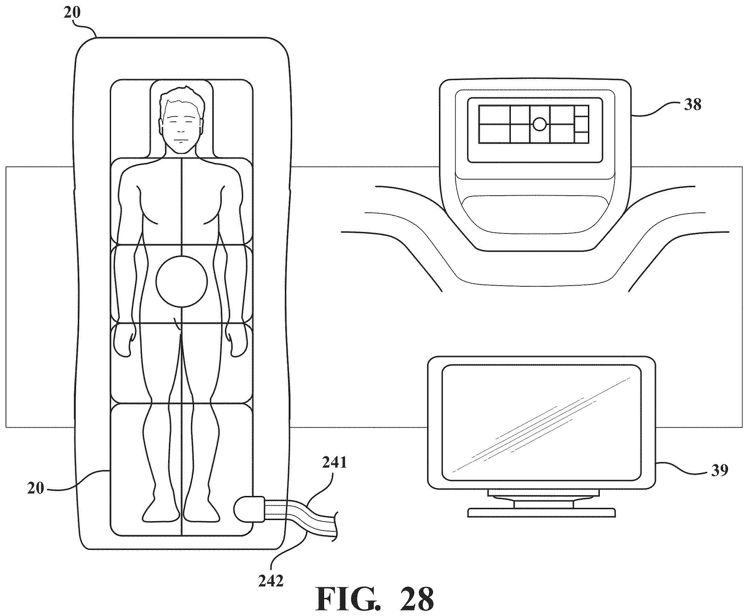

Fig. 28 depicts portions of the control system for the thermal control element 200 according to one aspect. In Fig. 28, the thermal control element 200 along with a supply conduit 242 and a return conduit 241 are depicted for a patient support in conjunction with the user interface 38. A caretaker, via the user interface 38, may control operation of the thermal control element 200 (e.g., supply of fluid from the heat exchanger 220 and the fluid mover 240) for affecting a temperature of a support surface on which the patient is disposed. This way, the thermal control element 200 under control of the caretaker may provide localized heating and or cooling to promote healing and comfort for the patient. The user interface 38 may provide status or information pertaining to control or operation of the thermal control element 200.

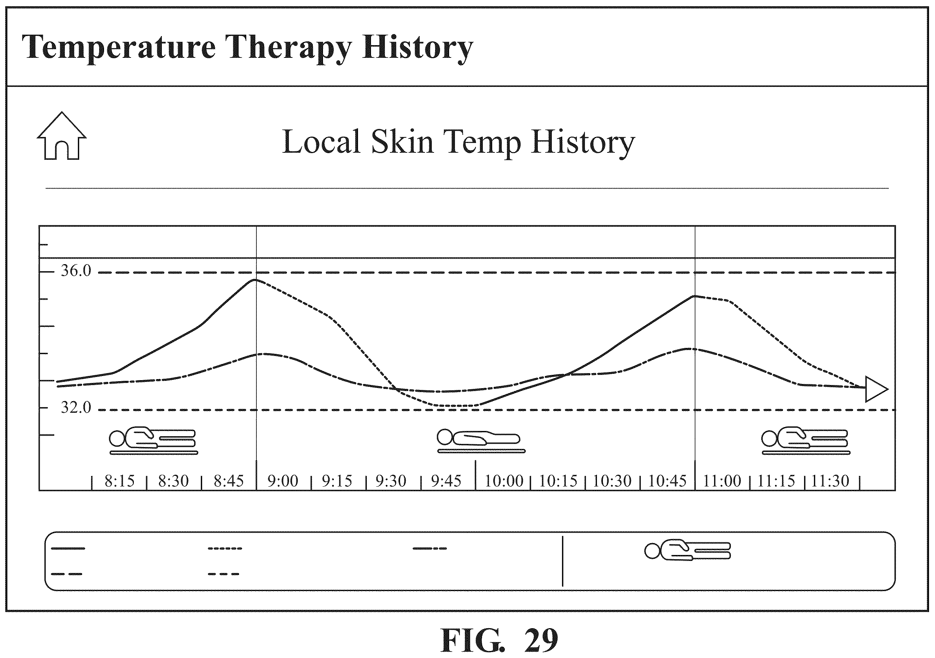

Additionally, or alternatively, the control board 128 of the patient support 20 may be in communication with a remote device 39 capable of receiving information and providing information or directives to the control board 128 for operation of the thermal control element 200. As an example, the remote device 39 may include a display capable of providing status or information pertaining to control or operation of the thermal control element 200, such as a temperature history associated with the patient as depicted in Fig. 29. The temperature history may be depicted remotely on the remote device 39 or locally on the user interface 38. The remote device 39 may include electronic health records or information associated with the patient, and may provide directives to the main controller 128 for operation of the thermal control element 200 based on this electronic health record or information associated with the patient. For instance, if the patient has been identified or associated with a potential pressure injury, the remote device 39 may provide information to the control board 128 with respect to this information so that an area or region associated with the potential pressure injury may be cooled via operation of the thermal control element 200.

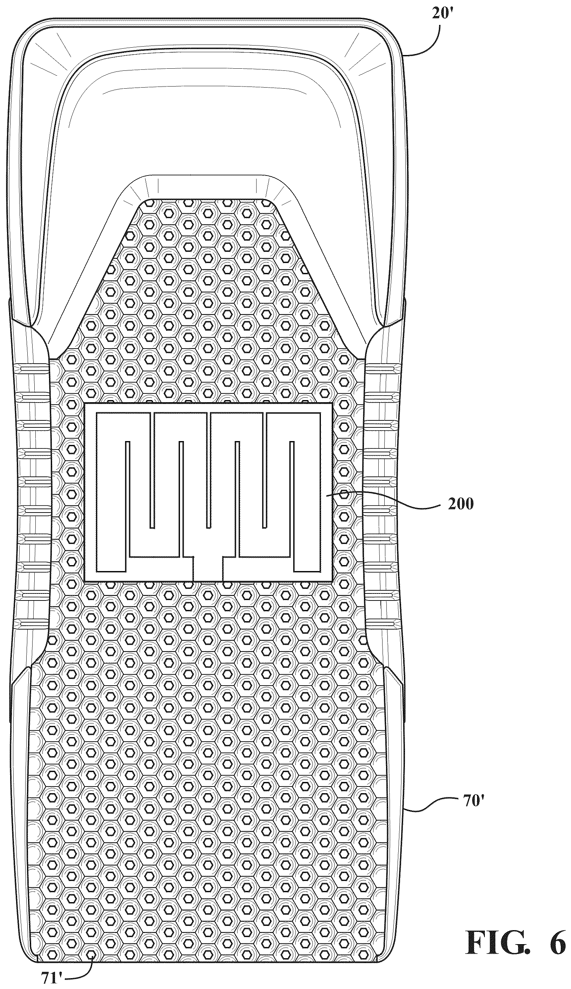

An alternative configuration of a patient support 20' that includes the thermal control element 200 is shown in further detail in Figs. 6-8. The patient support 20' in depictions of Figs. 6-8 is similar to the patient support 20 shown and described in connection with Figs. 2-3 with the exception of the inflatable bladders 66 being absent. The patient support 20' is depicted without an outer cover and fire barrier layer, with internal components visible, such as the foam crib 70' and the thermal control element 200. In place of a number of inflatable bladders 66, the patient support 20' in Figs. 6-8 includes a gel layer 71' constructed to cushion a patient disposed on the patient support 20'.

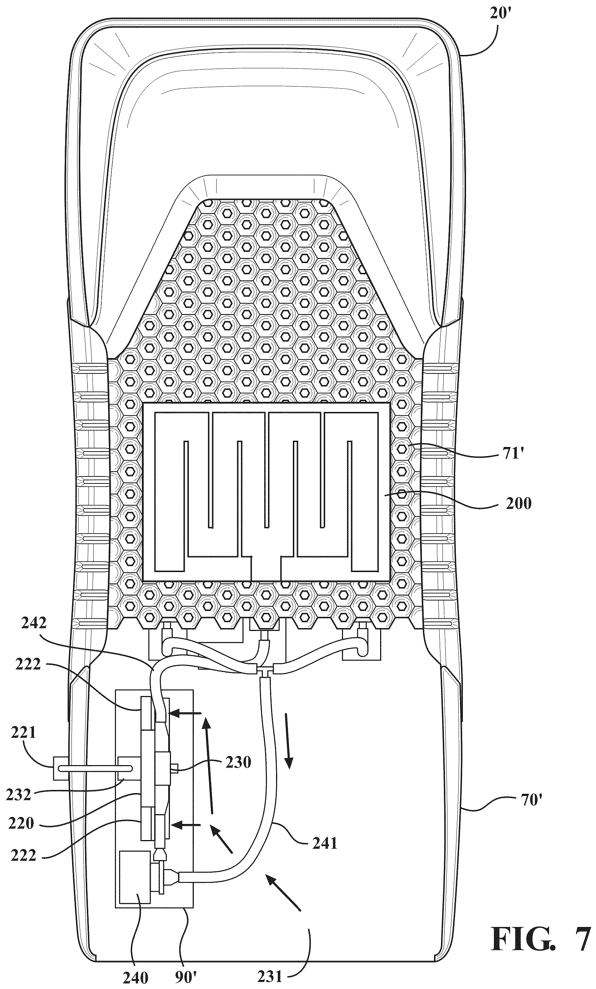

The patient support 20' includes a thermal control element 200, a heat exchanger 220, and a fluid mover 240. The heat exchanger 220 and the fluid mover 240 may be provided in a housing of a mattress control assembly 90', which is similar in many respects to the mattress control assembly 90 but configured to operate without aspects that control the inflatable bladders 66. The thermal control element 200, the heat exchanger 220, and the fluid mover 240 may be configured to operate in the patient support 20' and the patient support 20 in the same manner so that aspects of the thermal control element 200 described in conjunction with the patient support 20' may be provided in the patient support 20 and conversely relative to the patient support 20 and the patient support 20'. The fluid mover 240 in Fig. 7 is shown drawing air through a return conduit 241 from the thermal control element 200, pushing air through the heat exchanger 220 and then to the thermal control element 200 via a supply conduit 242. One or more return conduits 241 and one or more supply conduits 242 may be provided in conjunction respectively with one or more inputs and one or more outputs of the thermal control element 200.

The heat exchanger 220 in Fig. 7 may be configured to cool fluid flowing from the fluid mover 240 before being supplied to the thermal control element 200. The heat exchanger 220 in this configuration may be provided with a cold-side 230 and a hot-side 232, and may be configured to transfer heat from the cold-side 230 to the hot-side 232, passively or actively (e.g., via a thermoelectric cooler). In one aspect, the heat exchanger 220 may be configured to draw air (e.g., cold air) through a hot-side intake 231 and exhaust air through an exhaust port 221 (e.g., a hot-side outlet). One or more air movers 222 (e.g., fans) may be configured to draw air through the hot-side intake 231 and exhaust it through the exhaust port 221. The heat exchanger 220, as described herein, may include a thermoelectric cooling system, which can be controlled by supply of power under control by the control system 114. Further, fluid flow to and from the thermal control element 200 may be conducted by control of the fluid mover 240 by the control system 114. The heat exchanger 220 may be configured differently so that the hot-side and the cold-side are reversed, enabling supply of heated fluid to the thermal control element 200 instead of cooled fluid.

Although the fluid mover 240 is shown upstream of the heat exchanger 220, it is to be understood that the fluid mover 240 instead may be downstream of the heat exchanger 220. The heat exchanger 220 in one configuration may cool the air output from the fluid mover 240, which may impart heat into the air flowing through it. In one aspect, by virtue of being downstream of the fluid mover 240, the heat exchanger 220 may provide enhanced cooling of fluid received from the fluid mover 240 (e.g., offsetting the heat imparted by the fluid mover to the fluid) to increase cooling for the thermal control element 200.

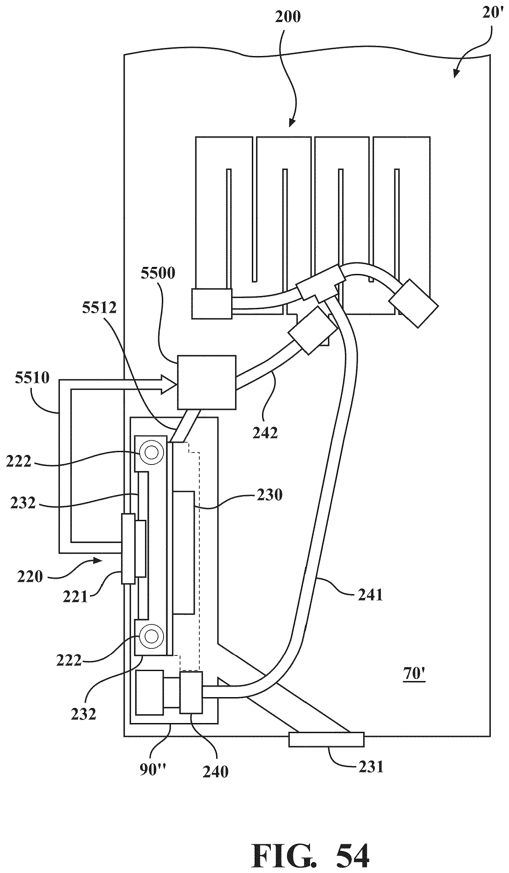

An alternative configuration is depicted in Fig. 54 with a thermal control element 200 being provided for a patient support 20' as described herein. The thermal control element 200 may be operable to receive fluid via a supply conduit 242 and return the fluid via a return conduit 241 to a fluid mover 240 and a heat exchanger 220. As described herein, the heat exchanger may include a cold side 230 and a hot side 232. The alternative configuration in Fig. 54 further includes a fluid mixer 5500 capable of receiving heated fluid exhausted from the hot side 232 via the exhaust port 221 and a first conduit 5510. A second conduit 5512 may be coupled between the cold side 230 of the heat exchanger 220 and the fluid mixer 5500. The fluid mixer 5500, optionally under control of the control system 114, may mix cold side and hot side fluid received respectively via the second conduit 5512 and the first conduit 5510 for supply to the thermal control element 200 via the supply conduit 242. The mixing of cold and hot side fluid may be conducted to achieve a target temperature with respect to fluid supplied to the supply conduit 242 and ultimately to the thermal control element 200. A sensor may be provided with respect to such fluid supplied to the supply conduit 242 in order to control the temperature of the fluid relative to a target temperature.

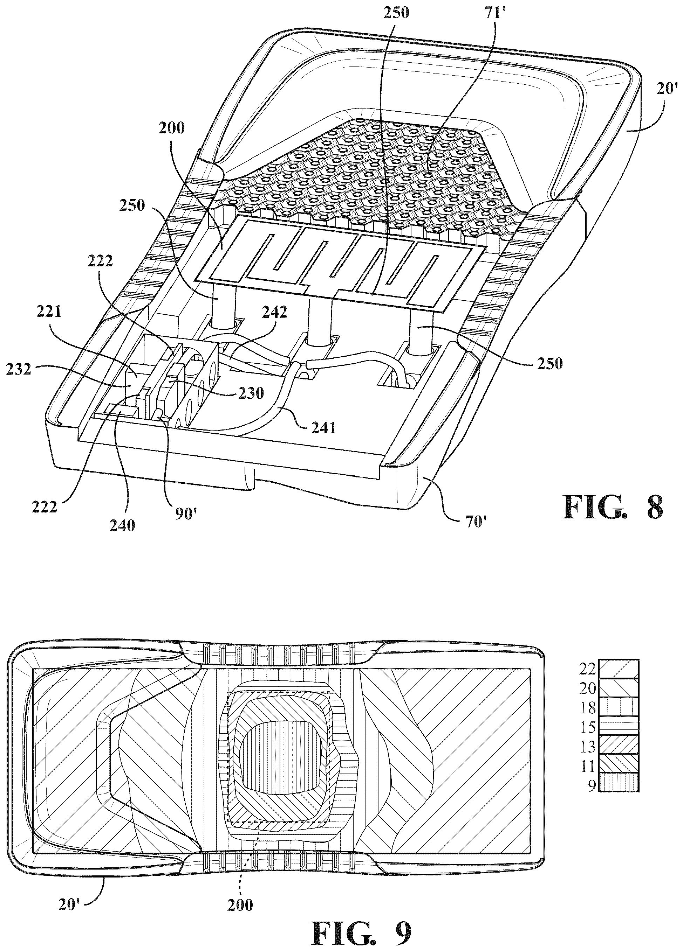

In Fig. 8, the thermal control element 200 may be coupled to the fluid mover 240 and the heat exchanger 220 in a variety of ways, including the return conduit 241 and the supply conduit 242. In one aspect, a flexible coupling 250 may be provided for connections respectively between the thermal control element 200 and one or more return conduits 241 and one or more supply conduits 242.

The flexible coupling 250 may be configured to enable a fluid connection between the return and supply conduits 241, 242 and the thermal control element 200 in a manner that enables immersion of the patient disposed on the patient support 20, 20' in a region associated with the flexible couplings 250. The flexible coupling 250 in one aspect may enable vertical compression in a manner similar to or less firm than the cushioning material adjacent to the flexible coupling 250. This way, the flexible coupling 250 may be substantially imperceptible to the patient disposed on the patient support 20.

As described herein, the fluid mover 240, the heat exchanger 220, and the thermal control element 200 may be configured to move fluid through the thermal control element 200 in a closed loop manner to provide cooled fluid thereto and return the cooled fluid to the heat exchanger 220 after flowing through the thermal control element 200. This configuration may enable cooling a patient disposed on the patient support 20, 20' without significantly affecting a core body temperature of the patient. For instance, a heat map of the patient support 20' is shown in Fig. 9 relative to the thermal control element 200, depicting a roughly 7 deg. C. delta between the thermal control element 200 and regions of the patient support 20' around the thermal control element 200. In one aspect, the thermal control element 200 may be configured to enable cooling of a patient in a manner that affects a core body temperature of the patient.

Although the thermal control element 200 is shown in the depicted configurations as being within the patient support 20, 20' below the fire barrier 97, it is to be understood that the thermal control element 200 may be disposed at other positions, such as above the fire barrier 97 but below the outer cover 96. As another example, the thermal control element 200 may be disposed external to the patient support 20, 20', such as on top of the outer cover 96.

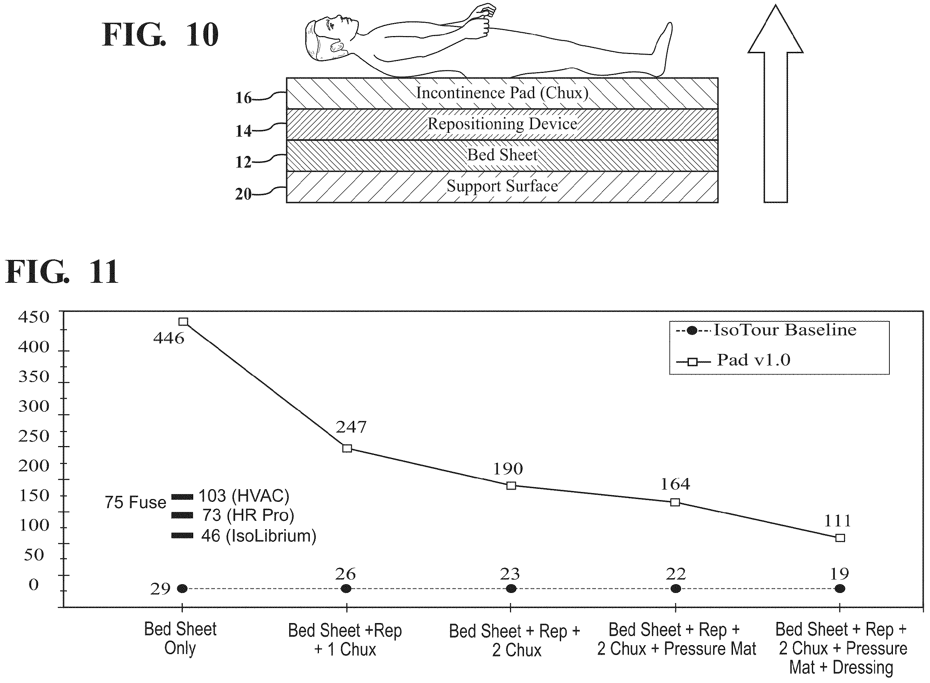

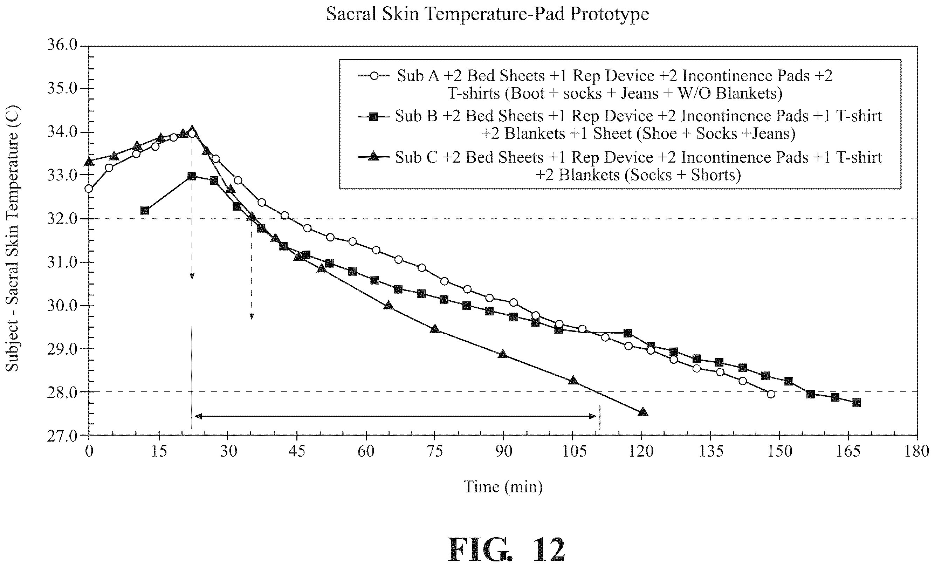

In use, there may be additional layers of material disposed between the patient support 20 and the patient, such as the one or more layers depicted in Fig. 10 including one or more of a bed sheet 12, a repositioning device 14, an incontinence pad 16 (e.g., a chux), a sensor system (e.g., a pressure mat for the interface sensor 125 and/or a temperature sensor), and a dressing on the patient. These one or more additional layers may impact an ability of the patient support 20 to affect a temperature of the patient supported on the patient support 20. The thermal control element 200 may be supplied fluid cooled by the heat exchanger 220 that allows the patient support 20 to affect a temperature of the patient despite the one or more additional layers, and in one aspect, to maintain a temperature of the patient below a target or threshold temperature without significantly affecting a core body temperature of the patient. For instance, in Fig. 11, the dry heat flux performance of a patient support 20 without the thermal control element 200 is compared against a patient support 20 that includes the thermal control element 200 for a variety of configurations that include one or more additional layers provided between the patient and the patient support 20, such as one or more of the following: a bed sheet 12, a repositioning device 14, and an incontinence pad 16, a pressure mat, and a dressing. It can be seen that the thermal control element 200 is capable of transferring thermal energy to the patient despite the one or more additional layers. For instance, performance of the thermal control element 200 over time for various configurations of one or more additional layers between the patient support 20 and the patient can be seen in Fig. 12, with the thermal control element 200 shown being capable of dropping the temperature of the patient's sacral skin below a target temperature (e.g., an upper limit) within a first time duration and further capable of dropping the temperature of the patient's sacral skin below a lower limit target temperature (e.g., a lower limit) within a second time duration. It is to be understood that, in operation, the control system 114 may be configured to avoid operating the thermal control element 200 to drop the patient's sacral skin temperature below the lower limit target temperature, thereby potentially avoiding significantly dropping a core temperature of the patient. Fig. 12 shows that the thermal control element 200 is capable of being operated to drop the patient's skin temperature despite one or more additional layers of various types being disposed between the patient and the patient support 20.

### IV. Thermal Control Element

The thermal control element 200 utilized in conjunction with the heat exchanger 220 and the fluid mover 240 may be configured in a variety of ways as described herein and any of the thermal control element configurations described herein or any aspect thereof may be implemented for the thermal control element 200 provided for the patient support 20, 20'. In one aspect, the thermal control element 200 may facilitate flow of fluid within the patient support 20, 20'.

In one aspect, the thermal control element 200 may be provided in the form of a thermal control layer within or outside the patient support 20, 20'. In one aspect, the thermal control layer may be a pad.

The thermal control element 200 according to one aspect may be configured to achieve sufficient heat removal to achieve a 28 °C sacral skin temperature. The construction of the thermal control element 200 may take into account a number of potential priorities. These priorities include one or more of the following:
- airflow channels to remain sufficiently open with the application of a patient load;
- limit the impact to patient pressure redistribution performance;
- spacer fabric porous enough to allow for a sufficient airflow rate;
- small enough pad size to not cool too large of a skin region and drop core body temperatures;
- large enough pad size to cool target anatomy with frame elevated head of bed angle, with patient migration, and throughout patient turns (e.g., thermal control element 200 may correspond to the full size or larger than the patient); and
- positioned strategically over supporting air bladders to account for patient turning, migration, and selective offloading.

The thermal control element 200 in one aspect may operate by circulating conditioned fluid (e.g., air) through a fluid tight (e.g., airtight) enclosure in a pattern (such as radiating from a central area outwardly to lateral areas) that cools from the patient center to the periphery and circulates the fluid back to a fluid mover / heat exchanger (e.g., a thermoelectric cooler) to then be recirculated through the thermal control element 200. This subsystem may include one or more of the following aspects as shown in Fig. 3B:
- Channel design → directs the airflow beneath the patient in a profile determined by an RF welded path
- Electrically conductive layer (e.g., a sensor) → a conductive path may be provided to measure the pad temperature and/or interface pressure
- Thermal conductive layer → a material with high thermal conductivity such as graphite or an additive to the outer layer to increase the thermal transfer efficiency of heat from the patient to the conditioned fluid within the thermal control element
- Spacer layer → a filler material provided for keeping the air channel open under a patient load while mitigating the impact to pressure redistribution
- Thermal reflective layer → a reflective material capable of reflecting heat energy
- Outer layer → a film or other elastic material capable of being RF welded, bonded, or sealed to create the channel configuration, and being sufficiently flexible to mitigate the effects of material "hammocking" which can negatively impact pressure redistribution performance
- Slits → slits may be provided by creating cuts in between the welds (e.g., seams) to decrease the "hammocking" effect that can negatively affect pressure redistribution performance
- Multi-zone → the thermal control element 200 may be constructed to facilitate selective cooling of different areas to focus cooling and accommodate patient movement due to elevated head of bed angle, patient migration, turning, or varied patient morphology / needs

The thermal control element 200 may be configured for targeted cooling from the center of the patient support 20, 20' to the outer width or lateral sides via a centrally located supply line 242 and two peripheral return lines 241. The thermal control element 200 may include upper and lower, air impermeable films that are radio frequency (RF) welded together with a spacer layer in the middle. Fluid may enter and exit the thermal control element 200 via welded fittings at the supply and return line locations. The RF welded lines may direct the fluid through the thermal control element 200 from the supply to the return locations. Fluid flow volume can be increased or decreased by increasing or decreasing channel width. These channels can create numerous flow profiles, including loops. Flow dynamics may be affected by the number of loops thereby impacting thermal performance. The number of loops provided in the thermal control element 200 may vary to affect the level of heat transfer between the patient and the fluid flowing through the thermal control element 200. The fluid flow path shape and cross-section may also vary from application to application. Analysis and construction of the fluid flow paths may be determined using sine and cosine profiles to enhance pressure redistribution performance.

As described herein, one configuration of the thermal control element 200 may include fluid holes in the upper layer. The holes may decrease thermal transfer performance by making the system open instead of closed. There is a possibility that the open holes may yield condensation by allowing higher humidity ambient fluid (e.g., air) into the system.

Supply and return line (e.g., supply conduit 242 and return conduit 241) quantity and locations can be varied to produce different thermal effects. In several depicted configurations, there is a single supply conduit 242 and two return conduits 241. By placing a single supply line in the center, the thermal control element 200 may concentrate the coldest fluid in the center and then allow the fluid to travel to two edges of the thermal control element 200 with two laterally located return conduits 241. Increasing the quantity and locations of these lines may alter the thermal properties to control the cooling profile delivery. However, limiting the quantity of supply lines provides increased thermal efficiency of the thermal control element 200. It is noted, however, that the number and location of the inlets to the thermal control element may vary from application to application.

In one configuration, the length of the supply line may be varied in response to flow testing results. Fluid flow may be measured using an indenter representing the trunk of a 50th percentile male. After analyzing the position of the indenter on the thermal control element 200, the interface pressure may be considered to occlude the supply air inlet due to the location on the patient support 20, 20'. Increasing the supply line length (e.g., as described in conjunction with the thermal control element 2300) may remove the semi-rigid fitting from the probable location of a patient load to increase fluid flow.

Apart from acting as an air-impermeable layer, the upper layer of thermal control element 200 may correspond to any type of material, such as film, textiles, or any other elastic material. An electrically conductive additive may be provided for the upper layer for incorporation of a sensor system therein, such as a sensor system operable to allow for temperature measurements for closed loop temperature control. Thermally conductive additive, such as carbon, may be added to the layer to increase the thermal transfer efficiency of heat from the patient to the circulating conditioned fluid within the thermal control element 200.

The lower layers of the thermal control element 200 may provide a fluid-impermeable membrane to contain the conditioned fluid circulating through the thermal control element 200 and maintain a closed-loop system. Layer composition, as described for the upper layer, may include any type of material, including elastic materials such as films, textiles, or other polymers. A plasticizer may be used to increase material elasticity. Materials or constructions that induce a negative Poisson coefficient may also be provided to improve pad pressure redistribution performance.

Thickness may have an effect in balancing thermal and pressure redistribution performance with durability and strength. In one aspect, the lower layer of the thermal control element 200 may be sized according to one of four TPU thicknesses: 0.25 mm, 0.15 mm, 0.10 mm, and 0.06 mm. The thinner the TPU, the more costly the welding process and potential adverse effect on durability.

As described herein, a filler material may be provided between the upper and lower layers of the thermal control element 200. This filler material may be a spacer fabric-but is not limited to such a configuration.

The thermal control element 200 may include upper and lower layers formed of any type of material, including TPU. The TPU in one example may have a thermal conductive additive, a thermal radiation reflective coating, and a plasticizer, or a combination thereof. The material for the upper layer and/or the lower layer may induce a negative Poisson coefficient.

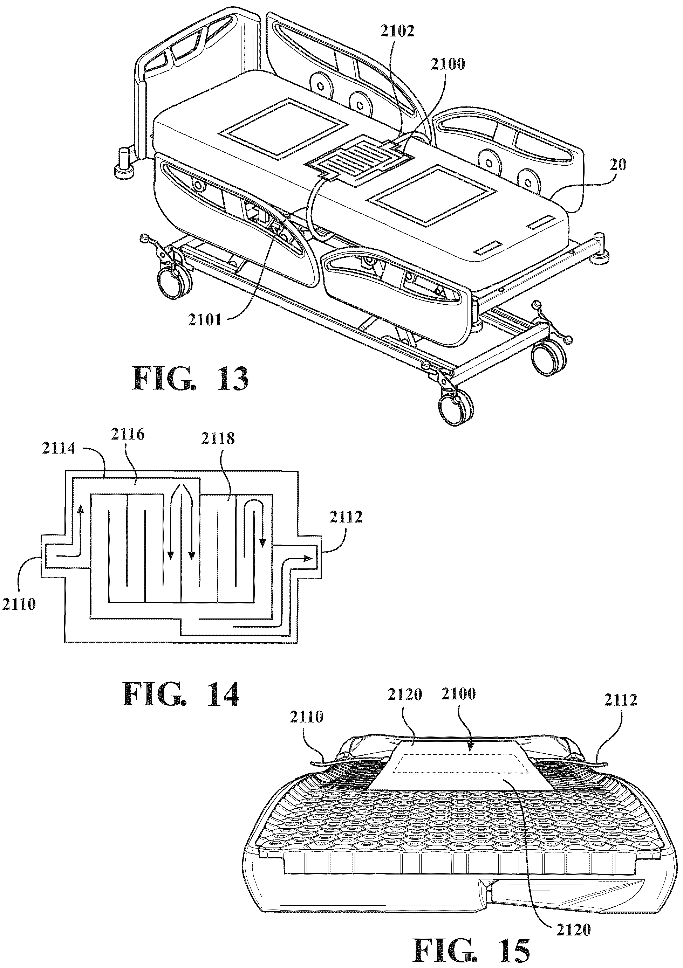

In Fig. 13, a thermal control element is shown in conjunction with a patient support 20 and generally designated 2100. The thermal control element 2100 in Fig. 13 may be incorporated into the patient support 20, 20' and may be similar in many respects to the thermal control element 200, with several exceptions such as being disposed outside the outer cover 96 of the patient support 20 so that the thermal control element 2100 is disposed between a bed sheet 12 and the outer cover of the patient support 20. The thermal control element 2100 is operable to receive and return fluid respectively via a first fluid conduit 2101 and a second fluid conduit 2102 to the fluid mover 240 and the heat exchanger 220. The thermal control element 2100 includes an inlet 2110 and an outlet 2112 with one or more fluid flow paths 2116 therebetween. The inlet 2110 and the outlet 2112 may be positioned at lateral sides of the thermal control element 2100 so that the inlet 2110 and the outlet 2112 are unlikely to be positioned between the patient and the patient support 20. Additional or fewer inlets 2110 and/or outlets 2112, respectively, may be utilized in the thermal control element 2100, or any of the thermal control elements described herein.

The fluid may be any type of fluid, including, for instance, air or water or a refrigerant.

The thermal control element 2100 in Fig. 13 includes a filler material 2114 disposed within one or more fluid flow paths 2116. The filler material 2114 may facilitate maintaining open flow paths for fluid flow within the fluid flow path 2116 through the thermal control element 2100. The filler material 2114 may be porous with respect to the fluid flowing through the thermal control element 2100, and the type of filler material 2114 may vary from application to application. As an example, the filler material 2114 in Fig. 13 corresponds to a spacer fabric that generally includes an upper and lower fabric layer connected by a middle layer of filaments or fibers that form a 3-dimensional structure and provides and supports the fluid flow path 2116 despite compression caused by the weight of the patient on the thermal control element 2100.

In Fig. 13, the thermal control element 2100 is formed of an upper and lower layer of material, such as polyvinyl chloride sheets or TPU sheets, that are welded together at seams 2118 that define the fluid flow paths 2116 with the filler material 2114 provided therein.

The thermal control element 2100 may be a separate component position with respect to the patient support 20. Alternatively, as depicted in Fig. 15, the thermal control element 2100 may be incorporated into another component capable of being positioned on or within the patient support 20, such as a chux or incontinence pad capable of being positioned between the patient and the outer cover 96 of the patient support. In Fig. 15, the incontinence pad may include additional material 2120 (e.g., absorbent material) provided proximal to the thermal control element 2100.

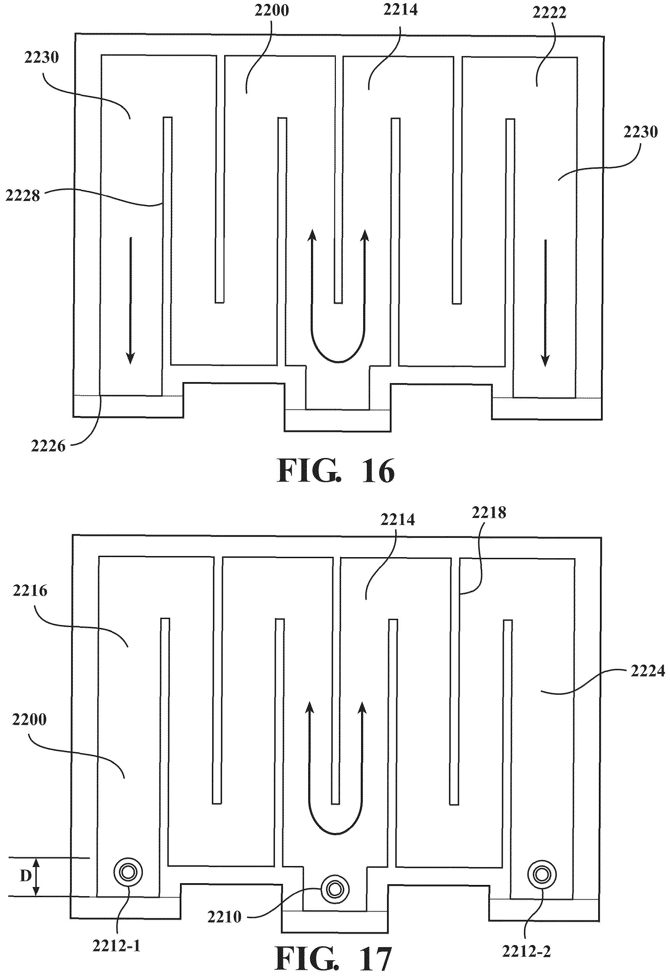

Turning to Figs. 16 and 17, a thermal control element according to one aspect is shown and generally designated 2200. The thermal control element 2200, or one or more features thereof, may be incorporated into the patient support 20, 20' as the thermal control element 200. The thermal control element 2200 may include a fluid inlet 2210 and first and second fluid outlets 2212-1, 2212-2. The fluid inlet 2210 may receive fluid from the heat exchanger 220 and the fluid mover 240, and the first and second fluid outlets 2212-1, 2212-2 may return the fluid to the heat exchanger 220 and the fluid mover 240 via one or more fluid conduits 241, 242 and/or flexible couplings 250 as described herein. Fluid flow through the thermal control element 2200 may facilitate controlling or affecting a temperature associated with the patient, such as affecting a skin temperature of a region of the patient (e.g., a sacral region).

The thermal control element 2200 in Fig. 16 includes an upper layer 2222 and a lower layer 2224 formed of sheets of material that are fluid impermeable-although different material constructions that are air and/or water permeable may be utilized. For instance, optionally, the upper layer 2222 may include a plurality of openings 2230 that enable fluid flow therethrough to escape the thermal control element 2200.

The sheets of material in one configuration are TPU or Polyvinyl chloride that are welded together at one or more seams 2218. The seams 2218 may include a periphery seam 2226 and a plurality of internal seams 2228 for which the upper and lower layers 2222, 2224 are welded together (e.g., RF welding) to form a permanent bond between the upper and lower layers 2222, 2224.

Similar to the thermal control element 2100, thermal control element 2200 may include a filler material 2214, which may be porous with respect to fluid flow therethrough and operable to provide separation between the upper and lower layers 2222, 2224 despite compression caused by the weight of the patient on the thermal control element 2200. The filler material 2214 may be constructed in a variety of ways depending on the configuration and may be similar to the filler material 2114.

The inlet 2210 in Fig. 17 may include an inlet coupling operable to couple to a flexible coupling 250 in a substantially airtight manner to avoid significant leakage. Likewise, the first and second outlets 2212-1, 2212-2 may include outlet couplings operable to couple to the flexible couplings 250 in a substantially airtight manner. The inlet 2210 may be spaced by a distance D from the fluid flow paths 2216 of the thermal control element 2200, and the first and second outlets 2212-1, 2212-2 may be spaced by the same distance D from the fluid flow paths 2216 of the thermal control element 2200.

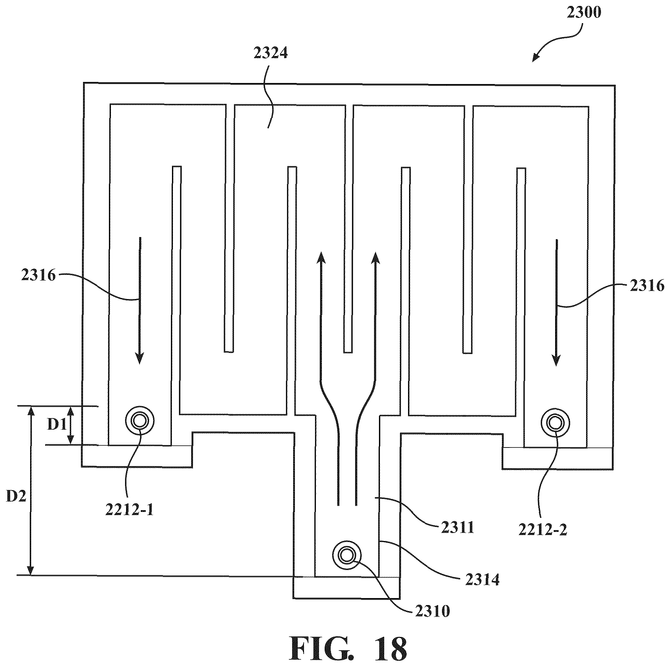

Fig. 18 shows a thermal control element according to one aspect and generally designated 2300. The thermal control element 2300, or one or more features thereof, may be incorporated into the patient support 20, 20' as the thermal control element 200. The thermal control element 2300 is similar to the thermal control element 2200 with the exception of a greater distance between the fluid flow paths 2316 and the inlet 2310, defined by the upper layer and the lower layer 2324 of the thermal control element 2300, and generally designated as an inlet extension 2311. As a result, components or aspects of the thermal control element 2300 that are substantially the same as corresponding aspects or features of the thermal control element 2200 utilize the same reference numbers. The thermal control element 2300 in Fig. 18 may be configured such that the upper layer and the lower layer 2324 are absent one or more openings or holes so that fluid flows from the inlet 2310 to an outlet without escaping through the upper layer and/or the lower layer 2324. It is to be understood that the thermal control element 2300 may be configured differently-e.g., with one or more openings, such as the openings 2230 described in conjunction with Fig. 16.

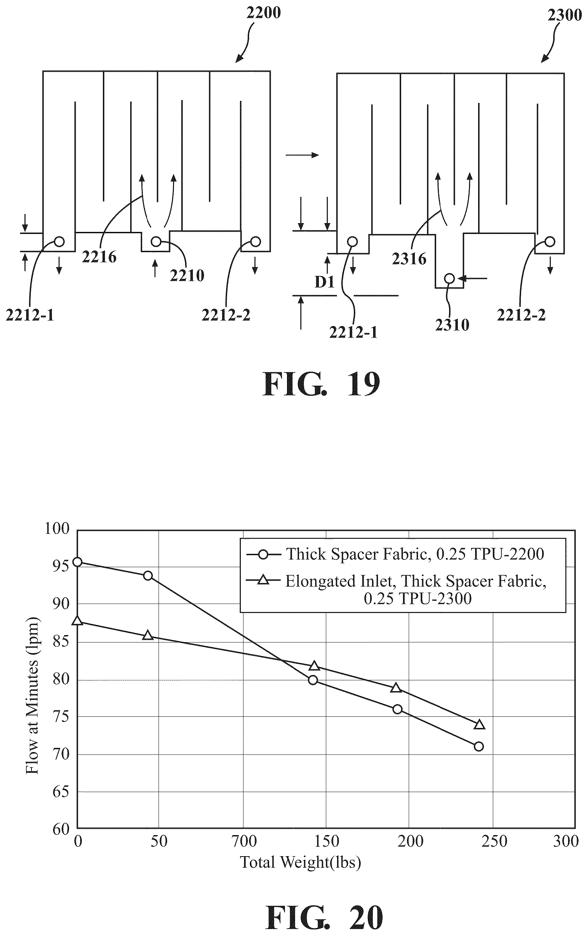

It can be seen in Fig. 18 and more so in Fig. 19 (which shows the thermal control element 2200 next to the thermal control element 2300) that the distance D2 between a coupling of the inlet 2310 and the fluid flow paths 2316 is greater than the distance D1 between a coupling of the first and second outlets 2312-1, 2312-2 and the fluid flow paths 2316 and the distance D between a coupling of the first and second outlets 2212-1, 2212-2 and the fluid flow paths 2216. This additional distance may facilitate greater flow rate of fluid through the thermal control element 2300 relative to the thermal control element 2200 with respect to greater weights on the thermal control element 2300. This greater flow rate can be seen in the flow rate analysis depicted in Fig. 20, with the thermal control element 2300 having greater flow rates than the thermal control element 2200 for weights greater than about 120 lbs. Filler material 2314, similar to the filler material 2214, may be provided within the elongated portion of the inlet extension 2311.

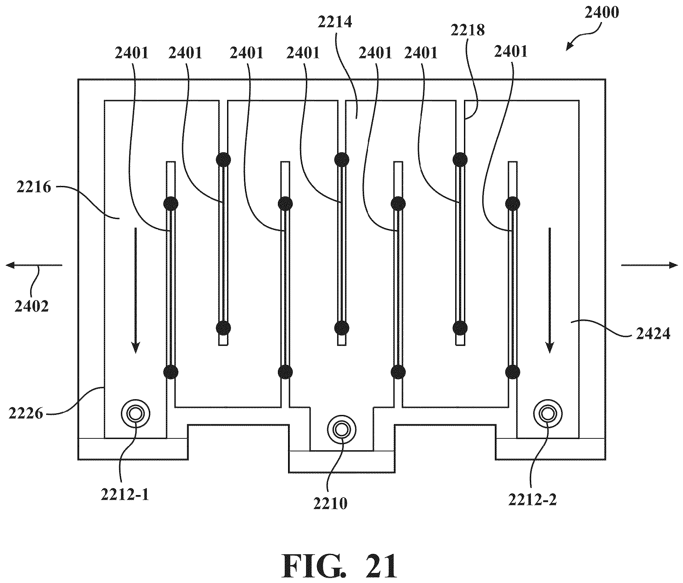

Fig. 21 shows a thermal control element according to one aspect and generally designated 2400. The thermal control element 2400, or one or more features thereof, may be incorporated into the patient support 20, 20' as the thermal control element 200. The thermal control element 2400 is similar to the thermal control element 2200 with the exception of slits 2401 defined by the upper layer and the lower layer 2424 of the thermal control element 2400. As a result, components or aspects of the thermal control element 2400 that are substantially the same as corresponding aspects or features of the thermal control element 2200 utilize the same reference numbers.

The slits 2401 may enable lateral movement of one or more portions of the thermal control element 2400 relative to a transverse axis 2402. For instance, with the slits 2401, one or more flow paths 2216 may separate from each other in response to a patient's weight being applied to a region (e.g., a central region) of the thermal control element 2400. This separation may prevent or dampen hammocking with respect to the patient (and allow immersion without significant lateral stress on the patient's skin). The size and configuration of the slits 2401 may vary from application to application-although, in general, the slits 2401 may be provided with respect to the seams 2218, so that the fluid flow paths 2216 remain substantially sealed and do not leak fluid via the seams 2218.

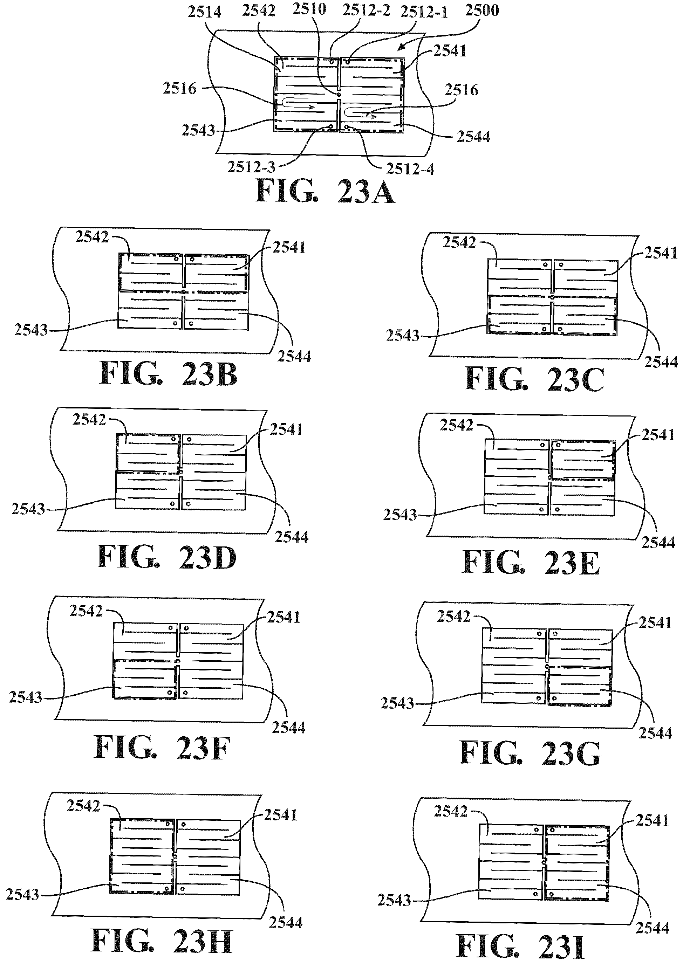

Fig. 23A shows a thermal control element according to one aspect and generally designated 2500. The thermal control element 2500, or one or more features thereof, may be incorporated into the patient support 20, 20' as the thermal control element 200. The thermal control element 2500 is similar to the thermal control element 2200 in many respects, including fluid flow paths 2516 similar to the fluid flow paths 2216 as well as filler material 2514 similar to the filler material 2214. The thermal control element 2500 may include a fluid inlet 2510 and a plurality of fluid outlets 2512-1, 2512-2, 2512-3, 2512-4.

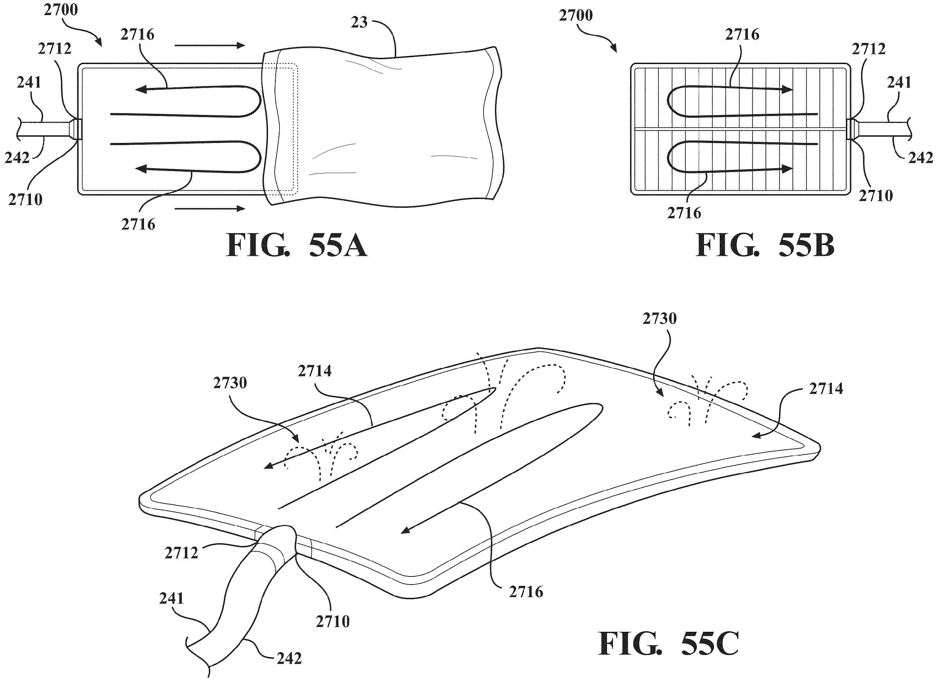

Figs. 55A, 55B, and 55C depict a thermal control element according to one aspect and are generally designated 2700. The thermal control element 2700 may be provided separate from the patient support 20, 20' and incorporated into another component associated with the patient. For instance, in the illustrations, the thermal control element 2700 may be incorporated into a pillow 23 for the patient (e.g., the thermal control element 2700 may be inserted in or slipped within a pillowcase of the pillow 23). Despite being configured for incorporation into a component other than the patient support 20, 20', the thermal control element 2700 may be similar in many respects to one or more other thermal control elements described herein, including, for example the thermal control element 2200. More particularly, similar to the thermal control element 2200, the thermal control element 2700 may include fluid flow paths 2716 similar to the fluid flow paths 2216 as well as filler material 2714 similar to the filler material 2214. The thermal control element 2700 may include a fluid inlet 2710 and a fluid outlet 2712, similar to the fluid inlets 2210 and the fluid outlet 2212. Additionally, optionally, the thermal control element 2700 may include a plurality of openings 2730 that enable fluid flow there through for purposes of cooling and drying of the patient.

### V. Zone Control

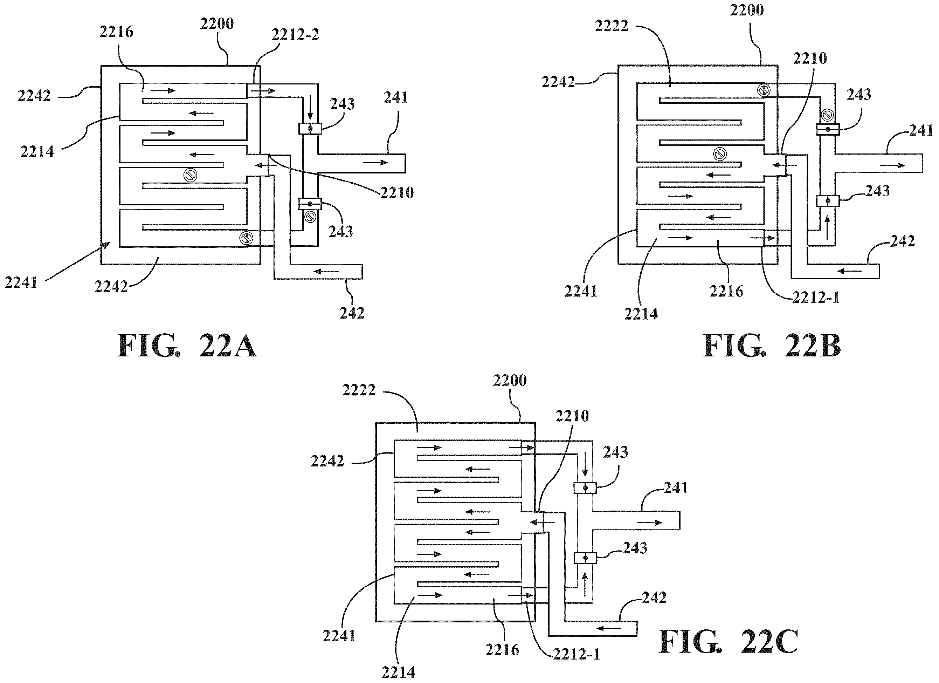

The thermal control element 200 may be configured to enable fluid flow through one or more portions thereof and fluid flow to be withheld from one or more other portions thereof. The thermal control element 2200 is shown in conjunction with such a configuration in Figs. 22A-22C-although it is to be understood that any of the thermal control element 200 described herein may be configured according to multizone operation based on the aspects described in conjunction with the thermal control element 2200, including the valve control aspects for the return conduits. The control system 114 may control operation of valves 243 in the return fluid conduit 241 with respect to each of the first and second outlets 2212-1, 2212-2. Selective control over the valves 243 may effectively provide first and second zones 2241, 2242 for the thermal control element 2200. The valves 243, in one aspect, may be provided in the return path, because there is a potential for the valves 243 to add heat into the system. By placing the valves 243 in the return path, this heat may be dissipated by the heat exchanger 220 prior to fluid entering the thermal control element 200. As described herein, the valves 243 may be provided in separate conduits or provided together in a centralized manifold.

For instance, in Fig. 22A, by shutting the valve 243 associated with the outlet 2212-1 of the thermal control element 2200, fluid flow through a first zone 2241 may be withheld. And, by opening the valve 243 associated with the outlet 2212-2 of the thermal control element 2200, fluid flow may be provided through the second zone 2242. In Fig. 22B, the valves 243 may be controlled in an opposite manner so that fluid flow is withheld from the second zone 2242 and fluid flow is provided through the first zone 2241 (e.g., the valve 243 associated with the outlet 2212-2 is closed and the valve 243 associated with the outlet 2212-1 is open). Fig. 22C shows both valves 243 being open so that fluid flow is provided through both the first and second zones 2241, 2242.

As another example, in Figs. 23A-I, by enabling return flow of fluid through one or more of the fluid outlets 2512-1, 2512-2, 2512-3, 2512-4, one or more zones 2541, 2542, 2543, 2544 may be selectively enabled or disabled for the thermal control element 2500. By selectively enabling fluid flow through the fluid outlet 2512-1, the first zone 2541 may be selectively enabled or disabled. The second zone 2542 may be selectively enabled or disabled, likewise, by selectively enabling fluid flow through the fluid outlet 2512-2. And by selectively enabling fluid flow through the fluid outlet 2512-3, the third zone 2543 may be selectively enabled or disabled. And similarly, by selectively enabling fluid flow through the fluid outlet 2512-4, the fourth zone 2544 may be selectively enabled or disabled. Any combination of the zones 2541, 2542, 2543, 2544 may be enabled or disabled, so that for example, one or more zones 2541, 2542, 2543, 2544 may be active with respect to fluid flow therethrough, while one or more other zones 2541, 2542, 2543, 2544 may be inactive so that fluid flow is withheld therefrom. Several example combinations are depicted in Figs. 23B-23I: Fig. 23B shows zones 2541, 2542 active; 23 C shows zones 2543, 2544 active; Fig. 23D shows zone 2542 active; Fig. 23 E shows zone 2541 active; Fig. 23F shows zone 2543 active; Fig. 23G shows zone 2544 active; Fig. 23 H shows zones 2542, 2543 active; and Fig. 23I shows zones 2541, 2544 active.

It is to be noted that activation or deactivation of one or more of the zones may be conducted in response to a variety of criteria. For example, in one aspect, the first zone 2541 may be activated or deactivated in response to a turning bladder (which may be aligned with the first zone or associated with the first zone based on the turning bladder causing increased pressure proximal to the first zone in response to inflation of the turning bladder) being activated (e.g., inflated) to facilitate turning a patient disposed on the patient support. One potential effect of this approach is to concentrate cooling for a patient in a turned position due to the change in pressure injury site risk. As another example, one or more zones of the thermal control element may be selectively activated or deactivated based on criterion such as a position or state of the frame 30 and/or the one or more deck sections 40, 42, 44, 46. In this way, one or more zones may be activated or deactivated with frame and/or deck articulation to account for patient migration and to avoid substantially affecting a core body temperature. It is to be understood that activation of the one or more zones in response to one or more criteria may be conducted with respect to any of the thermal control element configurations described herein. Zone activation may also be conducted in response to selective offloading of one or more of the inflatable bladders 66 or the air pod zones associated with a plurality of inflatable bladder 66. Offloading of one more inflatable bladders 66 or a zone associated with multiple inflatable bladders 66 may include selectively deflating one or more inflatable bladders 66 relative to one or more adjacent inflatable bladders 66 to form a gap or relieve pressure with respect to the patient and the selectively deflated one or more inflatable bladders 66. A variety of offloading techniques are described in PCT Appl. No. PCT/US2024/030233, filed May 20, 2024, to Derenne et al. and entitled INFLATABLE MATTRESS AND CONTROL THEREOF and U.S. Appl. No. 63/723,340, filed November 21, 2024, to Derenne et al. and entitled INFLATABLE MATTRESS AND CONTROL THEREOF-the disclosures of which are hereby incorporated by reference in their entirety. This may allow for tissue areas not under load to increase in temperature and thereby increase blood flow to improve tissue health/load tolerance, while anatomical areas under load may be cooled to decrease the sweat rate and metabolic demand of the tissue.

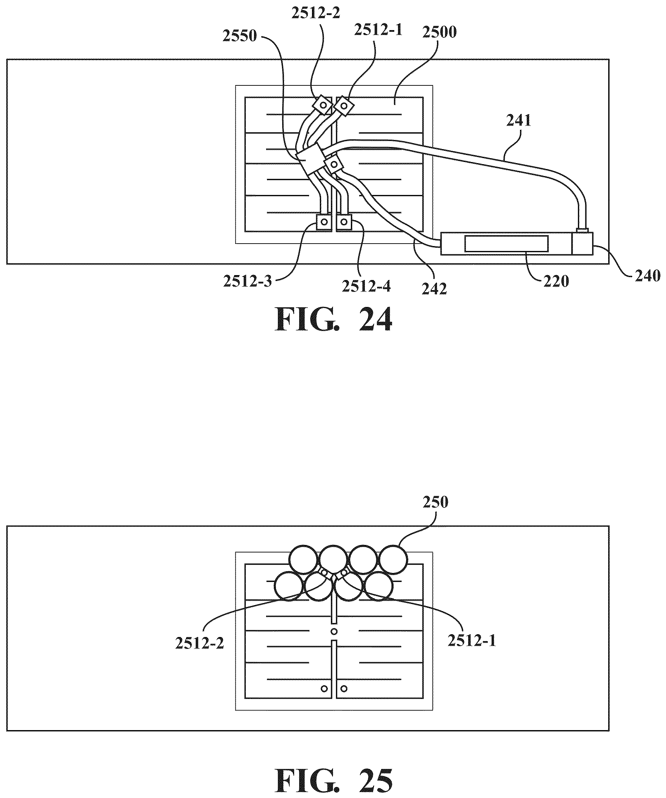

Fig. 24 shows a manifold 2250 operable to selectively control fluid flow with respect to the fluid outlets 2512-1, 2512-2, 2512-3, 2512-4. The manifold 2250 may be coupled to and directed by the control system 114 to selectively control which of the fluid outlets 2512-1, 2512-2, 2512-3, 2512-4 is enabled for fluid flow through the fluid conduit 241 back to the fluid mover 240, the heat exchanger 220, and returned to the thermal control element 2500 via the supply conduits 242. The manifold 2250 may be provided in a layer of the patient support 20, 20' that is distal from a support surface of the patient support 20, 20', such as being beneath cushioning layers (e.g., a gel layer and/or inflatable bladders). The manifold 2250 may be coupled to each of the fluid outlets 2512-1, 2512-2, 2512-3, 2512-4 via a plurality of flexible couplings 250, similar to the arrangement depicted in Fig. 25, with the fluid outlets 2512-1 coupled to a plurality of flexible couplings 250 for a fluid connection with the manifold 2250, and the fluid outlet 2512-2 coupled to a plurality of flexible couplings 250 for a fluid connection to the manifold 2250.

Target cooling areas or zones may vary based on patient morphology, migration, existing pressure injuries, and other conditions. Localized cooling may operate by focusing cooling on areas of highest pressure. If the cooling region is too large, there is a possibility that a patient's core body temperature may drop thereby inducing adverse health effects. However, too small of a cooling region may prevent effective therapy for a full range of the target patient population based on the target cooling area range described herein. Providing cooling to selected zones enables concentrated cooling on varying target regions.

In one aspect, as described herein, multi-zone control may be provided by placing valves on the return lines to block the return of fluid for a specified pad region (e.g., region of the thermal control element 200). Fluid can also be partially occluded to limit the amount of cooling to regions of the thermal control element 200. There may be a thermal efficiency advantage to having a single supply line, but in the case of multiple supply lines, valves may be added to direct cooling in different zones.

In one aspect, valves may be provided to add heat into the flow fluid into the thermal control element 200, and therefore having valves on the return lines can provide another thermal advantage. These valves can be placed on individual air lines or multiple air lines can be routed to one or more manifolds containing one or more valves for more efficient control. The thermal control element 200 may have one or more zones depending on the number of return lines.

### VI. Filler Material

As described herein, the thermal control element 200 may utilize a filler material (e.g., filler material 2214) that is porous with respect to the fluid flow without significantly restricting the fluid flow and that is operable to facilitate maintaining fluid flow through the thermal control element 200 despite compression caused by the patient's weight on the thermal control element 200. For instance, the filler material 2214 may facilitate providing separation between upper and lower layers of the thermal control element 200.

The filler material in one aspect may correspond to a spacer fabric, as described herein. However, the present disclosure is not so limited so that the filler material may be any type of material capable of being provided within a fluid flow path (e.g., the fluid flow path 2214 or any other fluid flow path described herein) to facilitate maintaining fluid flow through the fluid flow path under a variety of circumstances, including compression of the thermal control element 200 caused by the patient's weight. The filler material may be porous with respect to the fluid and/or at least capable of enabling the fluid flow through the fluid flow paths of the thermal control element 200. Example variables for the filler materials include material (polymer type [e.g., polyester] or mono-material or blend), fiber (top and bottom or middle), yarn (yarn spinning methods, such as twist angle, yarn density), knit (varying structures, including similar top and bottom yarn/knits and different top and bottom yarn/knits), slits or cuts, compression, and treatments (e.g., heat treated). The fiber may vary from application to application, including via extrusion drawing, crimped, shape (e.g., kidney, dog-bone), extrusion method, length, denier, and additives (e.g., additive to increase cooling).

The spacer fabric may correspond to a textile with upper and lower fabric layers connected by a middle layer, which may include filaments, yarns, or fibers, to form a 3-dimensional structure. The upper and lower layers may be substantially parallel to each other and formed of a variety of materials, including, for example, polyester, nylon, or another type of synthetic material. The upper and lower layers may have the same or different textures, including one or more of the following: smooth, mesh, or knitted. The middle layer may include a plurality of elements that span from the upper to lower layers, potentially in a generally vertical manner, to facilitate spacing the upper and lower layers from each other. The overall height of the spacer fabric may be determined at least in part by the distance spanned by the elements of the middle layer between the upper and lower layers. The density or number of elements in the middle layer may affect cushioning properties of the spacer fabric, as well as a porosity of the spacer fabric with respect to fluid flow there through.

The purpose of the spacer layer is to maintain an open-fluid pathway with the application of a patient load to the thermal control element 200. There may be a balance between a sufficient stiffness to produce an acceptable fluid passage for thermal performance while maintaining enough elasticity to limit the adverse pressure redistribution impact. Spacer fabric may be one type of material used to achieve this balance. The spacer thickness, rigidity, and porosity may be varied to achieve target performance for the thermal, flow, and pressure redistribution aspects. Potential materials include various polymers such as polyester, mono-materials, or blends. Other or additional filler materials may include 3D printed foam, elastic material, bonded polymer filament in three-dimensional shape, gel / silicon beads, and general filling, or a combination thereof.

Fibers for the spacer top, bottom, and filament middle may comprise various combinations, and include changing the extrusion, crimp, shape, length, and denier of the fibers. An additive can be placed in this layer to promote additional cooling. Various yarn can be employed changing the spinning method, twist angle, and yarn density as well as knits with changing the structures between the top and bottom knits or keeping them the same. Middle monofilaments can have varied filament densities and denier. Treatments such as heat treatments may further impact these properties. Changing these factors may affect the compression factor, which can be optimized or enhanced for pressure redistribution performance and flow. The thickness may also impact these properties.

Hammocking is a consideration when evaluating the filler material configuration and related pressure redistribution properties. Hammocking may relate to the phenomenon of continued pressure with an upper load due to a lack of material elasticity when a lower support has been removed. One way to address this phenomenon may be to increase the weld width and cut slits between the fluid looping channels as described herein. This may increase the pad flexibility to conform to applied loads. Such slits are described in conjunction with the thermal control element 2400-although the slits or a variant thereof may be incorporated into any of the thermal control elements described herein.

Alternative filler material configurations include but are not limited to 3D printed foam, 3D printed elastomer material, polymer ventilation mats (bonded polymer filament in a three-dimensional shape), gel, silicon beads, or any other type of material.

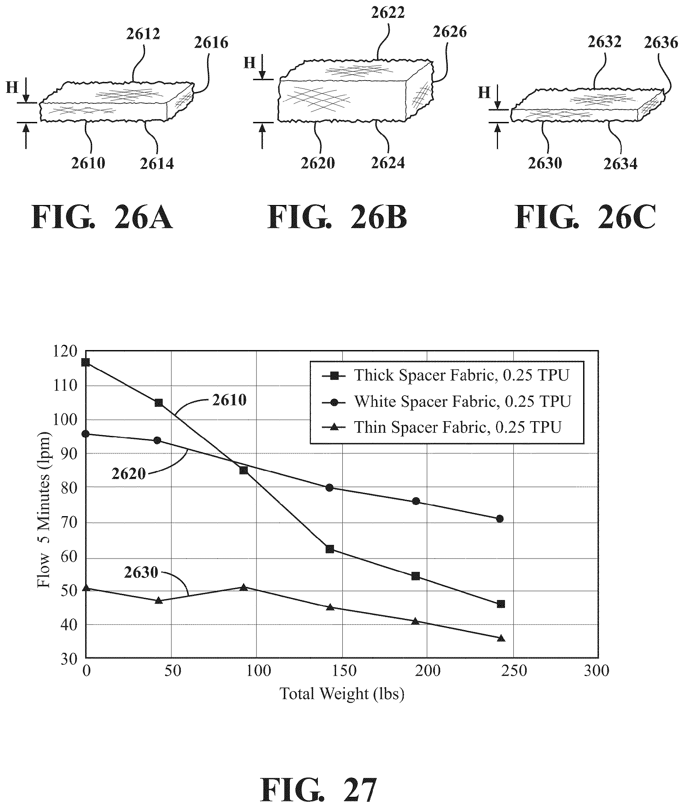

Three different configurations of spacer fabric according to various aspects are shown in Figs. 26A, 26B, and 26C and generally designated 2610, 2620, 2630, respectively. The spacer fabrics 2610, 2620, 2630, as noted herein, may include an upper layer 2612, 2622, 2632, a lower layer 2614, 2624, 2634, and a middle layer 2616, 2626, 2636. These layers may be configured in any manner as described herein with respect to the spacer fabric construction.

It can be seen in Figs. 26A, 26B, and 26C that the spacer fabrics 2610, 2620, 2630 each has a height H corresponding to a distance between the upper layer 2612, 2622, 2632 and the lower layer 2614, 2624, 2634, with elements of the middle layer 2616, 2626, 2636 provided from the upper layer 2612, 2622, 2632 to the lower layer 2614, 2624, 2634. The height H of the spacer fabric 2610, 2620, 2630 may vary from application to application, and may be selected for a target level of performance with respect to the potential weight of the patient and target flow rate through the thermal control element 200. For instance, performance of the spacer fabrics 2610, 2620, 2630 with different heights H is shown in Fig. 27, with the spacer fabric 2630 being the thinnest, the spacer fabric 2620 being the thickest, and the spacer fabric 2610 having a height H between the heights H of the spacer fabric 2630 and the spacer fabric 2620.

### VII. Flexible Coupling

The test results discussed herein highlight a performance gap created by the thermal control element 200 (e.g., a pad localized cooling system). This thermal control element 200 and its related system components may provide an integral role in achieving that performance and a flexible coupling 250 with collapsing channels may mitigate adverse pressure redistribution performance impact on the support surface of the patient support 20, 20'.

One way to address concerns over pressure redistribution performance relative to the thermal control element 200 is to create a collapsing channel system that buckles or changes shape under the load of a patient while maintaining sufficient airflow.

As described herein, a flexible coupling 250 may be configured to enable a fluid connection between the return and supply conduits 241, 242 and the thermal control element 200 in a manner that enables immersion of the patient disposed on the patient support 20, 20' in a region associated with the flexible couplings 250. The flexible coupling 250 in one aspect may enable vertical compression in a manner similar to or less firm than the cushioning material adjacent to the flexible coupling 250. This way, the flexible coupling 250 may be substantially imperceptible to the patient disposed on the patient support 20. The flexible coupling 250 may take a variety of forms depending on the application and any of the flexible coupling configurations described herein or any aspect thereof may be implemented for the flexible coupling 250 for the thermal control element 200 provided for the patient support 20, 20'.

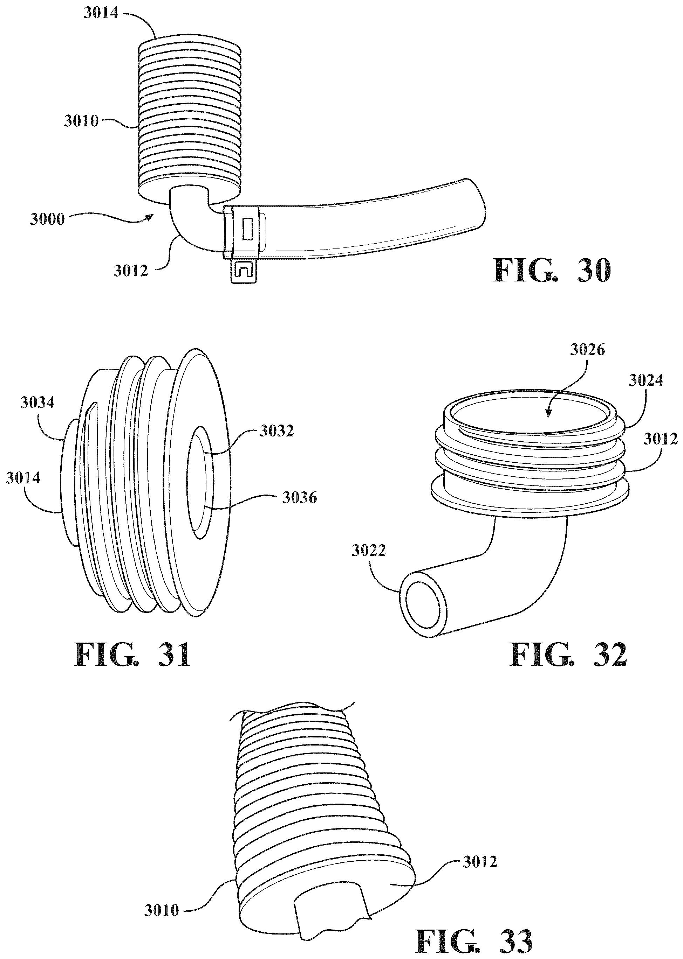

In Fig. 30, a flexible coupling in one aspect is shown and generally designated 3000. The flexible coupling 3000 may be implemented as the flexible coupling 250 for use with the thermal control element 200 according to any of the one or more aspects described herein.

The flexible coupling 3000 in Fig. 30 includes a flexible conduit 3010, a conduit coupler 3012, and a layer coupler 3014. The flexible conduit 3010 may be configurable to expand and collapse in response to movement of the thermal control element 200 relative to the conduit coupler 3012, which may remain substantially fixed relative to one or more lower layers of the patient support 20, 20'. For instance, in Fig. 30 and Fig. 33, the flexible conduit 3010 is operable to change in length (extend and/or contract) and is configured similar to either a flexible straw or a hose with a spiral wire wound within it along its length and providing structural reinforcement with respect to the flexible conduit 3010. Fig. 30 shows the flexible conduit 3010 in a contracted position, and Fig. 33 shows the flexible conduit 3010 in extended position. Example configurations include flexible conduit 3010 in the form of a spring-loaded compression tube that has a 2 inch diameter (outside diameter) or 1.44 inch diameter (outside diameter).

In one aspect, by being capable of extending and contracting, the flexible conduit 3010 may enable the layer coupler 3014 to rise and fall with respect to vertical movement of the thermal control element 200 under the weight of the patient. For instance, as described herein, the patient supports 20, 20' may include cushioning elements, such as inflatable bladders or gel, disposed beneath the thermal control element 200. As the weight of the patient is applied to the thermal control element 200 and the cushioning elements, the cushioning elements may compress thereby lowering the thermal control element 200 relative to the conduit coupler 3012. This movement of the thermal control element 200 and the cushioning elements may be accommodated by varying the length or shape of the flexible conduit 3010.

The conduit coupler 3012 may include a fluid flow path 3026 disposed between a first end 3022 and a second end 3024. The conduit coupler 3012 may be an L-shaped conduit for a tube connection with a fluid conduit of the system. The first end 3022 may be configured to interface with and connect to a fluid conduit, such as the supply conduit 242 or the return conduit 241, for receiving or directing fluid to or from the fluid mover 240 and the heat exchanger 220. In one aspect, the first end 3022 may connect to the fluid conduit in the form of a tube (e.g., a polyvinyl tube or TPU) by insertion therewithin and with a hose clamp compressing the fluid conduit in engagement with the first end 3022.

The second end 3024 may include a thread (or barbs) operable to engage and connect to an end of the flexible conduit. For instance, in the case of the flexible conduit 3010 including a spiral wound wire within it, the thread may engage with the spiral wound wire for maintaining a connection between the second end 3024 and the flexible conduit 3010.

The layer coupler 3014 may include a fluid flow path 3036 disposed between a first end 3032 and a second end 3034. The first end 3032 may be adapted to receive and engage an inlet or an outlet of the thermal control element 200, such as an inlet 2210 or outlets 2212. The first end 3032 may correspond to a flat top for a nozzle connection to the thermal control element 200, thereby providing little to no profile that is perceivable by the patient while laying on the thermal control element 200. The second end 3034 may include a thread, similar to the second end 3024 of the conduit coupler 3012, for connection to the flexible conduit 3010.

The flexible coupling 3000 in one aspect may be described as a type of "slinky" that provides a collapsing channel and utilizes spring loaded compression tubing or hose (e.g., flexible conduit 3010). The plastic housing (e.g., the housing 3020 provided for the flexible coupling 4000) may be provided to secure the bottom of the slink so that when there's no load on the pad, the hose (e.g., the flexible conduit 3010) is fully extended. When a load is applied, the hose collapses without a normal force applied to the patient. The tubing compressive ratio may be determined so that, with the hose mounted in the vertical configuration, the distance may be traversed between fully extended and relaxed states. Decreasing the hose diameter for mounting in a horizontal configuration may mitigate the height profile within the patient support 20, 20'.

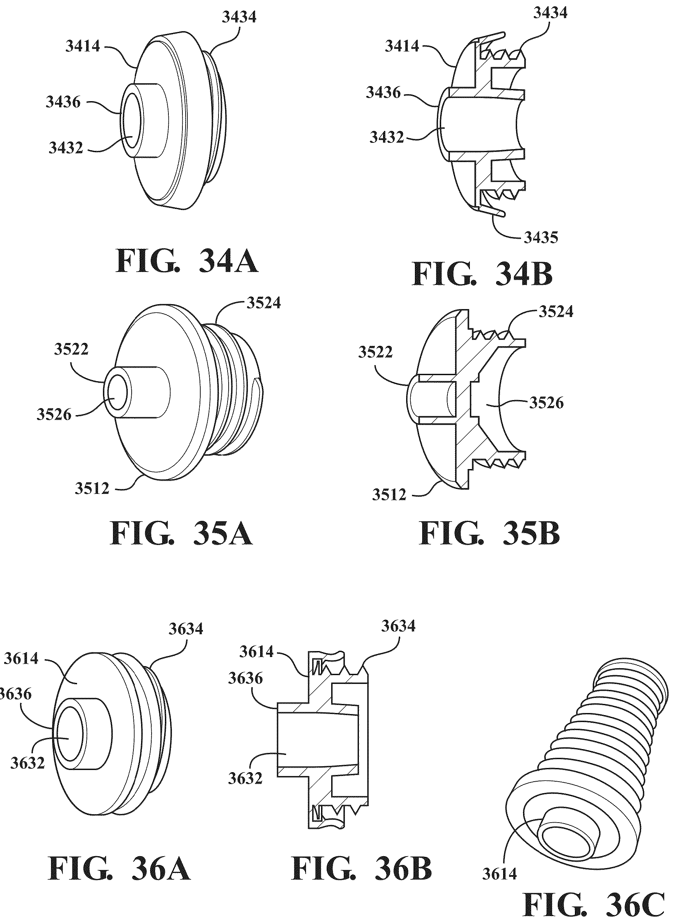

The conduit coupler and the layer coupler may vary from application to application. Example configurations different from the conduit coupler 3012 and the layer coupler 3014 are depicted in Figs. 34, 35, 36, 37, 38, and 39.

Figs. 34A, 34B depict a layer coupler 3414 similar in many respects to the layer coupler 3014, including a fluid flow path 3436 disposed between a first end 3432 and a second end 3434. The second end 3434 includes a thread, similar to the second end 3034, that is operable to engage the flexible conduit 3010. The second end 3434 may include an outer apron 3435 operable to engage an exterior surface of the flexible conduit 3010 for engagement or attachment thereto in conjunction with the thread of the second end 3434. Optionally, the outer apron 3435 may include internal threads or ridges that grip or engage the exterior surface of the flexible conduit 3010. For instance, the outer apron 3435 may include teeth that grip the flexible conduit 3010.

The fluid flow path of the layer coupler 3414 may taper from the second end 3436 to the first end 3434, as depicted in the cross-section view of Fig. 34B with the diameter of the fluid flow path proximal to the second end 3436 being greater than the diameter of the fluid flow path proximal to the first end 3434.

Figs. 36A, 36B, and 36C depict a layer coupler 3614 similar in many respects to the layer coupler 3414, including a fluid flow path 3636 disposed between a first end 3632 and the second end 3634. The second end 3634 may include threads similar to the second end 3034. Likewise, the second end 3634 may include an outer apron 3635 operable to engage an exterior surface of the flexible conduit 3010 similar to the outer apron 3435. The outer apron 3635 in Fig. 36B includes a greater number of teeth or ridges than the outer apron 3435 in Fig. 34B. Fig. 36C shows the layer coupler 3614 connected to the flexible conduit 3010. Additionally, similar to the layer coupler 3414, the layer coupler 3614 may be constructed so that the fluid conduit 3636 from the second end 3632 is tapered toward the first end 3634.

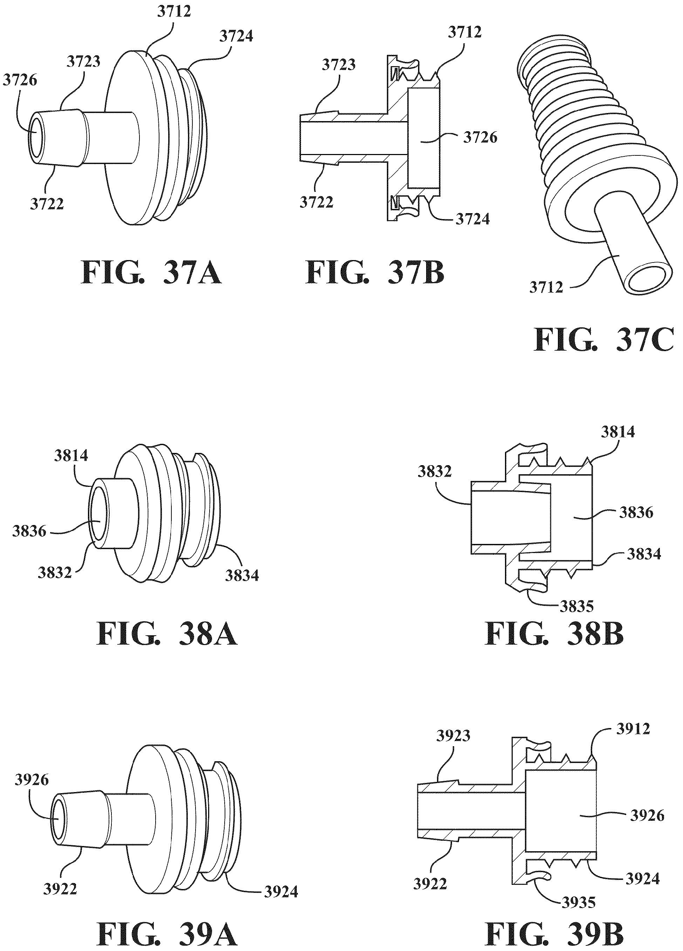

Figs. 38A and 38B depict a layer coupler 3814 similar in many respects to the layer coupler 3414, including a fluid flow path 3836 disposed between a first end 3832 and the second end 3834. The second end 3834 may include threads similar to the second end 3034. Likewise, the second end 3834 may include an outer apron 3835 operable to engage an exterior surface of the flexible conduit 3010 similar to the outer apron 3435. The length and spacing of the threads of the first end 3834 of the layer coupler 3814 may be greater than the length and spacing out threads of the layer couplers 3614, 3414. Additionally, similar to the layer coupler 3414, the layer coupler 3614 may be constructed so that the fluid conduit 3636 from the second end 3632 is tapered toward the first end 3634.

Figs. 35A, 35B depict a conduit coupler 3512 similar in many respects to the conduit coupler 3012, including a fluid flow path 3526 disposed between a first end 3522 and a second end 3524. The second end 3524 includes a thread, similar to the second end 3524, that is operable to engage the flexible conduit 3010. The conduit coupler 3512 unlike the conduit coupler 3012 may be generally straight so that the flow path 3526 is generally linear.

Figs. 37A, 37B depict a conduit coupler 3712 similar in many respects to the conduit coupler 3512, including a fluid flow path 3726 disposed between a first end 3722 and a second end 3724. The second end 3724 includes a thread, similar to the second end 3524, that is operable to engage the flexible conduit 3010. The conduit coupler 3712 in Figs. 37A, 37B includes a barbed end 3723 operable to facilitate engagement to the fluid conduit.

Figs. 39A, 39B depict a conduit coupler 3912 similar in many respects to the conduit coupler 3712, including a fluid flow path 3926 disposed between a first end 3922 and a second end 3924. The second end 3924 includes a thread, similar to the second end 3524, that is operable to engage the flexible conduit 3010. The conduit coupler 3912 in Figs. 39A, 39B includes a barbed end 3923 operable to facilitate engagement to the fluid conduit.

The second end 3924 may include an outer apron 3935 operable to engage an exterior surface of the flexible conduit 3010 for engagement or attachment thereto in conjunction with the thread of the second end 3924. Optionally, the outer apron 3935 may include internal threads or ridges that grip or engage the exterior surface of the flexible conduit 3010. For instance, the outer apron 3935 may include teeth that grip the flexible conduit 3010.

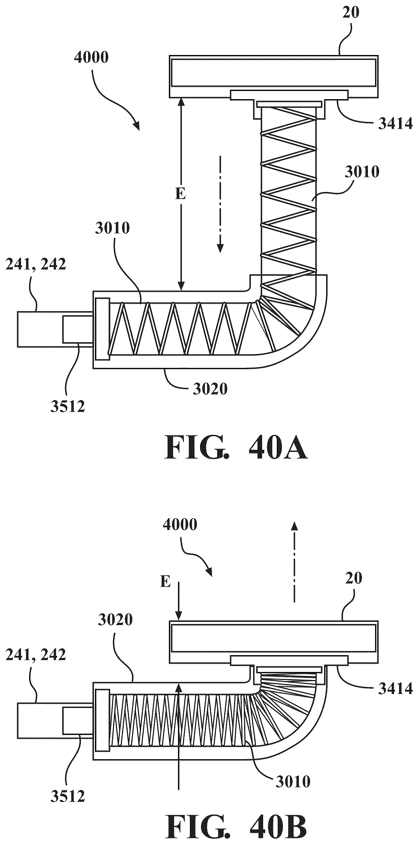

Figs. 40A, 40B depict operation of a flexible coupling configuration according to one aspect and generally designated 4000. The flexible coupling 4000 includes the following components described herein: a flexible conduit 3010, a layer coupler 3414, and a conduit coupler 3512 (a different combination of flexible conduits, layer couplers, and conduit couplers described herein may be utilized). As depicted in Fig. 40A, the flexible conduit 3010 is provided in an extended position with the thermal control element 200 that is at a distance E relative to a housing 3020. The housing conduit 3020 in one aspect may be disposed beneath one or more cushioning elements of the patient supports 20, 20' or proximal to a bottom of the patient supports 20, 20'. Fig. 40B shows the flexible conduit 3010 in a compressed state used so that the distance E is less than the distance E depicted in Fig. 40A. The housing 3020 may be operable to support the flexible conduit 3010 to enable compression and extension thereof. For instance, in Fig. 40B, the flexible conduit is provided in a substantially compressed state within the housing 3020. Relative to Fig. 40B, as the layer coupler 3414 and the thermal control element 200 are raised upward, the housing 3020 supports the flexible conduit to enable extension in a generally vertical manner rather than an arc-like manner relative to the position of the conduit coupler 3512.

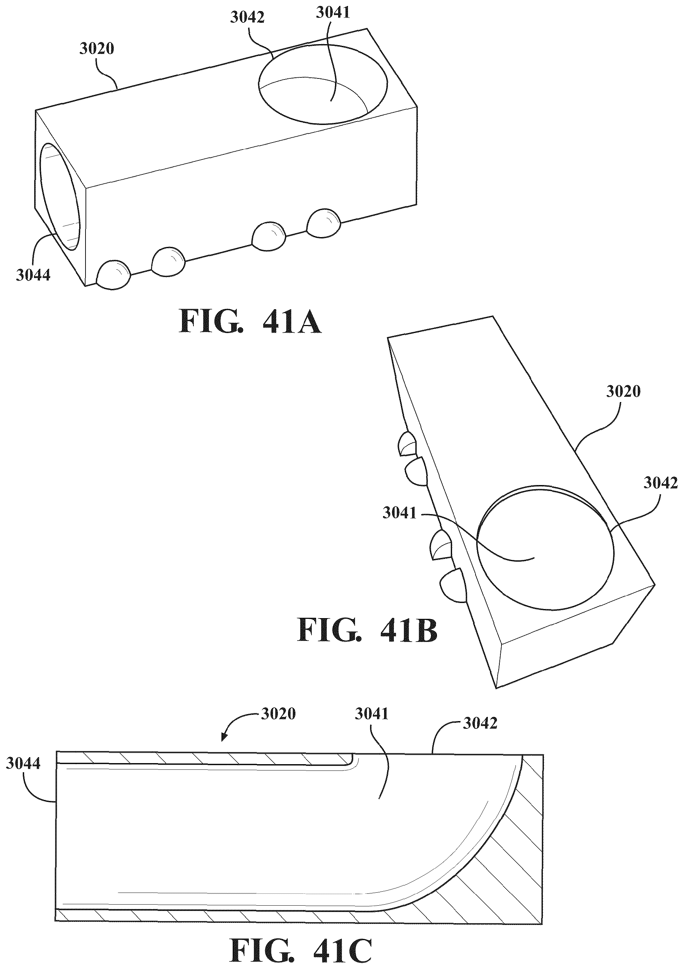

The housing 3020 is shown in further detail in Figs. 41A, 41B, and 41C. The housing 3020 may be provided to facilitate management of movement of the flexible conduit 3010 as described herein. The housing 3020 may include a main body with a first opening 3042 and a second opening 3044 with an interior space 3041 capable of accepting the flexible conduit 3010 from the first opening 3042 to the second opening 3044 and supporting the flexible conduit 3010 therein, as well as facilitating extension and compression of the flexible conduit 3010 as the thermal control element 200 rises and falls.

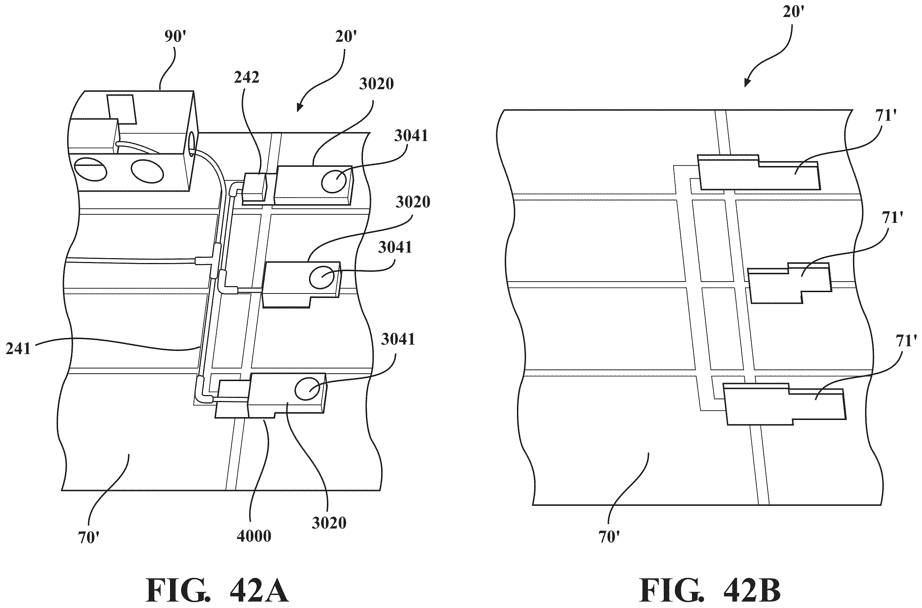

Turning to Figs. 42A, 42B, the housing 3020 is shown in conjunction with the mattress 20'. As can be seen, the foam crib 70' includes a plurality of cutouts 71' adapted to accept and maintain a position of a plurality of housings 3020 for use in conjunction with the flexible conduit configuration 4000 described herein. It is to be understood that the flexible conduit configuration 4000 may be configured differently according to any one of the flexible conduit configurations described herein, including any combination of conduit couplers and layer couplers with and without the housing 3020.

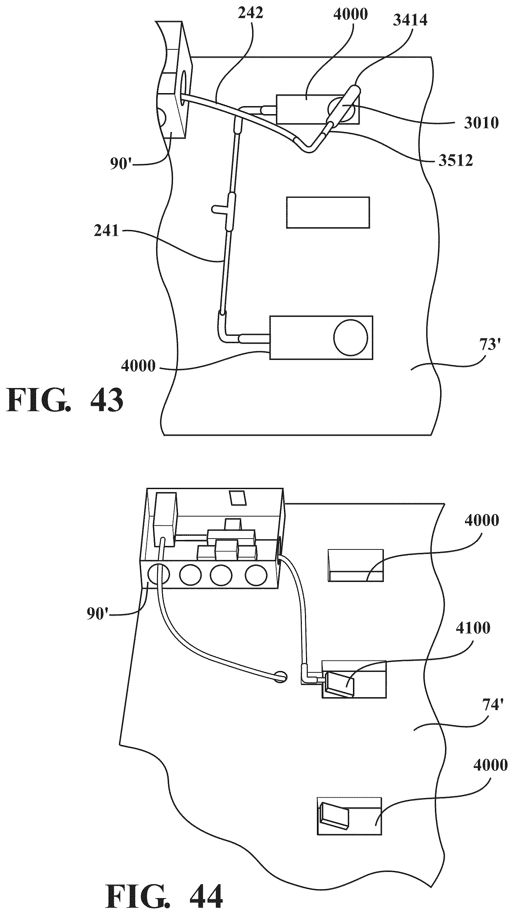

The mattress control assembly 90' is coupled to a supply conduit 242 which in turn is coupled to a conduit coupler 3512 for supply of fluid to the flexible conduit 3010. The mattress control assembly 90' is also coupled to a plurality of return conduits 241, which in turn are also coupled to conduit couplers 3512 for receipt of fluid from the flexible conduit 3010. The foam crib 70' and the plurality of cutouts 71' are disposed distal from an upper surface of the patient support 20' so that these components are substantially imperceptible to the patient. Fig. 43 shows an additional foam layer 73' disposed on top of the foam crib 70' depicted in Figs. 42A, 42B to further support the housings 3020, conduits, and couplers relative to the thermal control element 200. For purposes of disclosure, the one of the housings 3020 is missing in Fig. 43 to further illustrate the flexible conduit 3010, the layer coupler 3414, and the conduit coupler 3512. Fig. 44 shows the same configuration but with an additional layer of foam 74' disposed on top of the foam layer 73', thereby providing additional comfort and separation from the patient relative to components of the system so that the patient is substantially unable to sense the components underneath the patient.

It is to be understood that the flexible coupling configuration for supply and receipt of fluid from the thermal control element 200 may vary from application to application and need not be the same.

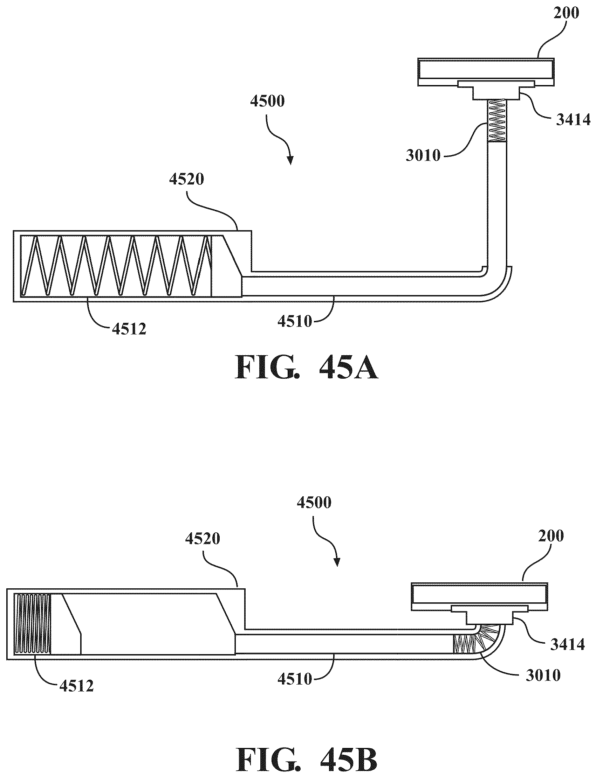

An alternative flexible coupling configuration is depicted in Figs. 45A and 45B and generally designated 4500. The flexible coupling configuration 4500 includes a layer coupler 3414 for forming a fluid connection with the thermal control element 200. The flexible coupling configuration 4500 may include a housing 4520, similar in some respects to the housing 3020 and operable to facilitate guiding fluid conduits of the flexible coupling configuration 4500 relative to the thermal control element 200 as it rises and falls based on movement of the patient and/or different loads applied to the patient support 20, 20'. The housing 4520 may be configured to support a second flexible conduit 4512 as well as an intermediate conduit 4510, which may be generally fixed in length, whereas the second flexible conduit 4512 and the flexible conduit 3010 may be variable in length. It can be seen in Fig. 45B that as the height of the thermal control element 200 falls so that the thermal control element 200 moves closer to the housing 4520, the second flexible conduit 4512 may reduce in length and the flexible conduit 3010 may be received within the housing 4520.

The flexible coupling 4500 may provide mixed partial regions of collapsing channels in both vertical and horizontal configurations. One end of the collapsing tubing may contain an adaptive fitting between the flexible tubing the semi-rigid tubing connected to the thermal electric cooling subsystem, while the other end may be a press fit with the inlet of the thermal control element 200. It is noted, although the slinky may improve pressure redistribution performance, it may be possible to feel the rigid plastic housing when sitting up on the mattress for the horizontal configuration and the vertical configuration may be limited by the mattress immersion. Further, it is noted that the flexible coupling may be sized for constraints within the support surface of the patient support 20, 20', such as the space between the inflatable bladders 66.

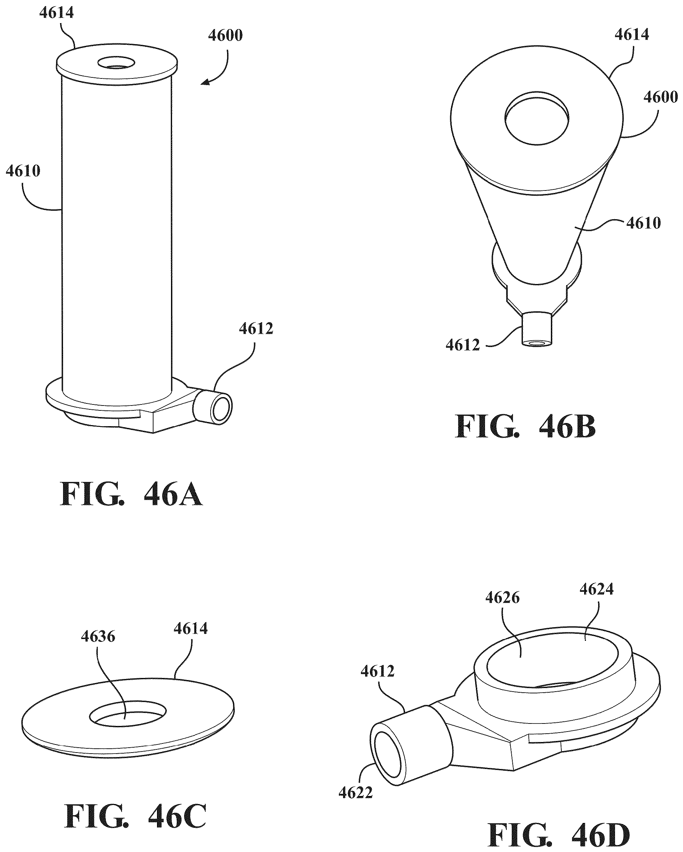

Turning to Figs. 46A, 46B, 46 C, 46D, a flexible coupling configuration is shown and generally designated 4600. The flexible coupler 4600 includes a flexible conduit 4610, a layer coupler 4614, and a conduit coupler 4612. The flexible conduit 4610, like the flexible conduit 3010 may be operable to change in length in response to movement of the patient or changes in a surface level of the thermal control element 200 relative to a lower portion of the patient supports 20, 20' that is proximal to the conduit coupler 4612. The flexible conduit 4610 may be formed of a variety of materials which may depend on the application. In one example, the flexible conduit 4610 may be formed of a polyvinyl chloride material or TPU capable of changing its shape and moving in response to movement of the thermal control element 200 as well as adjacent components of the patient supports 20, 20'.

The conduit coupler 4612 may include a fluid flow path 4626 disposed between a first end 4622 and a second end 4624. The conduit coupler 4612 may provide an L-shaped passage for a tube connection with the fluid conduit 4610. The first end 4622 may be configured to interface with and connect to a fluid conduit, such as the supply conduit 242 or the return conduit 241, for receiving or directing fluid to or from the fluid mover 240 and the heat exchanger 220. In one aspect, the first end 4622 may connect to the fluid conduit in the form of a tube (e.g., a polyvinyl tube) by insertion of the tube within the first end 4622.

The second end 4624 may be sized to connect to an end of the flexible conduit 4610 and may be attached in a variety of ways, including, for example, adhesive, friction fit, and bonding via welding.

The layer coupler 4614 may include a fluid flow path 4636 and may be adapted to engage an inlet or an outlet of the thermal control element 200, such as an inlet 2210 or outlets 2212. The layer coupler 4614 may provide little to no profile that is perceivable by the patient while laying on the thermal control element 200.

In Figs. 46A, 46B, 46C, and 46D, the flexible conduit 4610 may be coupled to the layer coupler 4614 and the conduit coupler 4612 in a variety of ways, including any one or more of a friction fit, adhesive, or a welded bond.

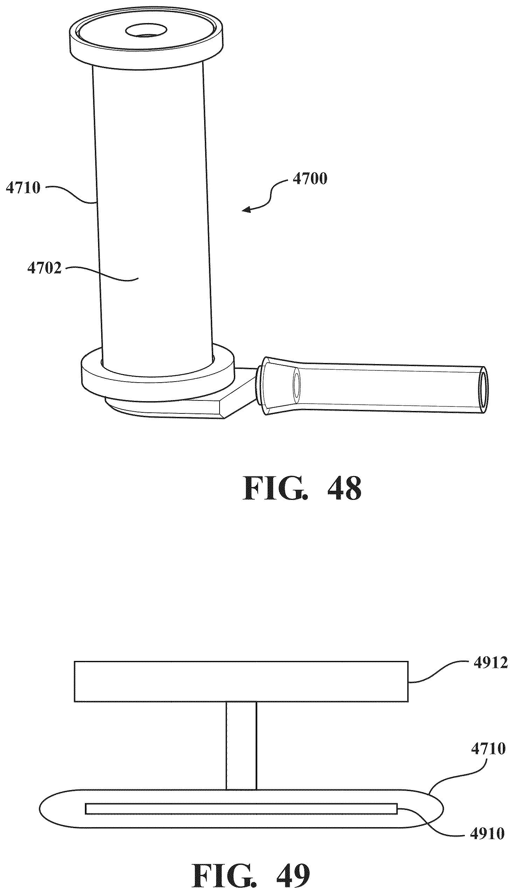

The flexible coupling 4600 may provide a more flexible fluid connection system with the thermal control element 200. The flexible coupling 4600 may include a TPU wall constructed by RF welding the film to itself to form the flexible conduit 4610. Plastic adapters (e.g., layer coupler 4610 and the conduit coupler 4612) may be provided to connect the thermal control element 200 and the semi-rigid tubing (e.g., supply conduit 242 and return conduit 241) within the bottom of the patient support 20, 20'. An adhesive may be provided to provide an air-tight connection between the flexible conduit 4610 and the plastic fittings (e.g., layer coupler 4610 and the conduit coupler 4612). As described herein, filler material (e.g., a spacer layer) may be provided in the flexible conduit 4610 to help keep the channel open as a load is applied. Top and bottom plastic fittings (e.g., layer coupler 4610 and the conduit coupler 4612) may be configured to be press fit with O-rings to the wall for a connection to the fluid conduit 4610 instead of an adhesive. To form the fluid conduit 4610, wall welding may be initially performed by folding the two sides of the film together, welding them, and then folding them inside-out to fit them over the plastic fittings. This process may increase the thickness of the wall, affecting the ability to position it over the plastic fittings and impairing the air-tight seal. A flush weld may be provided by adapting the RF weld process as depicted in Fig. 49 to place an insulating layer between the film when welding. This may enhance air-tight seal of the flexible coupler 4600.

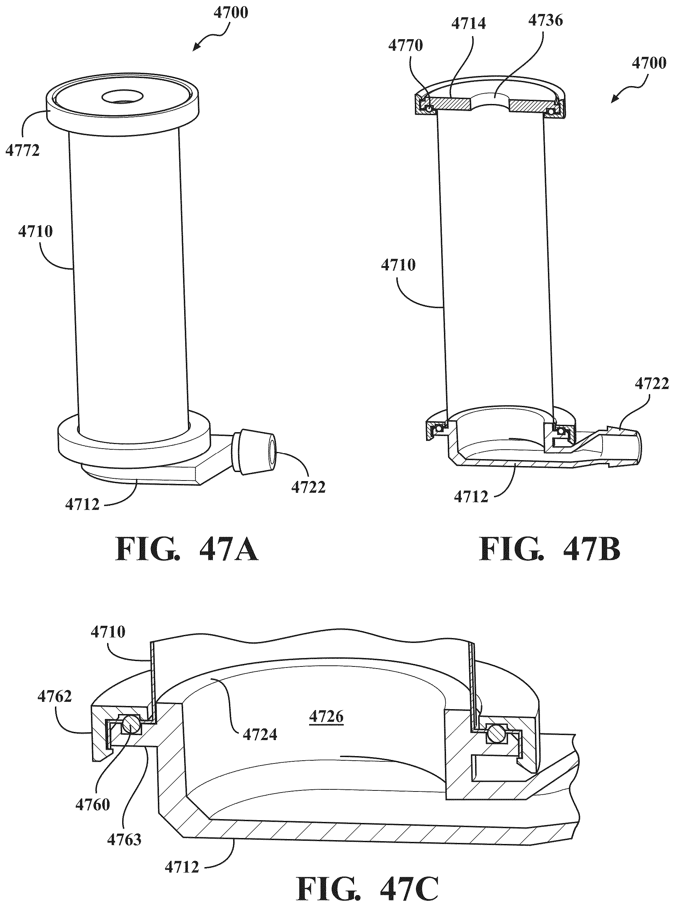

In Figs. 47A, 47B, 47C, 47D a flexible coupling configuration is shown and generally designated 4700. The flexible coupler 4700 includes a flexible conduit 4710, a layer coupler 4714, and a conduit coupler 4712. The flexible conduit 4710, like the flexible conduit 4610 may be operable to change in length or shape in response to movement of the patient or changes in a surface level of the thermal control element 200 relative to a lower portion of the patient supports 20, 20' that is proximal to the conduit coupler 4712. The flexible conduit 4710 may be formed of a variety of materials which may depend on the application. In one example, the flexible conduit 4710 may be formed of a polyvinyl chloride material or TPU material capable of changing its shape and moving in response to movement of the thermal control element 200 as well as adjacent components of the patient supports 20, 20'.

The conduit coupler 4712 may include a fluid flow path 4726 disposed between a first end 4722 and a second end 4724. The conduit coupler 4712 may be an L-shaped passage for a tube connection with the fluid conduit 4710. The first end 4722 may be configured to interface with and connect to the fluid conduit, such as the supply conduit 242 or the return conduit 241, for receiving or directing fluid to or from the fluid mover 240 and the heat exchanger 220. In one aspect, the first end 4722 may connect to the fluid conduit in the form of a tube (e.g., a polyvinyl tube) by insertion of the tube within the first end 4722.

The second end 4724 may be sized to connect to an end of the flexible conduit 4710 via a compression fitting (e.g., via compression ring 4762) operable to sandwich the flexible conduit 4710 between a ridge 4763 of the conduit coupler 4712 and a compression ring 4762. Optionally, an O-ring 4760 may be disposed within a channel of the ridge 4763 to facilitate connecting the flexible conduit 4710 to the conduit coupler 4712. For example, the compression ring 4762 may engage the ridge 4763 and sandwich the flexible conduit 4710 between the compression ring 4762 and the O-ring 4760.

The layer coupler 4714 may include a fluid flow path 4736 and may be adapted to engage an inlet or an outlet of the thermal control element 200, such as an inlet 2210 or outlets 2212. The layer coupler 4714 may provide little to no profile that is perceivable by the patient while laying above the thermal control element 200. The layer coupler 4714 may connect to the flexible conduit 4710 in a manner similar to the conduit coupler 4712 via a compression fitting. For instance, the layer coupler 4714 may include a ridge to which a compression ring 4772 is operable to engage to facilitate sandwiching the flexible conduit 4710 between the compression ring 4772 and an O-ring 4770.

It is to be understood that the flexible conduit 4710 may facilitate fluid flow therethrough as well as maintain flexibility in order to move and change in height relative to changes in height and movement of the patient and the thermal control element 200. In one aspect, in order to prevent collapse of the flexible conduit 4710 or to prevent the flexible conduit 4710 from changing shape in a manner that substantially limits fluid flow there through, a filler material 4702 may be disposed within the flexible conduit 4710. The filler material 4702 may be substantially similar to other filler materials described herein, including any type of filler material described in conjunction with the thermal control element 200 and variants thereof.

The flexible conduit 4710 may be formed of TPU and manufactured from a sheet thereof into a cylindrical structure that forms a fluid conduit for fluid flow there through. An end of the sheet may be welded with another end to form the cylindrical structure. In one aspect, an insulating material 4910 may be inserted within the cylindrical structure in order to provide a buffer material before welding the ends of the sheet together to form the flexible conduit 4710. In other words, RF welding of the TPU wall may provide a continuous profile by overlapping the TPU ends and inserting the buffer material or insulating material 4910 before welding by an RF welding tool 4912 as depicted in Fig. 49.

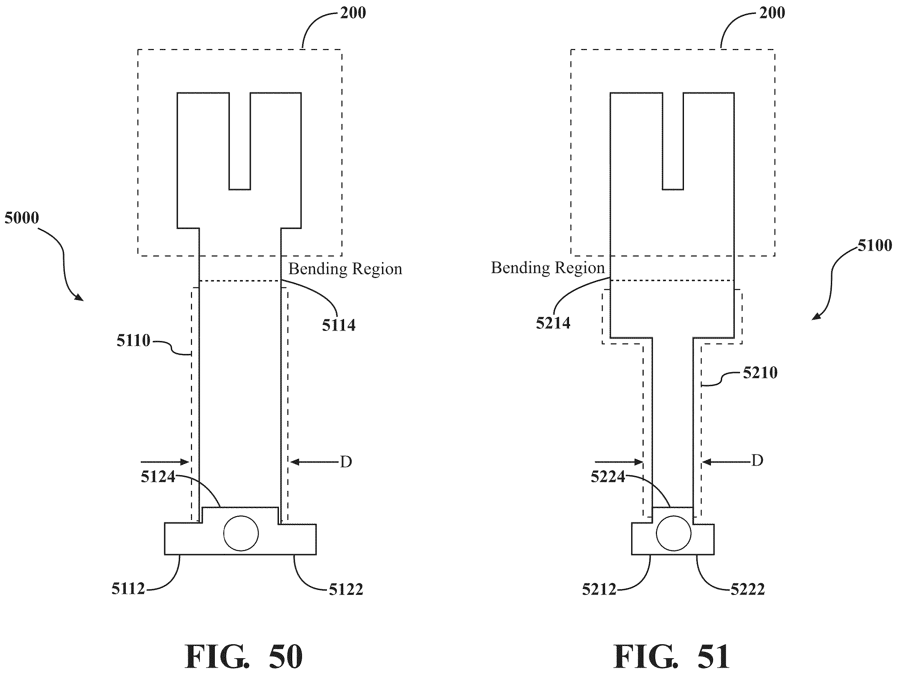

In Fig. 50, a flexible coupling configuration is shown and generally designated 5100. The flexible coupler 5100 includes a flexible conduit 5110, a layer coupling 5114, and a conduit coupler 5112. The flexible conduit 5110, like the flexible conduit 4710, may be operable to change in length in response to movement of the patient or changes in a surface level of the thermal control element 200 relative to a lower portion of the patient supports 20, 20' that is proximal to the conduit coupler 5112. The flexible conduit 5110 may be formed from a variety of materials which may depend on the application. In one example, the flexible conduit 5110 may be formed of any material, such as a polyvinyl chloride material or TPU material, capable of changing its shape and moving in response to movement of the thermal control element 200 as well as adjacent components of the patient supports 20, 20'.

The conduit coupler 5112 may include a fluid flow path disposed between a first end 5122 and a second end 5124. The conduit coupler 5112 may be an L-shaped passage for a tube connection with the flexible conduit 5110. The first end 5122 may be configured to interface with and connect to the fluid conduit, such as the supply conduit 242 or the return conduit 241, for receiving or directing fluid to or from the fluid mover 240 and the heat exchanger 220. In one aspect, the first end 5122 may connect to the fluid conduit in the form of a tube (e.g., a polyvinyl tube) by insertion of the tube within the first end 5122.

The second end 5124 may be sized to connect to an end of the flexible conduit 5110 and may be attached in a variety of ways, including, for example, adhesive, friction fit, and bonding via welding or a compression fit configuration, similar to the compression ring 4762 and the ridge 4763 described in conjunction with Figs. 47A, 47B, 47C.

The layer coupling 5114 may be integral to the thermal control element 200 rather than a separable connection. The layer coupling 5114 may define a bending region with respect to the flexible conduit 5110 and the thermal control element 200. Both the thermal control element 200 and the flexible conduit 5110 may be formed of the same material.

In Fig. 50, the flexible conduit 5110 may have an external surface area defined by the distance D, and, optionally, this external surface area may be increased in size to facilitate greater heat absorption. The flexible conduit 5110 may define a cylindrical construction including filler material disposed therein, or may define a generally flat construction similar to the thermal control element 200, with first and second layers defining sides of the flexible conduit 5110 and a filler material disposed between the first and second layers.

The flexible coupler 5100 may involve removal of a top adapter piece (e.g., plastic pieces) connecting the flexible coupler to the thermal control element 200. These two pieces may affect interface pressure, and so their removal may simplify the configuration.

In Fig. 51, a flexible coupling configuration is shown and generally designated 5200. The flexible coupler 5200 includes a flexible conduit 5210, a layer coupling 5214, and a conduit coupler 5212. The flexible conduit 5210, like the flexible conduit 4710, may be operable to change in length in response to movement of the patient or changes in a surface level of the thermal control element 200 relative to a lower portion of the patient supports 20, 20' that is proximal to the conduit coupler 5212. The flexible conduit 5210 may be formed from a variety of materials which may depend on the application. In one example, the flexible conduit 5210 may be formed of any material, such as a polyvinyl chloride material or TPU material, capable of changing its shape and moving in response to movement of the thermal control element 200 as well as adjacent components of the patient supports 20, 20'.

The conduit coupler 5212 may include a fluid flow path disposed between a first end 5222 and a second end 5224. The conduit coupler 5212 may be an L-shaped passage for a tube connection with the flexible conduit 5110. The first end 5222 may be configured to interface with and connect to the fluid conduit, such as the supply conduit 242 or the return conduit 241, for receiving or directing fluid to or from the fluid mover 240 and the heat exchanger 220. In one aspect, the first end 5222 may connect to the fluid conduit in the form of a tube (e.g., a polyvinyl tube) by insertion of the tube within the first end 5222. The base of the conduit coupler 5212 may be enlarged to facilitate anchoring or maintaining a position of the conduit coupler 5212.

The second end 5224 may be sized to connect to an end of the flexible conduit 5210 and may be attached in variety of ways, including, for example, adhesive, friction fit, and bonding via welding or a compression fit configuration, similar to the compression ring 4762 and the ridge 4763 described in conjunction with Figs. 47A, 47B, 47C.

Similar to the layer coupling 5114, the layer coupling 5214 may be integral to the thermal control element 200 rather than a separable connection. The layer coupling 5214 may define a bending region with respect to the flexible conduit 5210 and the thermal control element 200. Both the thermal control element 200 and the flexible conduit 5110 may be formed of the same material. The bending region of the layer coupling 5214 may be wider and/or larger in cross section relative to the bending region of the layer coupling 5114, such that the greater cross section may facilitate avoidance of a pressure drop or optionally minimizing pressure drop across the layer coupling 5214 (potentially caused by bending in this region).

In Fig. 51, the flexible conduit 5210 may have an external surface area defined by the distance D, and, optionally, this external surface area may be reduced in size to reduce heat loss or add thermal insulation. The flexible conduit 5210 may define a cylindrical construction including filler material disposed therein, or may define a generally flat construction similar to the thermal control element 200, with first and second layers defining sides of the flexible conduit 5210 and a filler material disposed between the first and second layers.

It is noted that one or more aspects described in conjunction with the flexible coupling configuration 5200 may be incorporated into the flexible coupling configuration 5100, and conversely one or more aspects described in conjunction with the flexible coupling configuration 5100 may be incorporated into the flexible coupling configuration 5200.

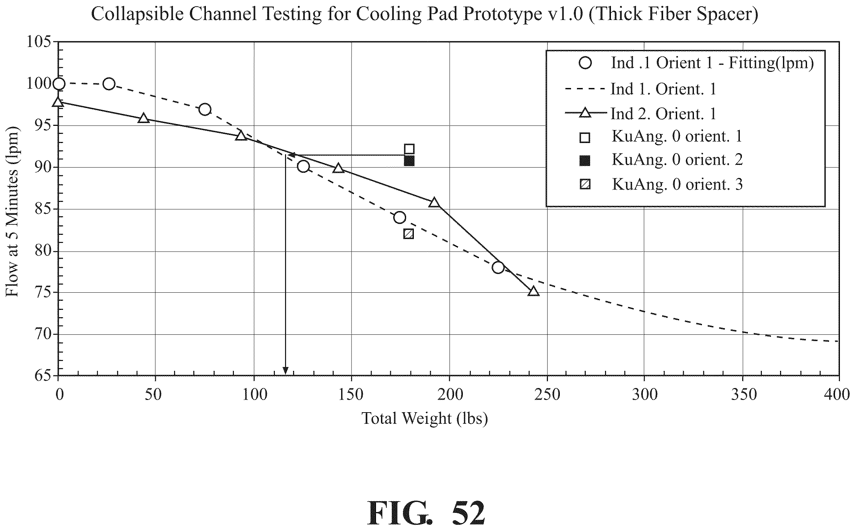

Flow rate analysis results of the flexible coupling configurations described herein are shown in Fig. 52.

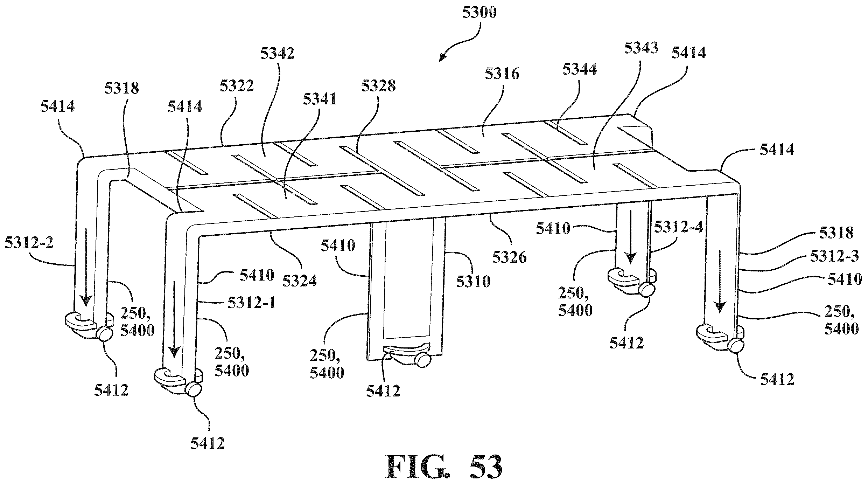

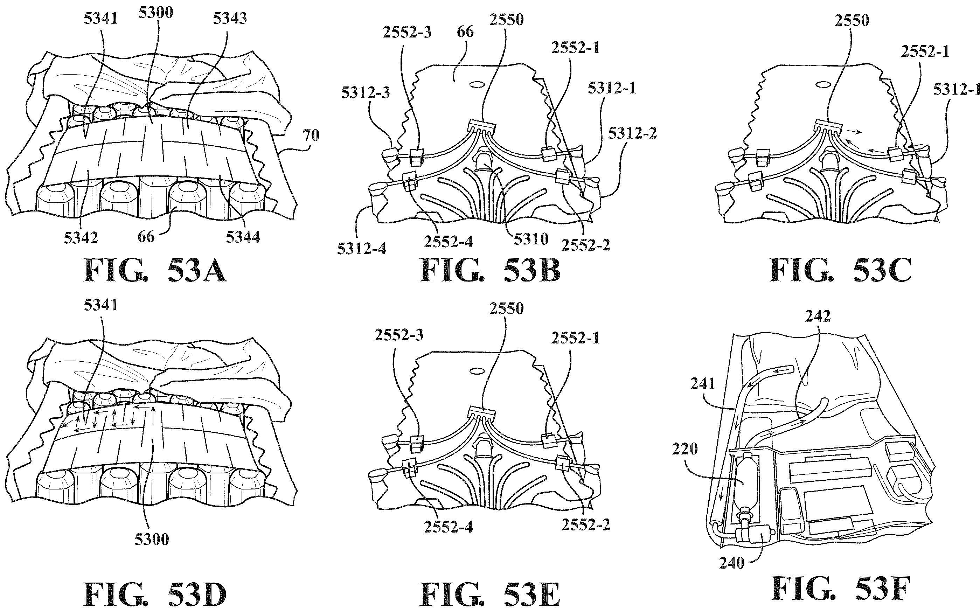

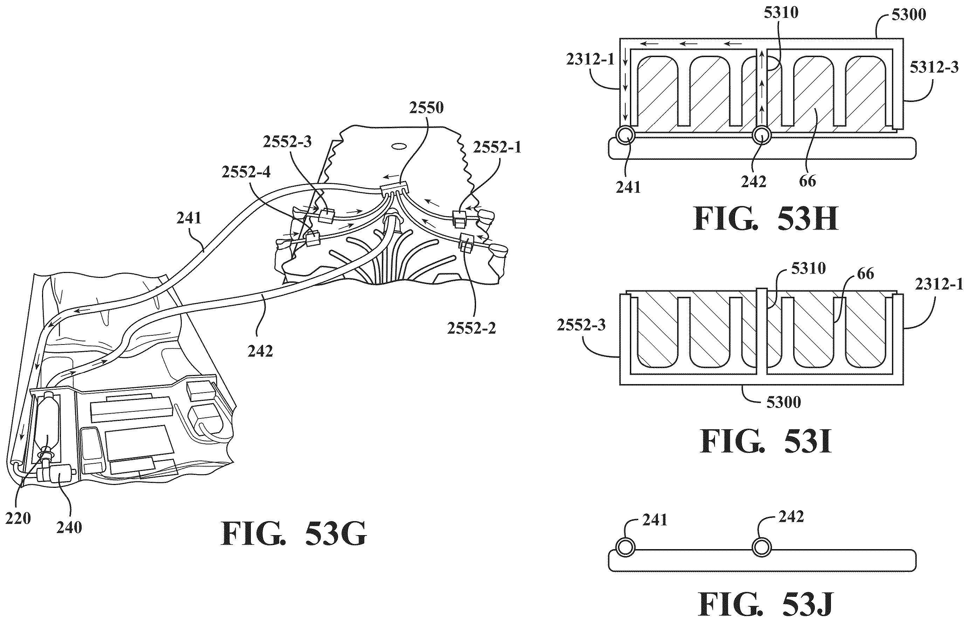

Figs. 53 and 53A-53F show a thermal control element according to one aspect that is generally designated 5300 that utilizes a plurality of flexible couplings 250 with an integral coupling between a flexible conduit and the thermal control element 5300.

The thermal control element 5300, or one or more features thereof, may be incorporated into the patient support 20, 20' as the thermal control element 200. The thermal control element 5300 may include a fluid inlet 5310 and a plurality of fluid outlets 5312-1, 5312-2. The fluid inlet 5310 may receive fluid from the heat exchanger 220 and the fluid mover 240, and the plurality of outlets 5312-1, 5312-2 may return the fluid to the heat exchanger 220 and the fluid mover 240 via one or more fluid conduits 241, 242 and/or flexible couplings 250 as described herein. Fluid flow through the thermal control element 5300 may facilitate controlling or affecting a temperature associated with the patient, such as affecting a skin temperature of a region of the patient (e.g., a sacral region).

The thermal control element 5300 in Fig. 53 includes an upper layer 5322 and a lower layer 5324 formed of sheets of material that are fluid impermeable-although different material constructions that are air and/or water permeable may be utilized. For instance, optionally, the upper layer 5322 may include a plurality of openings that enable fluid flow therethrough to escape the thermal control element 5300.

The sheets of material in one configuration are TPU or polyvinyl chloride that are welded together at one or more seams 5318. The seams 5318 may include a periphery seam 5326 and a plurality of internal seams 5328 for which the upper and lower layers 5322, 5324 are welded together (e.g., RF welding) to form a permanent bond between the upper and lower layers 5322, 5324.

Similar to the thermal control element 2500, thermal control element 5300 may include a filler material 5316, which may be porous with respect to fluid flow therethrough and operable to provide separation between the upper and lower layers 5322, 5324 despite compression caused by the weight of the patient on the thermal control element 5300. The filler material 5316 may be constructed in a variety of ways depending on the configuration and may be similar to the filler material 2114 or any other filler material described herein.

The inlet 5310 and the plurality of outlets 5312 in Fig. 53 may be integrally coupled to the flexible coupling 250, similar to the flexible coupler 5100, 5200. The inlet coupling is integral with respect to the flexible coupling 250 for a substantially airtight connection. The flexible coupling 250 may be formed from the same upper and lower layers 5322, 5324 of the thermal control element 5300, including the peripheral seam 5318 and with an optional filler material provided within the flexible coupling.

The flexible coupling 250 in Fig. 53, as mentioned, may be based on one or more aspects of the flexible couplings 5100, 5200 described herein and is generally designated 5400 for purposes of discussion. For instance, the flexible coupler 5400 includes a flexible conduit 5410, a layer coupling 5414, and a conduit coupler 5412. The conduit coupler 5412 may be similar to the conduit coupler 5212 or the conduit coupler 5112.

The flexible conduit 5410, like the flexible conduit 5210, may be operable to change in length in response to movement of the patient or changes in a surface level of the thermal control element 200 relative to a lower portion of the patient supports 20, 20' that is proximal to the conduit coupler 5412. The flexible conduit 5410 may be formed from a variety of materials which may depend on the application. In one example, the flexible conduit 5410 may be formed of any material, such as a polyvinyl chloride material or TPU material, capable of changing its shape and moving in response to movement of the thermal control element 200 as well as adjacent components of the patient supports 20, 20'.

The thermal control element 5300 in one aspect may be operable for zone control so that one or more zones of the thermal control element 5300 may be active or inactive (e.g., supplied with fluid flow or withheld from fluid flow). The thermal control element 5300 in Fig. 53 includes first, second, third, and fourth zones 5341, 5342, 5343, 5344. Similar to the thermal control element 2500 described herein, selectively enabling fluid flow through the fluid outlets 5312-1, 5312-2, 5312-3, and 5312- 4 (via the conduit couplers 5412 of the flexible conduits 250, 5400) may enable selectively enabling or disabling fluid flow through associated zones 5341, 5342, 5343, 5344. Any combination of the zones 5341, 5342, 5343, 5344 may be active or inactive for selectively controlling a temperature with respect to the associated zone and the patient disposed and proximity thereto.

In one aspect, a manifold, such as the manifold 2550 may be operable to selectively control fluid flow with respect to the zones 5341, 5342, 5343, 5344. The manifold 2550 may operate in conjunction with one or more valves, such as the valves 2552-1, 2552-2, 2552-3, 2552-4 shown in Figs. 53A-J and may be selectively activated to enable fluid flow respectively for the first, second, third, and fourth zones 5341, 5342, 5343, 5344. The manifold 2550, as described herein, may return fluid via a return conduit 241 to the fluid mover 240 and the heat exchanger 220, which may supply the fluid to the thermal control layer 5300 via the supply conduit 242. Optionally, one or more valves, such as the valves 2552-1, 2552-2, 2552-3, 2552-4 that control fluid flow through the thermal control layer 200 (e.g., the thermal control layer 5300) may be integral to the manifold 2550.

An example mode of operation with the first zone 5341 having fluid flow enabled while the second, third, and fourth zones 5342, 5343, 5344 are disabled is shown in Figs. 53C-D and Fig. 53H. The valve 2552-1 is enabled in this mode, while the valves 2552-2, 2552-3, 2552-4 are disabled, so that fluid flows from the supply conduit 241 to the first zone 5341 of the thermal control layer 5300. It is to be understood that any of the valves 2552-1, 2552-2, 2552-3, 2552-4 may be selectively activated to control fluid flow respectively to the associated first, second, third, and fourth zones 5341, 5342, 5343, 5344.

Directional terms, such as "vertical," "horizontal," "top," "bottom," "upper," "lower," "inner," "inwardly," "outer" and "outwardly," are used to assist in describing embodiments and aspects of the present disclosure based on the orientation of the embodiments and aspects shown in the illustrations. The use of directional terms should not be interpreted to limit embodiments or aspects to any specific orientation(s).

The above description is that of current embodiments and aspects of the disclosure. Various alterations and changes can be made without departing from the spirit and broader aspects of the disclosure as defined in the appended claims, which are to be interpreted in accordance with the principles of patent law including the doctrine of equivalents. This disclosure is presented for illustrative purposes and should not be interpreted as an exhaustive description of all embodiments or aspects of the disclosure or to limit the scope of the claims to the specific elements illustrated or described in connection with these embodiments or aspects. For example, and without limitation, any individual element(s) of the described embodiments or aspects may be replaced by alternative elements that provide substantially similar functionality or otherwise provide adequate operation. This includes, for example, presently known alternative elements, such as those that might be currently known to one skilled in the art, and alternative elements that may be developed in the future, such as those that one skilled in the art might, upon development, recognize as an alternative. Further, the disclosed embodiments and aspects include a plurality of features that are described in concert and that might cooperatively provide a collection of benefits. The present disclosure is not limited to only those embodiments or aspects that include all of these features or that provide all of the stated benefits, except to the extent otherwise expressly set forth in the issued claims. Any reference to claim elements in the singular, for example, using the articles "a," "an," "the" or "said," is not to be construed as limiting the element to the singular. Any reference to claim elements as "at least one of X, Y and Z" is meant to include any one of X, Y or Z individually, and any combination of X, Y and Z, for example, X, Y, Z; X, Y; X, Z; and Y, Z.

While several forms have been shown and described, other changes and modifications will be appreciated by those skilled in the relevant art. Therefore, it will be understood that the embodiments shown in the drawings and described above are merely for illustrative purposes, and are not intended to limit the scope of the disclosure which is defined by the claims which follow as interpreted under the principles of patent law including the doctrine of equivalents.

The present disclosure also provides for the following Examples:
1. A patient support system for a patient support apparatus including a deck with one or more deck sections, the patient support system comprising:
   a patient support being supported on the deck;
   a thermal control element including a fluid flow path for directing fluid within the thermal control element, the thermal control element including an inlet to receive fluid and direct the fluid to the fluid flow path, the thermal control element including an outlet to receive fluid from the fluid flow path;
   a fluid mover operable to direct fluid to the inlet of the thermal control element and to receive fluid output from the outlet of the thermal control element; and
   a heat exchanger operable to change a temperature of fluid directed by the inlet of the thermal control element.
2. A patient support system for a patient support apparatus including a deck with one or more deck sections, the patient support system comprising:
   a patient support being supported on the deck;
   a thermal control element operable to affect a temperature of a portion of a patient without significantly affecting a core body temperature of the patient;
   a fluid mover operable to direct fluid to the thermal control element and to receive fluid output from the thermal control element; and
   a heat exchanger operable to change a temperature of fluid directed to the thermal control element.
3. The patient support system according to any preceding Example, wherein the deck is movable relative to a base of the patient support system, wherein at least one of the one or more deck sections is capable of moving relative to another of the one or more deck sections.
4. The patient support system of Example 2, wherein the thermal control element includes a fluid flow path for directing fluid within the thermal control element, wherein the thermal control element includes an inlet to receive fluid and direct the fluid to the fluid flow path, and wherein the thermal control element includes an outlet to receive fluid from the fluid flow path.
5. The patient support system of any preceding Example, wherein the thermal control element, the fluid mover, and the heat exchanger form a closed loop system for circulating fluid to and from the thermal control element.
6. The patient support system of Example 5, wherein the heat exchanger is operable to cool fluid prior to being supplied to the thermal control element.
7. The patient support system of any preceding Example, wherein the thermal control element corresponds to a pad provided within the patient support and arranged proximal to a zone of the patient support to provide localized cooling for a patient supported on the patient support.
8. The patient support system of any preceding Example, wherein the thermal control element includes a plurality of thermal control zones that are selectively enabled or disabled.
9. The patient support system of any preceding Example, wherein the thermal control element includes a plurality of selectable zones for which fluid flow is selectively enabled and disabled, whereby selectively enabling and disabling fluid flow enables selective cooling with respect to each of the plurality of selectable zones.
10. The patient support system of Example 9, comprising one or more valves operable to selectively control fluid flow through the plurality of selectable zones of the thermal control element.
11. The patient support system of Example 9, wherein the patient support system includes one or more inflatable pods, and wherein a zone of the thermal control element is selectively activated or deactivated based on selectively offloading of the one or more inflatable pods located proximal to the zone of the thermal control element.
12. A patient support system for a patient support apparatus including a deck with one or more deck sections, the patient support system comprising:
   a patient support being supported on the deck;
   a thermal control element including a fluid flow path for directing fluid within the thermal control element, the thermal control element including an inlet to receive fluid and direct the fluid to the fluid flow path, the thermal control element including an outlet to receive fluid from the fluid flow path, the thermal control element including a plurality of selectable zones for which fluid flow is selectively enabled and disabled, whereby selectively enabling and disabling fluid flow enables selective cooling with respect to each of the plurality of selectable zones;
   a fluid mover operable to direct fluid to the inlet of the thermal control element and to receive fluid output from the outlet of the thermal control element;
   a heat exchanger operable to change a temperature of fluid directed by the inlet of the thermal control element; and
   a control system operable to direct selection of the plurality of selectable zones, the control system operable to direct selective enablement of a first selectable zone of the plurality of selectable zones based on a status of the patient support system.
13. The patient support system of Example 12, wherein the status of the patient support system corresponds to sensor information output from a sensor associated with a patient.
14. The patient support system of Example 13, wherein the sensor is an interface pressure sensor mat capable of outputting information indicative of an interface pressure for one or more locations of the patient support.
15. The patient support system of Example 12, wherein the status of the patient support system corresponds to a turning bladder being on or off.
16. The patient support system of any preceding Example, wherein the thermal control element is configured to cool a portion of a patient without significantly affecting a core body temperature of the patient.
17. The patient support system of Example 1, wherein the thermal control element is configured to affect a core body temperature of a patient.
18. The patient support system of any preceding Example, wherein the thermal control element includes a plurality of fluid outlets.
19. The patient support system of any preceding Example, wherein the thermal control element includes a plurality of fluid inlets.
20. The patient support system of any preceding Example, wherein the heat exchanger is operable to supply heated fluid to the thermal control element.
21. The patient support system of any preceding Example, wherein the thermal control element is a non-disposable component of the patient support.
22. The patient support system of any preceding Example, wherein the fluid mover is upstream of the heat exchanger, and wherein a fluid inlet of the thermal control element receives fluid output from the heat exchanger via a supply conduit.
23. The patient support system of Example 22, wherein the heat exchanger cools fluid received from the fluid mover, wherein the fluid mover imparts heat energy to fluid as it passes through the fluid mover.
24. The patient support system of Example 23, wherein the heat exchanger provides enhanced cooling of fluid received from the fluid mover to increase cooling for the thermal control element.
25. The patient support system of any preceding Example, wherein the fluid mover is downstream of the heat exchanger so that the fluid mover supplies fluid to the thermal control element via a supply conduit.
26. The patient support system of any preceding Example, wherein the fluid mover is arranged to at least one of draw fluid from the thermal control element under vacuum and supply fluid to the thermal control element under pressure.
27. The patient support system of any preceding Example, comprising a first flexible coupling configured to extend from a lower portion of the patient support to an upper portion of the patient support proximal to the thermal control element, wherein the first flexible coupling is fluidly connected to the inlet of the thermal control element, and wherein the first flexible coupling is operable to change in length in response to vertical and/or lateral movement of the thermal control element due to at least one of movement and weight of a patient on the thermal control element and movement of a component of the patient support system in response to control over the patient support system by a caregiver.
28. The patient support system of Example 27, wherein the first flexible coupling is integral to the thermal control element.
29. The patient support system of Example 27, wherein the first flexible coupling includes a layer coupler and a conduit coupler, wherein the layer coupler is connected to the inlet of the thermal control element, and wherein the conduit coupler is connected to a supply conduit that is fluidly coupled to the heat exchanger.
30. The patient support system of Example 29, wherein the first flexible coupling includes a filler material disposed within a fluid flow path, and wherein the filler material is porous and operable to maintain flow in the fluid flow path despite compression caused by compression of the first flexible coupling.
31. The patient support system of Example 27, comprising a second flexible coupling configured to extend from the lower portion of the patient support to the upper portion of the patient support proximal to the thermal control element, wherein the second flexible coupling is fluidly connected to the outlet of the thermal control element, and wherein the second flexible coupling is connected to a return conduit that is fluidly coupled to the fluid mover.
32. The patient support system of Example 27, comprising a second flexible coupling configured to extend from the lower portion of the patient support to the upper portion of the patient support proximal to the thermal control element, wherein the second flexible coupling is fluidly connected to the outlet of the thermal control element, wherein the second flexible coupling is operable to change in length in response to vertical and/or lateral movement of the thermal control element due to movement and weight of a patient on the thermal control element.
33. The patient support system of Example 32, wherein the second flexible coupling includes a filler material disposed within a fluid flow path, and wherein the filler material is porous and operable to maintain flow in the fluid flow path despite compression caused by compression of the second flexible coupling.
34. The patient support system of any preceding Example, wherein the thermal control element includes a filler material disposed within a fluid flow path, wherein the filler material is porous and operable to support separation between layers of the thermal control element for facilitating flow through the fluid flow path despite compression caused by weight of a patient on the thermal control element.
35. The patient support system of any preceding Example, wherein the patient support includes at least one inflatable bladder.
36. The patient support system of Example 35, wherein the at least one inflatable bladder includes first and second turn bladders operable to facilitate turning a patient disposed on the patient support.
37. The patient support system of Example 36, wherein the thermal control element includes first and second zones associated respectively with the first and second turn bladders, wherein the first zone is activated or deactivated in response to activation of the first turn bladder to turn the patient, and wherein the second zone is activated or deactivated in response to activation of the second turn bladder to turn the patient.
38. The patient support system of Example 37, wherein the first zone is activated in response to activation of the first turn bladder to concentrate cooling for the patient in a turned position due to the change in pressure injury site potential caused by activation of the first turn bladder.
39. The patient support system of Example 37, wherein the first zone is associated with the first turn bladder based on an increase in pressure proximal to the first zone caused by activation of the first turn bladder.
40. The patient support system of any preceding Example, wherein the fluid mover is a compressor.
41. The patient support system of any preceding Example, wherein the thermal control element includes a plurality of openings through which the fluid is capable of escaping for cooling purposes relative to a patient.
42. The patient support system of any preceding Example, wherein the patient support is a mattress.
43. The patient support system of any preceding Example, wherein the thermal control element is disposed inside the patient support or outside the patient support.
44. The patient support system of any preceding Example, wherein the thermal control element is provided within the patient support.
45. The patient support system of Example 44, wherein the thermal control element is a thermal control layer within the patient support.
46. The patient support system of Example 44, wherein the thermal control element is a pad within the patient support.
47. The patient support system of Example 44, wherein the thermal control element facilitates flow of fluid within the patient support.
48. The patient support system of any preceding Example, wherein the fluid mover is disposed outside the patient support.
49. The patient support system of any preceding Example, wherein the heat exchanger is disposed outside the patient support.
50. The patient support system of any preceding Example, wherein the thermal control element is a thermal control layer, wherein a plurality of additional layers of material are disposed between the thermal control layer and a patient, and wherein a cooling capability of the thermal control layer in response to fluid flow is operable to affect a temperature of the patient despite presence of the plurality of additional layers disposed between the thermal control layer and the patient.
51. The patient support system of Example 50, wherein fluid flow to the thermal control layer is controlled to adjust a temperature of the patient toward a target temperature.
52. The patient support system of Example 51, comprising a sensor associated with the patient and capable of transmitting sensor information indicative of the temperature of the patient, and wherein the fluid flow to the thermal control layer is controlled based on the sensor information received from the sensor.
53. The patient support system of any preceding Example, wherein the fluid mover is a blower, and wherein the heat exchanger is a thermoelectric cooler.

## Claims

1. A patient support system for a patient support apparatus including a deck with one or more deck sections, the patient support system comprising:
a patient support being supported on the deck;
a thermal control element including a fluid flow path for directing fluid within the thermal control element, the thermal control element including an inlet to receive fluid and direct the fluid to the fluid flow path, the thermal control element including an outlet to receive fluid from the fluid flow path;
a fluid mover operable to direct fluid to the inlet of the thermal control element and to receive fluid output from the outlet of the thermal control element; and
a heat exchanger operable to change a temperature of fluid directed by the inlet of the thermal control element.

2. A patient support system for a patient support apparatus including a deck with one or more deck sections, the patient support system comprising:
a patient support being supported on the deck;
a thermal control element operable to affect a temperature of a portion of a patient without significantly affecting a core body temperature of the patient;
a fluid mover operable to direct fluid to the thermal control element and to receive fluid output from the thermal control element; and
a heat exchanger operable to change a temperature of fluid directed to the thermal control element.

3. The patient support system of any preceding claim, wherein the thermal control element, the fluid mover, and the heat exchanger form a closed loop system for circulating fluid to and from the thermal control element, wherein the heat exchanger is operable to cool fluid prior to being supplied to the thermal control element.

4. The patient support system of any preceding claim, wherein the thermal control element corresponds to a pad provided within the patient support and arranged proximal to a zone of the patient support to provide localized cooling for a patient supported on the patient support.

5. The patient support system of any preceding claim, wherein the thermal control element includes a plurality of selectable zones for which fluid flow is selectively enabled and disabled, whereby selectively enabling and disabling fluid flow enables selective cooling with respect to each of the plurality of selectable zones.

6. The patient support system of claim 5, comprising one or more valves operable to selectively control fluid flow through the plurality of selectable zones of the thermal control element, wherein the patient support system includes one or more inflatable pods, and wherein a zone of the thermal control element is selectively activated or deactivated based on selectively offloading of the one or more inflatable pods located proximal to the zone of the thermal control element.

7. The patient support system of claim 1, wherein the thermal control element includes a plurality of selectable zones for which fluid flow is selectively enabled and disabled, whereby selectively enabling and disabling fluid flow enables selective cooling with respect to each of the plurality of selectable zones, wherein the patient support system further comprises:
a control system operable to direct selection of the plurality of selectable zones, the control system operable to direct selective enablement of a first selectable zone of the plurality of selectable zones based on a status of the patient support system.

8. The patient support system of claim 7, wherein the status of the patient support system corresponds to sensor information output from a sensor associated with a patient, wherein the sensor is an interface pressure sensor mat capable of outputting information indicative of an interface pressure for one or more locations of the patient support.

9. The patient support system of claim 7, wherein the status of the patient support system corresponds to a turning bladder being on or off.

10. The patient support system of any preceding claim, wherein the thermal control element is configured to cool a portion of a patient without significantly affecting a core body temperature of the patient.

11. The patient support system of any preceding claim, wherein the fluid mover is downstream of the heat exchanger so that the fluid mover supplies fluid to the thermal control element via a supply conduit.

12. The patient support system of any preceding claim, comprising a first flexible coupling configured to extend from a lower portion of the patient support to an upper portion of the patient support proximal to the thermal control element, wherein the first flexible coupling is fluidly connected to the inlet of the thermal control element, and wherein the first flexible coupling is operable to change in length in response to vertical and/or lateral movement of the thermal control element due to at least one of movement and weight of a patient on the thermal control element and movement of a component of the patient support system in response to control over the patient support system by a caregiver.

13. The patient support system of any preceding claim, wherein the thermal control element includes a filler material disposed within a fluid flow path, wherein the filler material is porous and operable to support separation between layers of the thermal control element for facilitating flow through the fluid flow path despite compression caused by weight of a patient on the thermal control element.

14. The patient support system of any preceding claim, wherein the thermal control element is a thermal control layer, wherein a plurality of additional layers of material are disposed between the thermal control layer and a patient, and wherein a cooling capability of the thermal control layer in response to fluid flow is operable to affect a temperature of the patient despite presence of the plurality of additional layers disposed between the thermal control layer and the patient, wherein fluid flow to the thermal control layer is controlled to adjust a temperature of the patient toward a target temperature.

15. The patient support system of any preceding claim, wherein the fluid mover is a blower, and wherein the heat exchanger is a thermoelectric cooler.
